# EUROPEAN PATENT SPECIFICATION

(11) **EP 1 313 713 B1**
(45) Date of publication and mention of the grant of the patent: **11.06.2008**
(21) Application number: 01959581.8
(22) Date of filing: 06.08.2001
(51) Int. Cl.: C07D 239/42, C07D 239/52, C07D 239/12, C07D 401/12, C07D 405/04, C07D 409/04, C07D 495/04, A61K 31/506, A61P 25/28

(54) **4-PYRIMIDINAMINE DERIVATIVES, PHARMACEUTICAL COMPOSITIONS AND RELATED METHODS**
4-PYRIMIDINAMINE DERIVATE, PHARMAZEUTISCHE ZUSAMMENSTELLUNGEN UND VERWANDTE METHODEN
DERIVES 4-PYRIMIDINAMINES, COMPOSITIONS PHARMACEUTIQUES ET PROCEDES ASSOCIES

(30) Priority: 08.08.2000 US 223791 P
(43) Date of publication of application: 28.05.2003
(73) Proprietor: Ortho-McNeil Pharmaceutical, Inc., Raritan, NJ 08869 (US)
(72) Inventor: GRANT, Elfrida, R., Perrineville, NJ 08535 (US); BROWN, Frank, K., Whitehouse Station, NJ 08889 (US); ZIVIN, Robert, Allan, Skillman, NJ 08558 (US); MCMILLAN, Michael, Somerville, NJ 08876 (US); ZHONG, Zhong, Bridgewater, NJ 08807 (CN); SCOTT, Malcolm, Telford, PA 18969 (US); REITZ, Allen, B., Lansdale, PA 19446 (US); ROSS, Tina, Morgan, Audubon, PA 19403 (US)
(74) Representative: Fisher, Adrian John
(86) International application number: PCT/US2001/024659
(87) International publication number: WO 2002/012198

(56) References cited:
- EP-A- 0 384 370
- EP-A- 0 945 443

## Description

### Cross Reference to Related Application

This application claims priority from United States provisional application Serial No. 60/223,791, filed August 8, 2000, which is hereby incorporated by reference.

### Field of the Invention

The present invention relates to novel neuroprotective 4-pyrimidineamine derivatives, neuroprotective 4-pyrimidinamine and 2-pyridinamine compositions, and methods of using same to prevent cell death after an ischemic event. The instant compounds and compositions have particular importance in preventing neuronal cell death and its resulting disorders.

### Background of the Invention

Glutamate is the major fast excitatory neurotransmitter in the mammalian central nervous system. It depolarizes neurons by opening three classes of ligand-gated ion channels: AMPA, kainate, and NMDA receptors. Transient increases in synaptic glutamate levels occur during normal excitatory transmission. However, excessive increases in synaptic glutamate levels are toxic to neurons, and trigger the process of neuronal cell death commonly referred to as glutamate excitotoxicity (Meldrum and Garthwaite 1990). Glutamate excitotoxicity contributes to ischemia-induced brain damage, epilepsy, and various chronic neurodegenerative diseases (Meldrum and Garthwaite 1990).

Of the three classes of glutamate-gated channels, specific overactivation of the N-methyl-D-aspartate (NMDA) receptor is primarily responsible for triggering excitotoxic neuron death in a variety of neuron types (Meldrum and Garthwaite 1990). In animal stroke models, ischemia-induced brain damage can be largely alleviated by pretreatment with the specific NMDA receptor antagonist, MK-801 (Park et al. 1988). In many types of neurons, glutamate exictotoxicity is thought to result primarily from excessive influx of calcium ions due to the high permeability of the NMDA receptor for calcium (Schneggenburger et al. 1993). High intracellular calcium levels may lead to overactivation of calcium-regulated enzymes such as nitric oxide synthase, phospholipases, proteases and kinases. Further, high intracellular calcium levels may mediate excitotoxicity.

Glutamate signaling through the NMDA receptor induces phosphorylation and activation of mitogen-activated protein kinases (MAPK) in primary neuronal cultures (Bading and Greenberg 1991; Xia et al. 1995). Animal models of ischemic brain injury suggest that increased activity of MAPK family members may mediate neuronal injury (Alessandrini et al. 1999; Yang et al. 1997). Deletion of Jnk3, a member of the JNK family of MAP kinases which is predominantly expressed in brain, protects hippocampal neurons from kainic acid-induced excitotoxicity in vivo, although the role of the NMDA receptor in this form of toxicity is not clear (Yang et al. 1998). Specific inhibition of the upstream activating kinases of ERK1/2, (p44/42) MAP kinase, protects against neuronal damage due to focal cerebral ischemia (Alessandrini et al. 1999). In cultured primary hippocampal neurons, inhibition of the ERK1/2 (p44/42 MAPK) signaling pathway protects against neuron death induced by removal of kynurenate, a broad spectrum glutamate-receptor antagonist (Murray et al. 1998). Non-receptor-mediated glutamate-induced oxidative toxicity is also blocked by inhibition of the ERK1/2 signaling pathway (Stanciu et al. 2000). Collectively, these reports clearly indicate an important role for ERK1/2 MAPK signaling in glutamate-induced neuronal toxicity. However, it remains unclear as to what class of glutamate receptor can trigger the excitotoxic signaling cascade in which the ERK1/2 MAPK pathway is so critically involved.

Substantial evidence from the literature suggests that MEK (MAP Kinase or ERK Kinase, a threonine-tyrosine kinase activator of ERK1 and ERK2) inhibition is an effective neuroprotective strategy in vivo (Alessandrini et al. 1999; Hu and Wieloch 1994; Kindy 1993). These reports indicate that transient cerebral ischemia induces p42 MAP kinase phosphorylation in rodent brain. A selective inhibitor of MEK1/2, PD 098059, can block this induction in phosphorylation, and can reduce the extent of neuronal damage (Alessandrini et al. 1999). Primary neuronal culture literature also suggests that the MAP kinase pathway is relevant to excitotoxic damage *in vitro* (Bading and Greenberg 1991; Fiore et al. 1993; Kurino et al. 1995; Murray et al. 1998; Rosen et al. 1994; Xia et al. 1996). These reports indicate that glutamate signaling through its various ionotropic and/or metabotropic receptors results in p42/44 MAP kinase activation. Increased p44/42 MAP kinase activation induces immediate early gene transcription (Xia et al. 1996) and is implicated in seizure activity-induced cell death of cultured hippocampal neurons (Murray et al. 1998).

The signaling pathways that link the NMDA receptor to p42/44 MAP kinase activation, or the downstream pathways which link p42/44 MAP kinase to delayed neurotoxicity, are not well understood. The upstream activators of p42/44 MAP kinases are MEK1 and MEK2 (Anderson et al. 1990; Crews, Alessandrini, and Erikson 1992; Zheng and Guan 1993). MEK1/2 are phosphorylated by the Raf family of kinases (Jaiswal et al. 1994; Moodie et al. 1993), which are activated by the Ras family of small GTP-binding proteins (Papin et al. 1995).

One candidate intermediate molecule that may couple NMDA receptor activation to the Ras/Raf/MEK/p42/44 MAPK signaling cascade is the calcium-dependent tyrosine kinase PYK2 (Lev et al. 1995). Increased intracellular calcium levels can activate PYK2, which can in turn activate MAP kinase signaling.

A second candidate intermediate that may link ion channel activation to MAP kinase signaling is calmodulin kinase (CaM-K). Two types of CaM-Ks are highly expressed in neurons, CaM-KII and CaM-KIV (Sakagami and Kondo 1993; Sola, Tusell, and Serratosa 1999). These protein kinases are activated upon binding of calcium and calmodulin, and they can regulate p38, JNK, and p42/44 MAP kinase activity (Enslen et al. 1996).

A third candidate intermediate molecule may be nitric oxide (NO). In cortical neurons, NMDA receptor coupling to NO production through PSD-95 is required for NMDA receptor-triggered neurotoxicity (Sattler et al. 1999). Increased NO production can also increase p42/44 MAP kinase activity (Lander et al. 1996).

Molecules that are downstream of p42/44 MAP kinase include transcription factors such as CREB, Elk-1, c-Jun, and c-Fos (Vanhoutte et al. 1999). The p42/44 MAP kinase pathway can also induce phosphorylation of cytoskeletal components such as neurofilaments (Li et al. 1999a), regulate synapsin I-actin interactions (Jovanovic et al. 1996), phosphorylate myelin basic protein (Ahn et al. 1991), and regulate the secretion of amyloid precursor protein (Desdouits-Magnen et al. 1998). Therefore, there are many potential mediators of neurotoxicity downstream of p42/44 MAP kinase activation.

A detailed understanding of the signaling pathways that are activated downstream of glutamate receptor stimulation would be useful for determining efficient means of preventing hypoxialischemia-induced neuronal damage. Numerous attempts to study these pathways have been made toward this end. Lipton, U.S. Patent 5,506,231, describes a method of reducing damage to CNS neurons in a patient infected with human immunodeficiency virus by administration of a compound that antagonizes the NMDA receptor. This patent does not suggest neuroprotective effects by administration of a compound that modulates signal transduction pathway components downstream of the NMDA receptor. Maiese, U.S. Patent 5,519,035, describes Protein Kinase C inhibitors as neuroprotective from cerebral ischemia induced by nitric oxide administration. A model utilizing hippocampal neuronal cultures is described. Mahanthappa, WO 99/00117, describes compounds, including H89, that mimic the Hedgehog effects on the Patched-mediated signals, particularly inhibitors of protein kinase A (PKA) as neuroprotective agents. Liu, WO 99/58982, describes methods for identifying neuroprotective compounds that antagonize c-Jun N-termina Kinase (JNK) or mixed-lineage kinase (MLK) in neuronal cells, particularly HN33 hippocampal neuronal cells. Finally, Alessandrini, WO 99/34792, describes a mouse model of stroke in which focal cerebral ischemia is induced, and MEK1 inhibitors are administered to monitor neuroprotective effects.

Despite what is known about glutamate receptor-mediated excitotoxicity, much remains to be learned about its mechanisms of action and compounds that can selectively inhibit the neuronal cell death it causes.

### Summary of the Invention

The present invention is directed to novel neuroprotective 4-pyrimidineamine derivatives of the general formula (I) 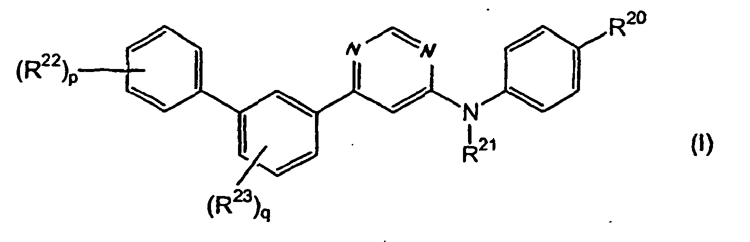 wherein
R²⁰ is selected from the group consisting of 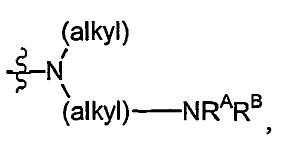 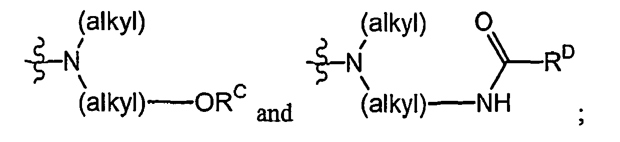
wherein R^{A} and R^{B} are independently selected from hydrogen, alkyl, halogenated alkyl, amino, alkylamino, dialkylamino, aminoalkyl, alkylaminoalkyl, dialkylaminoalkyl, aryl, aralkyl, cycloalkyl, cycloalkyl-alkyl, heteroaryl, heteroarylalkyl, alkoxyalkyl, aryloxy, aryloxyalkyl and dehydroabietyl; wherein the aryl cycloalkyl, heteroaryl or heterocycloalkyl portion is optionally substituted with one or more substituents independently selected from halogen, alkyl, alkoxy, halogenated alkyl, amino, allcylamino, dialkylamino, arylamino, aralkylamino, amido, alkylamido, dialkylamido, arylamido, aralkylamido, azo, nitro, cyano, aryl, aralkyl, aryloxy, carboxy, alkoxycarbonyl, aryloxycarbonyl, alkylthio, arylthio, a1kylsulfonylN(H), or alkylsulfonylN(alkyl);
   alternatively R^{A} and R^{B} are taken together with the nitrogen atom to which they are bound to form a compound selected from the group heteroaryl and heterocycloalkyl; wherein the heteroaryl or heterocycloalkyl is optionally substituted with one or more substituents independently selected from halogen, alkyl, alkoxy, alkoxycarbonyl, halogenated alkyl, alkylcarbonyl, amino, alkylamino, dialkylamino, arylamino, azo, nitro or cyano;
R^{C} is selected from the group consisting of alkyl, aralkyl, alkylcarbonyl, arylcarbonyl, aralkylcarbonyl, (±)-N-benzoyl-aminoalkylcarbonyl or [3aS-(3aa,4β,6aα)]-hexahydro-2-oxo-1H-thieno[3,4-d]imidazole-alkylcarbonyl;
R^{D} is selected from alkyl, aryl, aralkyl, (±)-N-benzoyl-aminoalkyl, [3aS-(3aα,4(3,6aα)]-hexahydro-2-oxo-1H-thieno[3,4-d]imidazole-alkyl or biphenyl; wherein the alkyl or aryl portion of the alkyl, aryl or aralkyl group is optionally substituted with one or more substituents independently selected from halogen, alkyl, alkoxy, amino, alkylamino, dialkylamino, azo, nitro, cyano, or trifluoromethyl);
R²¹ is selected from the group consisting of hydrogen, alkyl, aryl, aralkyl, alkylcarbonyl, arylcarbonyl and aralkylcarbonyl; wherein the aryl portion is optionally substituted with one or more substituents independently selected from halogen, alkyl, alkoxy, halogenated alkyl or nitro;
p is an integer selected from 0 to 3;
q is an integer selected from 0 to 3;
R²² and R²³ are each independently selected from the group consisting of halogen, alkyl, alkoxy, amino, alkylamino, dialkylamino, nitro, cyano, carboxy, alkoxycrbonyl, aryoxycarbonyl, aminocarbonyl, alkylaminocarbonyl and dialkylaminocarbonyl;
or a pharmaceutically acceptable salt thereof.

Illustrative of the invention is a pharmaceutical composition comprising a pharmaceutically acceptable carrier and any of the compounds of formula (I) described above. An illustration of the invention is a pharmaceutical composition made by mixing any of the compounds of formula (I) described above and a pharmaceutically acceptable carrier. Illustrating the invention is a process for making a pharmaceutical composition comprising mixing any of the compounds of formula (I) described above and a pharmaceutically acceptable carrier.

Another example of the invention is the use of any of the compounds of formula (I) described herein in the preparation of a medicament for reducing ischemic death in a cell population in a subj ect in need thereof.

This invention further provides a pharmaceutical composition comprising a pharmaceutically acceptable carrier and a compound having the general formula (II) 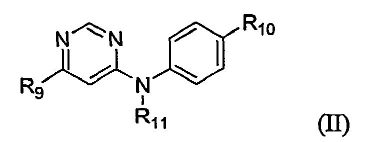 or a pharmaceutically acceptable salt thereof, wherein
(a) R₉ is selected from the group consisting ofH, thienyl, furanyl, pyrrolyl, phenyl, substituted phenyl, pyridinyl, substituted pyridinyl, naphthyl, benzo[b]thien-2-yl, 2-benzofuranyl, pyrimidine and 2,4-(bismetho3cyphenyl)-5-pyrimidinyl, `
   said substituted phenyl having the general formula (III) 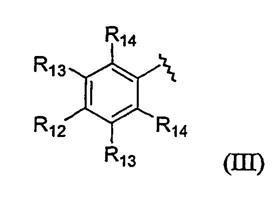 wherein (i) R₁₂ is H, OH, lower alkylthio, alkoxy, alkylamine, dialkylamine, halogen-substituted lower alkyl, halogen substituted lower alkoxy, cyano, cyanoalkyl, phenyl, phenylalkoxy or substituted piperazinyl, N-(t-butoxy)carbamylalkyl, (ii) each R₁₃ is independently H, NO₂, alkoxy, alkylamino, dialkylamino, halogen-substituted lower alkyl, halogen-substituted lower alkoxy or phenyl, and (iii) each R₁₄ is independently H, alkoxy, phenyloxy or phenylalkoxy;
(b) R₁₀ is selected from the group consisting of cyanoalkyl, alkylamino, dialkylamino, hydroxy-substituted alkylamino and hydroxy-substituted dialkylamino; and
(c) R₁₁ is H or lower alkyl.

This invention also provides a pharmaceutical composition comprising a pharmaceutically acceptable carrier and a compound having the general formula (IV) 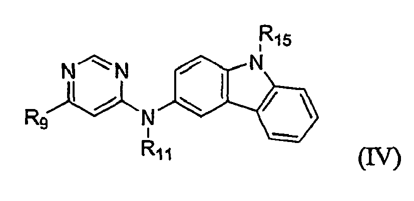 or a pharmaceutically acceptable salt thereof, wherein
(a) R₉ is selected from the group consisting of H, thienyl, furanyl, pyrrolyl, phenyl, substituted phenyl, pyridinyl, substituted pyridinyl, naphthyl, benzo[b] thien-2-yl, 2-benzofuranyl, pyrimidine and 2,4-(bismethoxyphenyl)-5-pyrimidinyl,
   said substituted phenyl having the general formula (V) 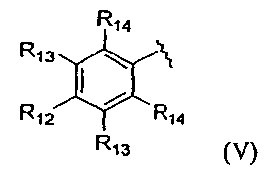 wherein (i) R₁₂ is H, OH, lower alkylthio, alkoxy, alkylamine, dialkylamine, halogen-substituted lower alkyl, halogen substituted lower alkoxy, cyano, cyanoalkyl, phenyl, phenylalkoxy or substituted piperazinyl, N-(t-butoxy)carbamylalkyl, (ii) each R₁₃ is independently H, NO₂, alkoxy, alkylamino, dialkylamino, halogen-substituted lower alkyl, halogen-substituted lower alkoxy or phenyl, and (iii) each R₁₄ is independently H, alkoxy, phenyloxy or phenylalkoxy;
(b) R₁₁ is H or lower alkyl; and
(c) R₁₅ is H or alkyl.

This invention further provides the use of a prophylactically effective amount of the compound contained in any of the instant pharmaceutical compositions of the invention for the manufacture of a medicament for reducing ischemic death in a cell population.

This invention further provides the use of a prophylactically effective amount of the compound contained in any of the instant pharmaceutical compositions of the invention for the manufacture of a medicament for reducing neuronal cell death in response to a traumatic event, wherein said medicament is adapted for administration prior to, during, or within a suitable time period following the traumatic event.

This invention still further provides the use of a prophylactically effective amount of any of the instant pharmaceutical compositions of the invention for the manufacture of a medicament for reducing neuronal cell death in response to a traumatic event in a subject, wherein said medicament is adapted for administration prior to, during, or within a suitable time period following the traumatic event.

Finally, this invention provides an apparatus for administering to a subject any of the instant pharmaceutical compositions comprising a container and the pharmaceutical composition therein, wherein the container has a device for delivering to the subject a prophylactic dose of the pharmaceutical composition.

### Brief Description of the Figures

Figure 1. A: NMDA receptor-mediated functional intracellular calcium response. Filled square symbols represent the control, filled triangle symbols represent 100µM MK-80-1; B: [³H]-NM-801 binding in differentiated P19 neurons.
Figure 2. A: P19 neuron viability experiment using Alamar Blue fluorescence measurements. B: Glutamate dose-response of death with alamar blue readings. Data presented as % control. C: MK-801 dose-dependent block.
Figure 3. A: Compound A, a p38 inhibitor, pretreatment dose response. B: U0126, a MEK1/2 inhibitor, pretreatment dose response.
Figure 4. A: U0126 does not block glutamate-induced calcium responses. B: U0126 does not block [3H]-MK-801 binding in P 19 neurons.
Figure 5. A: U0126 post treatment time course of efficacy. B: Compound A post treatment time course of efficacy.
Figure 6. A: U0126 does not inhibit staurosporine-induced toxicity. Filled square symbols represent no compound; filled triangle symbols represent 10µM U0126. B: U0126 does not block A23187-induced toxicity. C: U0126 does not affect basal P19 neuron viability.

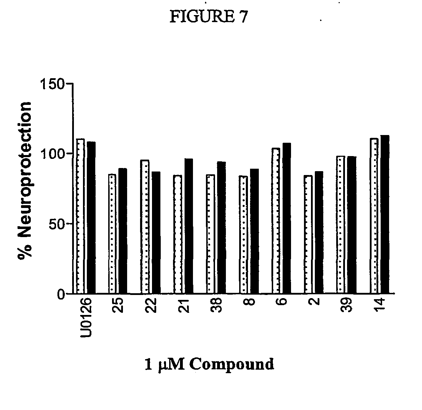

Figure 7. 2-pyridinamine and 4-pyrimidinamine compounds (listed by compound number) exhibit post-treatment delayed neuroprotection. Efficacy that is achieved at 2 hours post-glutamate treatment is equivalent to what is achieved when P19 neurons are pretreated with these compounds. This temporal profile matches that of the MEK inhibitor, U0126. Open, dotted bars represent pre treatment % NP; filled bars represent %NP 2 hours post treatment.

### Detailed Description of the Invention

The present invention provides novel neuroprotective 4-pyrimidineamine derivatives of the general formula (I) 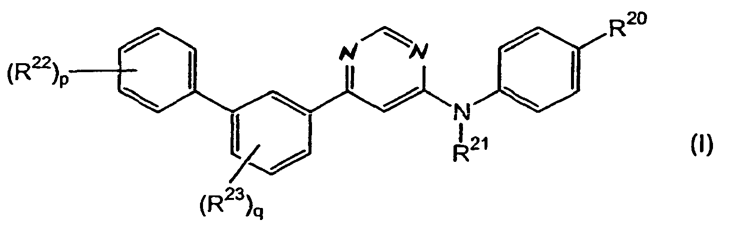 or a pharmaceutically acceptable salt thereof, wherein R²⁰, R²¹, p, q, R²² and R²³ are as previously defined, useful in reducing ischemic death in a cell population.

In.an embodiment of the present invention is a compound of the formula (I) wherein R²⁰ is selected from the group consisting of 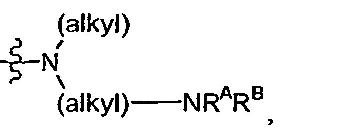 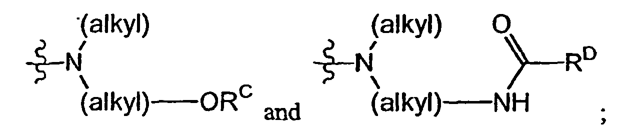
R^{A} is selected from the group consisting of hydrogen, alkyl, aryl and aralkyl; wherein the aryl portion of any of the groups is optionally substituted with one to three substituents independently selected from halogen, lower alkyl, lower alkoxy, halogenated lower alkyl, amino, lower alkylamino, di(lower alkyl)amino, arylamino, aralkylamino, azo, nitro, cyano, aryl, aralkyl, aryloxy, carboxy, lower alkoxycarbonyl, aryloxycarbonyl, lower alkylthio and arylthio;
R^{B} is selected from the group consisting of hydrogen, alkyl, halogenated lower alkyl, amino, lower alkylamino, di(lower alkyl)amino, amino-lower alkyl, lower alkylamino-lower alkyl, di(lower alkyl)amino-lower alkyl, aryl, aralkyl, cycloalkyl, cycloalkyl-lower alkyl, heteroaryl, heteroaryl-lower alkyl, lower alkoxy-lower alkyl, aryloxy, aryloxy-lower alkyl and dehydroabietyl; wherein the aryl, cycloalkyl, heteroaryl or heterocycloalkyl portion of any of the groups is optionally substituted with one to three substituents independently selected from halogen, lower alkyl, lower alkoxy, halogenated lower alkyl, amino, lower alkylamino, di(lower alkyl)amino, arylamino, aralkylamino, azo, nitro, cyano, aryl, aralkyl, aryloxy, carboxy, lower alkoxycarbonyl, aryloxycarbonyl, lower alkylthio and arylthio;
alternatively, R^{A} and R^{B} are taken together with the nitrogen atom to which they are bound to form a ring structure selected from the group consisting of heteroaryl and heterocycloalityl; wherein the heteroaryl or heterocycloalkyl group is optionally substituted with one to two substituents independently selected from halogen, lower alkyl, lower alkoxy, lower alkoxycarbonyl, trifluoromethyl, lower alkylcarbonyl, amino, lower alkylamino, di(lower alkyl)amino, arylamino, azo, nitro or cyano.
R^{C} is selected from the group consisting of lower alkyl, aralkyl, lower alkylcarbonyl, arylcarbonyl, aralkylcarbonyl, (±)-N-benzoyl-amino-lower alkylcarbonyl and [3aS-(3aα,4β,6aα)]-hexahydro-2-oxo-1H-thieno[3,4-d]imidazole-lower alkylcarbonyl;
R^{D} is selected from the group consisting of alkyl, aryl, aralkyl, (±)-N-benzoyl-amino-lower alkyl, [3aS-(3aα,4,β,6aα)]-hexahydro-2-oxo-1H-thieno[3,4-d]imidazole-lower alkyl and biphenyl; wherein the aryl portion of the aryl or aralkyl group is optionally substituted with one to two substituents independently selected from halogen, lower alkyl, lower alkoxy, amino, lower alkylamino, di(lower alkyl)amino, azo, nitro, cyano or trifluoromethyl);
R²¹ is selected from the group consisting of hydrogen, lower alkyl, aryl, aralkyl, lower alkylcarbonyl, arylcarbonyl and aralkylcarbonyl (wherein the aryl portion of the aryl, aralkyl, arylcarbonyl or aralkylcarbonyl group is optionally substituted with one to two substituents independently selected from halogen, lower alkyl, lower alkoxy or trifluoromethyl);
p is an integer from 0 to 2;
q is an integer form 0 to 2;
R²² and R²³ are each independently selected from the group consisting of halogen, lower alkyl, lower alkoxy, amino, lower alkylamino, di(lower alkyl)amino, nitro, cyano, carboxy, lower alkoxycarbonyl, phenyloxycarbonyl, aminocarbonyl, lower alkylaminocarbonyl and di(lower alkyl)aminocarbonyl;
or a pharmaceutically acceptable salt thereof.

In an embodiment of the present invention R²⁰ is

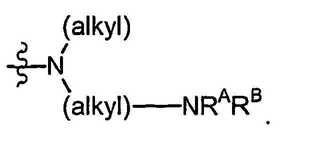

Preferably, R^{A} is selected from the group consisting of hydrogen, lower alkyl and aralkyl. More preferably, R^{A} is selected from the group consisting of hydrogen, methyl, ethyl and benzyl. More preferably still, R^{A} is selected from the group consisting of hydrogen, methyl and benzyl. Most preferably, R^{A} is selected from the group consisting of hydrogen and methyl.

Preferably, R^{B} is selected from the group consisting of hydrogen, lower alkyl, halogenated lower alkyl, aralkyl, substituted aralkyl (wherein the substituents on the aralkyl are one to three independently selected from halogen, alkyl, alkoxy or trifluoromethyl), alkoxyalkyl, cycloalkyl-alkyl, dehydroabietyl, dialkylaminoalkyl, biphenyl, heteroaryl, substituted heteroaryl (wherein the substituents on the heteroaryl group are one to two independently selected from halogen or lower alkyl), heteroarylalkyl, aryloxyalkyl and alkoxycaronylalkyl.

More preferably, R^{B} is selected from the group consisting of hydrogen, methyl, ethyl, propyl, n-butyl, benzyl, phenylethyl, methoxypropyl, cyclohexylmethyl, 3-chlorobenzyl, 2,4-dimethoxybenzyl, 2-ethoxybenzyl, 2,5-difluorobenzyl, dehydroabietyl, 3,4-dimethoxybenzyl, diethylaminoethyl, 4-bromo-2-pyridyl, dimethylaminoethyl, 4-biphenyl, 2-furanylmethyl, 3-iodobenzyl, 2,2,2-trifluoroethyl, 3,4-difluorobenzyl, 2-theinylethyl, 3,5-dimethyl-2-pyridyl, 2-(ethoxy)acetyl, 2-methoxybenzyl, 4-bromobenzyl, 3,5-ditrifluoromethylbenzyl, 3-methoxyphenylethyl, 3,4,5-trimethoxybenzyl, 4-methoxyphenylethyl, 4-imidazolylethyl, 2-trifluoromethylbenzyl, 3-methoxybenzyl, 3-methylbenzyl, 3,5-dimethoxybenzyl, 2-bromobenzyl, 4-fluorobenzyl, 1-naphthylmethyl, phenoxyethyl, 4-trifluoromethylbenzyl, 3-pyridyl, 2-methylbenzyl, 2-fluorobenzyl and 3,4-dimethoxyphenylethyl.

More preferably still, R^{B} is selected from the hydrogen, methyl and benzyl; and R^{B} is selected from the group consisting of methyl, methoxypropyl, cyclohexylmethyl, 3-chlorobenzyl, 2,5-difluorobenzyl, phenylethyl, 4-bromo-2-pyridyl, dimethylaminoethyl, n-butyl, 2-furanylmethyl, 3,4-difluorobenzyl, 2-thienylethyl, 4-bromobenzyl, 3-methoxyphenylethyl, 3,4,5-trimethoxybenzyl, benzyl, 3-methylbenzyl and phenoxyethyl.

More preferably still, R^{B} is selected from the group consisting of methyl, metoxypropyl, 2,5-difluorobenzyl, 3,4difluorobenzyl, 4-bromobenzyl, 3,4,5-trimethoxybenzyl, benzyl, 3-methylbenzyl and n-butyl.

Most preferably, R^{B} is methyl.

In an embodiment of the present invention, R^{A} and R^{B} are taken together with the nitrogen atom to which they are bound to form a ring structure selected from the group consisting of N-pyrrolidinyl, N-morpholinyl, N-imidazolyl, N-1,2,3,4-tetrahydroisoquinolinyl, N-hexamethyleneimine and N-(4-tertiarybutoxycarbonyl)piperazinyl.

Preferably, R^{A} and R^{B} are taken together with the nitrogen atom to which they are bound to form a ring structure selected from the group consisting of N-morpholinyl and N-(4-tertiarybutoxycarbonyl)piperazinyl.

More preferably, R^{A} and R^{B} are taken together with the nitrogen atom to which they are bound to form N-morpholinyl.

In an embodiment of the present invention R²⁰ is

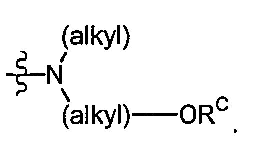

Preferably R^{C} is selected from the group consisting of aralkylcarbonyl, (±)-N-benzoyl-2-aminoalkylcarbonyl and [3aS-(3aα,4p,6aα)]-hexahydro-2-oxo-1H-thieno[3,4-d]imidazole-alkylcarbonyl. More preferably, R^{C} is selected from the group consisting of benzoyl, (±)-N-benzoyl-2-aminopropionoyl and [3aS-(3aα,4β,6aα)]-hexahydro-2-oxo-1H-thieno[3,4-d]imidazole-4-pentanoyl.

In an embodiment of the present invention R²⁰ is

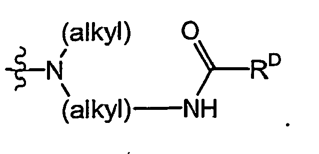

Preferably, R^{D} is selected from the group consisting of (±)-N-benzoyl-2-aminodkyl, [3aS-(3aα,4β,6aα)]-hexahydro-2-oxo-1H-thieno[3,4-d]imidazole-alkyl, alkyl, aryl, substituted aryl (where the substituents on the aryl are independently selected from azo, nitro, halogen, alkoxy, trifluoromethyl), biphenyl, aralkyl and substituted aralkyl (wherein the alkyl portion of the aralkyl group is substituent with a substituent selected from halogen). More preferably, R^{D} is selected from the group consisting of (±)-N-benzoyl-2-aminoeth-2-yl, [3aS-(3aα,4β,6aα)]-hexahydro-2-oxo-1H-thieno[3,4-d]imidazole-but-4-yl, 1-chloro-1-phenyl-methyl, 3-azo-6-nitrophenyl, pentadecanyl, 4-biphenyl, 4-butoxyphenyl, 4-trifluoromethylphenyl, 3-fluorophenyl and propyl.

In an embodiment of the present invention R²¹ is selected from the group consisting of hydrogen, alkyl, aralkyl, alkylcarbonyl and arylcarbonyl, wherein the aryl group is optionally substituted with a substituent selected from halogen and trifluoromethyl. Preferably, R²¹ is selected from the group consisting of hydrogen, ethyl, benzyl, propylcarbonyl, 3-fluorophenylcarbonyl and 4-trifluoromethylphenylcarbonyl. More preferably, R²¹ is hydrogen.

In an embodiment of the present invention p is an integer from 0 to 2. Preferably p is an integer from 0 to 1. In an embodiment of the present invention q is an integer from 0 to 2. Preferably q is an integer from 0 to 1.

In an embodiment of the present invention, R²² and R²³ are each independently selected from the group consisting of halogen, lower alkyl, lower alkoxy, amino, lower alkylamino, di(lower alkyl)amino, nitro, cyano, carboxy, lower alkoxycarbonyl, phenyloxycarbonyl, aminocarbonyl, lower alkylaminocarbonyl and di(lower alkyl)aminocarbonyl.

Compounds of formula (I) wherein R²⁰ is selected from the group consisting of 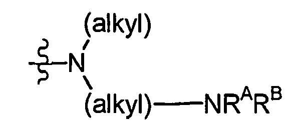 may be prepared according to the process outlined in Scheme 1 and 2.

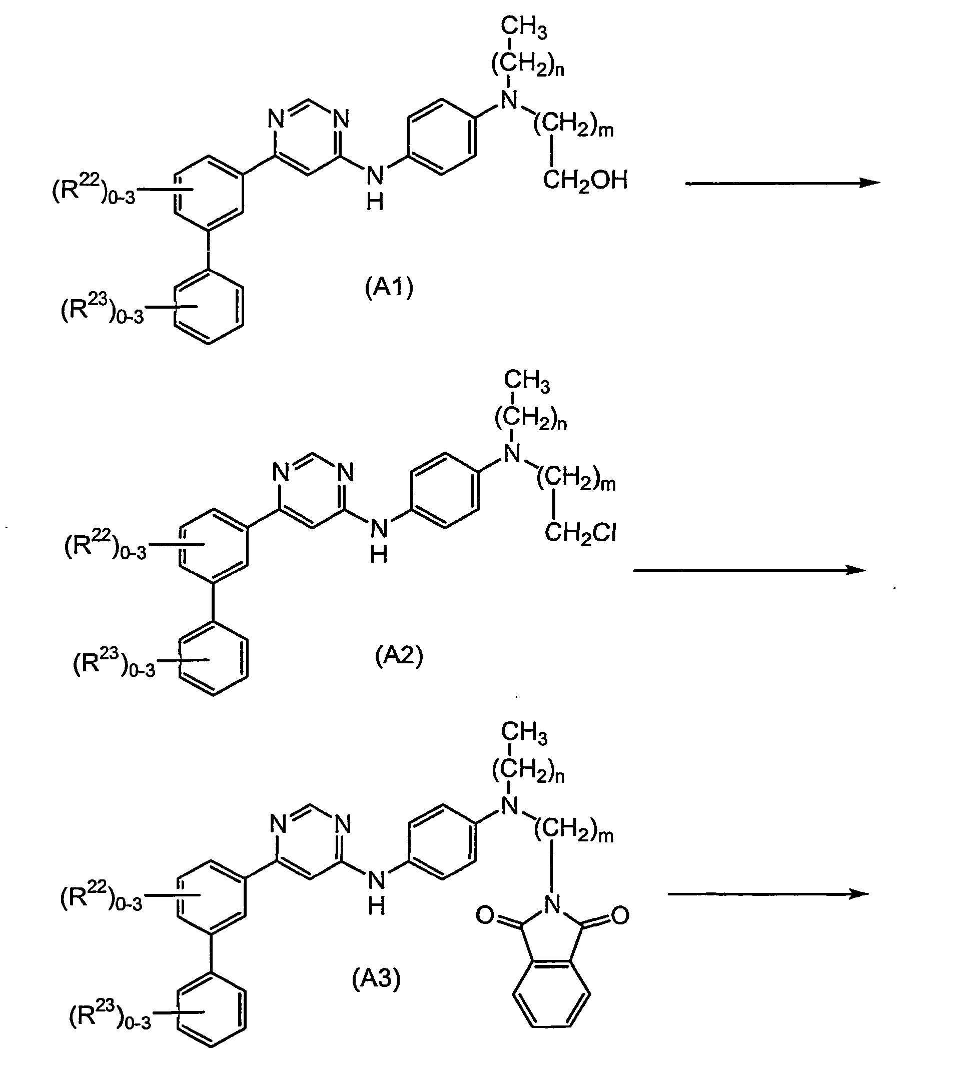

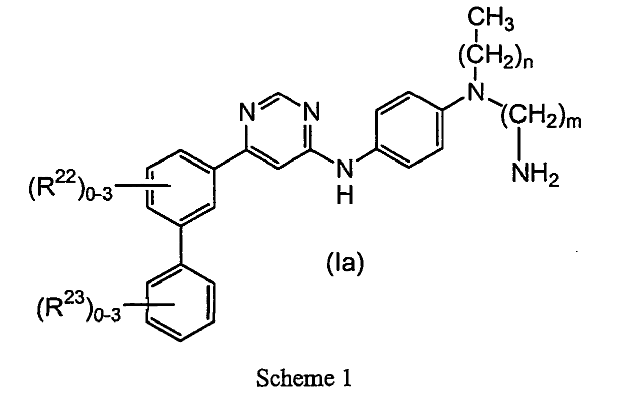

More particularly, a suitably substituted compound of formula (A1), wherein n and m are each independently an integer selected from 0 to 7, a known compound or compound prepared by known methods (for example wherein n and m are each 1, the compound may be purchased from BioFocus, PCC (UK)), is reacted with thionyl chloride, in a halogenated organic solvent such dichloromethane, chloroform, and the like, under anhydrous conditions, to yield the corresponding compound of formula (A2).

The compound of formula (A2) is reacted with potassium phthalimide, in an organic solvent such as N-methyl-pyrrolidinone (NMP), N, N-dimethylformamide (DMF), and the like, under anhydrous conditions, to yield the corresponding compound of formula (A3).

The compound of formula (A3) is reacted with hydrazine monohydrate, in an organic solvent such as ethanol, methanol, isopropanol, and the like, at reflux temperature, to yield the corresponding compound of formula (Ia).

Compounds of formula (I) wherein R^{A} and/or R^{B} are other than hydrogen may be prepared according to the process outlined in Scheme 2.

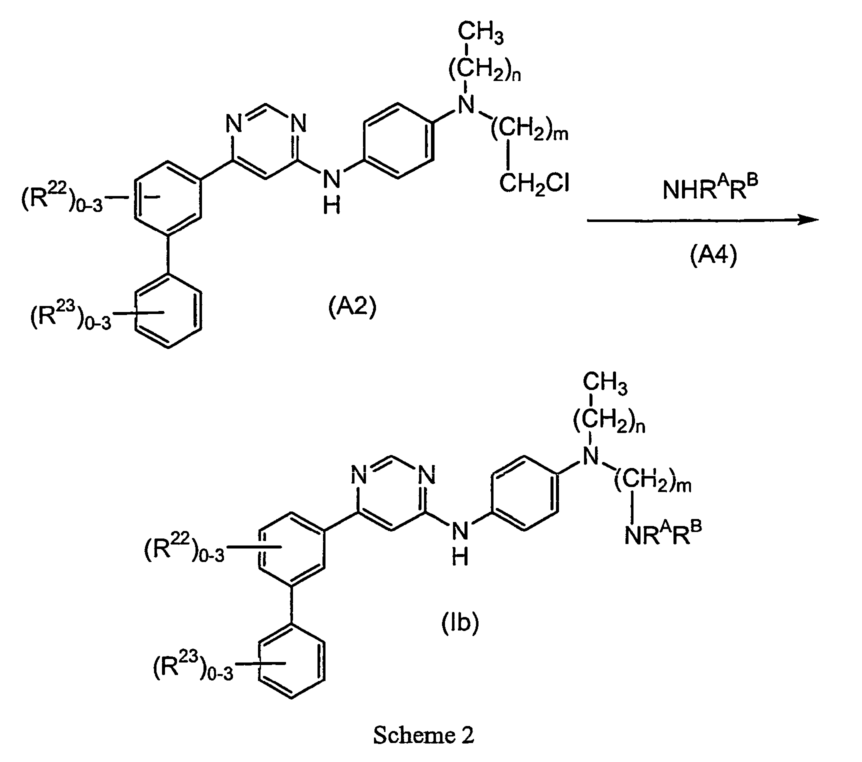

Accordingly, a suitably substituted compound of formula (A2), prepared as in Scheme 1 is reacted with suitably substituted primary, secondary or cyclic amine, a compound of formula (A4), in an organic solvent such as N-methyl-pyrrolidinone (NMP), N,N-dimethylformamide (DMF), and the like, at an elevated temperature in the range of about 100-150°C, preferably at a temperature in the range of about 100-135°C, to yield the corresponding compound of formula (Ib).

Compound of formula (I) wherein R²⁰ is selected from the group consisting of 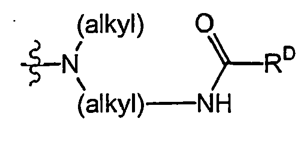 may be prepared according to the process outlined in Scheme 3.

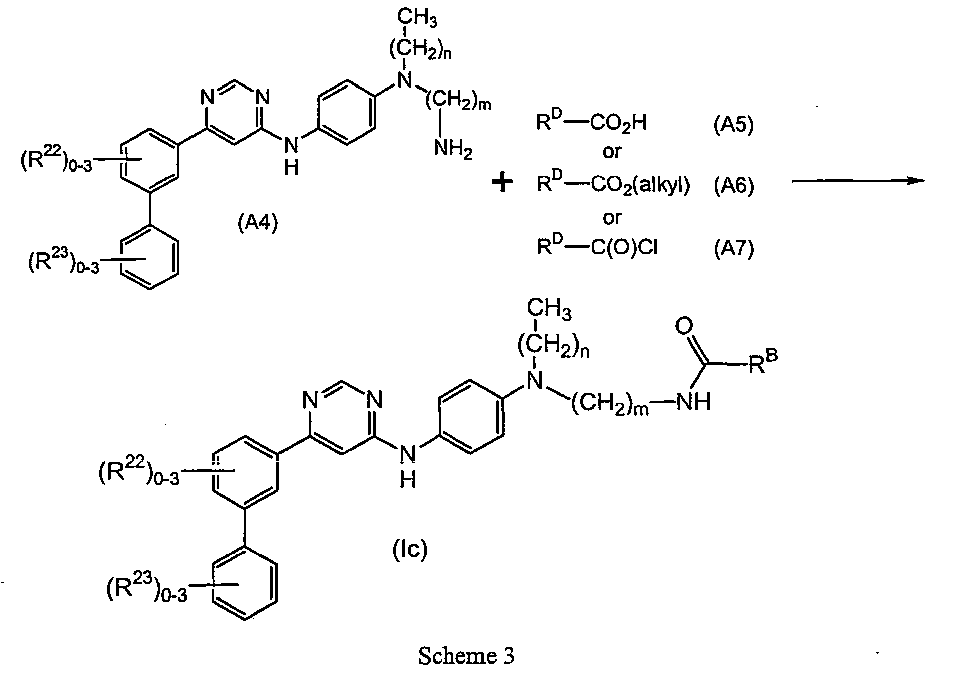

Accordingly, a suitably substituted compound of formula (A4), prepared as in Scheme 1, is reacted with a suitably substituted acid, a compound of formula (A5), a suitably substituted alkyl ester, a compound of formula (A6) or a suitably substituted acid chloride, a compound of formula (A7), to yield the corresponding compound of formula (Ic).

Wherein the compound of formula (A4) is reacted with an acid, a compound of formula (A5), the reaction is carried out in an organic solvent such as NMP, DMF, and the like, in the presence of a coupling catalyst such as O-benzoyltriazol-1-yl-N,N,N'N'-tetramethyluronium hexafluorophosphate (HBTU), O-(7-azabenzotriazol-1-yl)-N,N,N",N"-tetramethyluronium hexafluorophosphate (HATU), and the like, in the presence of a tertiary amine such as triethylamine (TEA), diisopropylethylamine (DIPEA), pyridine, and the like, to yield the corresponding compound of formula (Ic).

Wherein the compound of formula (A4) is reacted with an alkyl ester, a compound of formula (A6), the reaction is carried out in an organic solvent such as dioxane, tetrahydrofuran (THF), toluene, and the like, in the presence of a TEA, DIPEA, pyridine, and the like, to yield the corresponding compound of formula (Ic).

Wherein the compound of formula (A4) is reacted with an acid chloride, a compound of formula (A7), the reaction is carried out in an organic solvent such as dioxane, THF, toluene, and the like, in the presence of a tertiary amine such as TEA, DIPEA, pyridine, and the like, to yield the corresponding compound of formula (Ic).

For compounds of formula (I) wherein R²¹ is selected from the group consisting of alkylcarbonyl, arylcarbonyl or aralkylcarbonyl, and wherein R^{D} is selected from the group consisting of alkyl, aryl or aralkyl, the compound of formula (A4) may be reacted with a suitably substituted compound of formula (A5) or (A7), preferably with two or more equivalents of the compound of formula (A5) or (A7), to yield the corresponding compound of formula (Id).

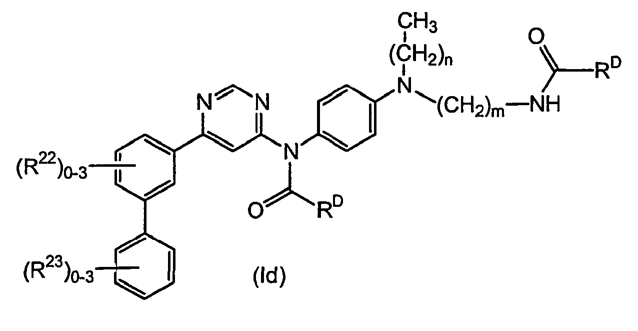

Compounds of formula (I) wherein R²⁰ is selected from the group consisting of 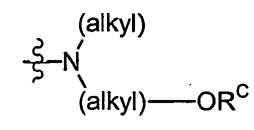 may be prepared according to the process outlined in Scheme 4.

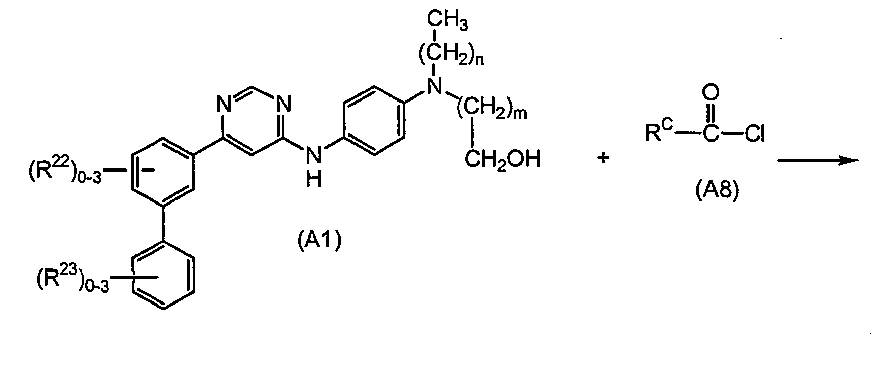

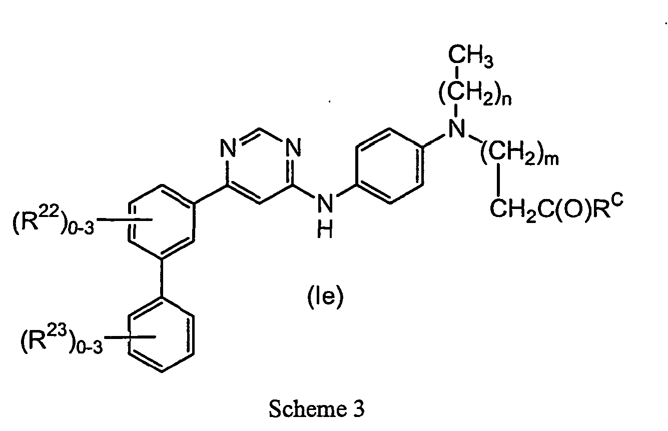

Accordingly, a suitably substituted compound of formula (A1), is reacted with a suitably substituted acid chloride, a compound of formula (A8), in an organic solvent such as toluene, dioxane, THF, and the like, in the presence of a tertiary amine such as TEA, DIPEA, pyridine, and the like, optionally at an elvated temperature in the range of about 50-100°C, to yield the corresponding compound of formula (Ie).

Compounds of formula (I) wherein R²¹ is other than hydrogen may be prepared according to the process outlined in Scheme 5.

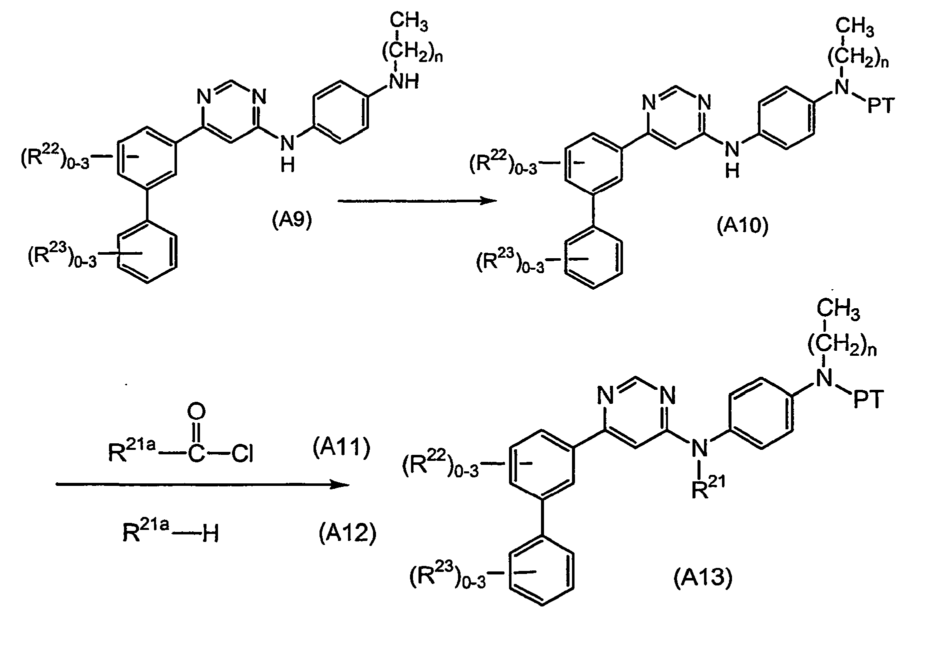

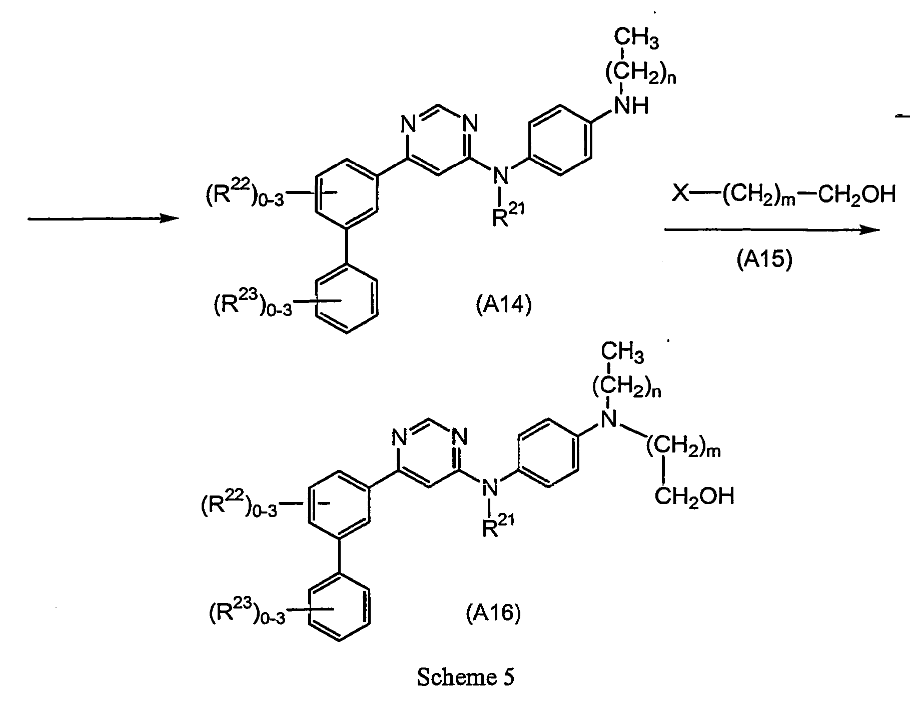

More particularly, a compound of formula (A9), a known compound or compound prepared by known methods, is reacted with a protecting reagent such as 2-(tertiarybutoxycarbonyloxyimino)-2-phenylacetonitrile chloride (BOC-ON), and the like, in an organic solvent such as chloroform, methylene chloride, and the like, to yield the corresponding compound of formula (A10), wherein PT is the corresponding protecting group, such as tertiarybutoxycarbonyl, and the like.

The compound of formula (A10) is reacted with a suitably substituted acid chloride of formula (A11), wherein R^{21a} is alkyl, aryl or aralkyl, or with a suitably substituted acid of formula (A12), to yield the corresponding compound of formula (A13).

Wherein the compound of formula (A10) is reacted with a suitably substituted acid chloride of formula (A11), the reaction is carried out in an organic solvent such as methylene chloride, chloroform, and the like, in the presence of an organic amine such as N-methyl-morpholine, DIPEA, TEA, pyridine, and the like, to yield the corresponding compound of formula (A13).

Wherein the compound of formula (A10) is reacted with a suitably substituted acid of formula (A12), the reaction is carried out in an organic solvent such as N,N-dimethylformamide (DMF), NMP, and the like, in the presence of an activating agent such as carbonyldiimidazole, cyclohexylcarbodiimine, and the like, to yield the corresponding compound of formula (A13).

The compound of formula (A13) is deprotected under standard deprotection conditions, known to one skilled in the art (for example, where the protecting group is tertiarybutoxycarbonyl, the deprotection is carried out in an organic solvent such as methylene chloride, chloroform, and the like, in the presence of an acid such as trifluoroacetic acid, hydrochloric acid, and the like), to yield the corresponding compound of formula (A14).

The compound of formula (A14) is then reacted with a halo alcohol, a compound of formula (A15), wherein X is a halogen such as Br, Cl, I, F, (for example 2-bromoethanol), in an organic solvent such as ethanol, metahnol, and the like, optionally at reflux temperature, to yield the corresponding compound of formula (A16).

One skilled in the art will recognize that the compound of formula (A16) is then substituted for the compound of formula (A1) in Scheme 1 or Scheme 4, and reacted according to the process outlined in Scheme 1, 2, 3 and/or 4 to yield the desired corresponding compound of formula (I).

The present invention further provides pharmaceutical compositions comprising a pharmaceutically acceptable carrier and a compound of formula (I).

The present invention further provides the use of a therapeutically or prophylactically effective amount of one or more compounds of formula (I) or a pharmaceutical composition containing one or more compounds of formula (I) for the manufacture of a medicament for reducing ischemic death in a cell population in a subject in need thereof.

The invention also provides a pharmaceutical composition comprising a pharmaceutically acceptable carrier and a compound having the general formula (II) 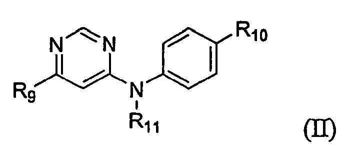 or a pharmaceutically acceptable salt thereof, wherein
(a) R₉ is selected from the group consisting of H, thienyl, furanyl, pyrrolyl, phenyl, substituted phenyl, pyridinyl, substituted pyridinyl, naphthyl, benzo[b]thien-2-yl, 2-benzofuranyl, pyrimidine and 2,4-(bismethoxyphenyl)-5-pyrimidinyl,
   said substituted phenyl having the general formula (III) 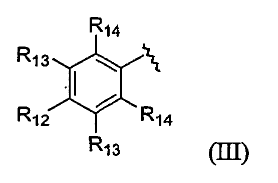 wherein (i) R₁₂ is H, OH, lower alkylthio, alkoxy, alkylamine, dialkylamine, halogen-substituted lower alkyl, halogen substituted lower alkoxy, cyano, cyanoalkyl, phenyl, phenylalkoxy or substituted piperazinyl, N-(t-butoxy)carbamylalkyl, (ii) each R₁₃ is independently H, NO₂, alkoxy, alkylamino, dialkylamino, halogen-substituted lower alkyl, halogen-substituted lower alkoxy or phenyl, and (iii) each R₁₄ is independently H, alkoxy, phenyloxy or phenylalkoxy;
(b) R₁₀ is selected from the group consisting of cyanoalkyl, alkylamino, dialkylamino, hydroxy-substituted alkylamino and hydroxy-substituted dialkylamino; and
(c) R₁₁ is H or lower alkyl.

In one embodiment of the instant pharmaceutical composition, R₉ is substituted phenyl. In another embodiment, R₁₁ is H and R₁₀ is dialkylamino or hydroxy-substituted dialkylamino.

In the preferred embodiment of the instant pharmaceutical composition, the compound contained therein is selected from the following group, whose structures are set forth in the Experimental Details:
N1,N1-dimethyl-N4-[6-[4-(phenylmethoxy)phenyl]-4-pyrimidinyl]-1,4-benzenediamine;
N1-(6-[1,1'-biphenyl]-3-yl-4-pyrimidinyl)-N4,N4-dimethyl-1,4-benzenediamine;
N1-[6-[3,5 -bis(trifluoromethyl)phenyl]-4-pyrimidinyl]-N4,N4-dimethyl-1,4-benzenediamine;
2-[[4-[(6-[1,1'-biphenyl]-3-yl-4-pyrimidinyl)amino]phenyl]ethylamino]-ethanol;
2-[[4-[(6-benzo[b]thien-2-yl-4-pyrimidinyl)ami-no]phenyl]ethylamino]-ethanol;
2-[ethyl[4-[[6-[4-(trifluoromethoxy)phenyl]-4-pyrimidinyl]amino]phenyl] amino]-ethanol; and
2-{[4-(2',4'-bis-benzyoxy-[4,5']bipyrimidyl-6-ylamino)-phenyl]-ethyl-amino}-ethanol, the compound having the structure shown below.

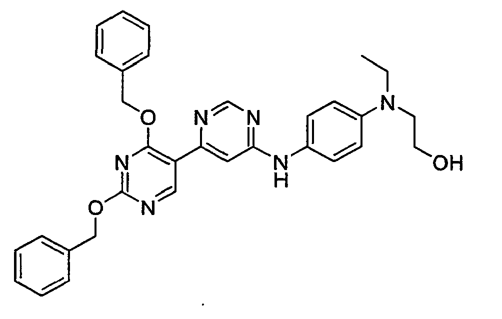

The invention also provides a pharmaceutical composition comprising a pharmaceutically acceptable carrier and a compound having the general formula (IV) 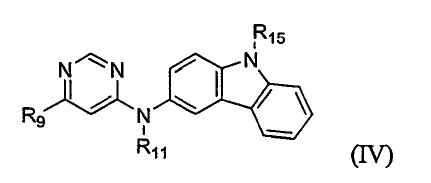 or a pharmaceutically acceptable salt thereof, wherein
(a) R₉ is selected from the group consisting of H, thienyl, furanyl, pyrrolyl, phenyl, substituted phenyl, pyridinyl, substituted pyridinyl, naphthyl, benzo[b]thien-2-yl, 2-benzofuranyl, pyrimidine and 2,4-(bismethoxyphenyl)-5-pyrimidinyl,
   said substituted phenyl having the general formula (V) 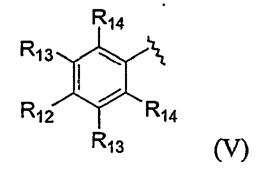 wherein (i) R₁₂ is H, OH, lower alkylthio, alkoxy, alkylamine, dialkylamine, halogen-substituted lower alkyl, halogen substituted lower alkoxy, cyano, cyanoalkyl, phenyl, phenylalkoxy or substituted piperazinyl, N-(t-butoxy)carbamylalkyl, (ii) each R₁₃ is independently H, NO₂, alkoxy, alkylamino, dialkylamino, halogen-substituted lower alkyl, halogen-substituted lower alkoxy or phenyl, and (iii) each R₁₄ is independently H, alkoxy, phenyloxy or phenylalkoxy;
(b) R₁₁ is H or lower alkyl; and
(c) R₁₅ is H or alkyl.

Unless specified otherwise, as used herein, the term "alkyl" refers to a saturated straight, branched or cyclic substituent consisting solely of carbon and H. Preferably, the alkyl chain comprises one to twenty carbon atoms, more preferably, one to fifteen carbon atoms, more preferably still, one to eight carbon atoms. Lower alkyl refers to an alkyl containing 1 to 4 carbon atoms.

The term "alkenyl" refers to an unsaturated straight, branched or cyclic substituent consisting solely of carbon and H that contains at least one double bond. The term "alkynyl" refers to an unsaturated straight, branched or cyclic substituent consisting solely of carbon and H that contains at least one triple bond.

The term "alkoxy" refers to O-alkyl where alkyl is as defined. The term "alkylthio" refers to S-alkyl where alkyl is as defined and the alkylthio group is bound through the S atom.

An acidic alkyl is a carbon chain with a terminal COOH group. As used herein, the term "alkylamino" shall mean an alkyl substituted amine group. Similarly, the term "dialkylamino" shall mean an amino group substituted with two independently selected alkyl groups. The term "hydroxy substituted dialkylamino" shall refer to a dialkylamino group wherein either or both of the alkyl groups are independently substituted with a hydroxy group, independently at any of the carbon atoms of the alkyl group(s).

The term "halo" means fluoro, chloro, bromo and iodo.

The symbol "Ph" or "PH" refers to phenyl.

As used herein, unless otherwise noted, "aryl" shall refer to unsubstituted carbocylic aromatic groups such as phenyl, naphthyl, and the like.

As used herein, unless otherwise noted, "aralkyl" shall mean any lower alkyl group substituted with an aryl group such as phenyl, naphthyl and the like. For example, benzyl, phenylethyl, phenylpropyl, naphthylmethyl, and the like.

As used herein, unless otherwise noted, "heteroaryl" shall denote any five or six membered monocyclic aromatic ring structure containing at least one heteroatom selected from the group consisting of O, N and S, optionally containing one to three additional heteroatoms independently selected from the group consisting of O, N and S; or a nine or ten membered bicyclic aromatic ring structure containing at least one heteroatom selected from the group consisting of O, N and S, optionally containing one to four additional heteroatoms independently selected from the group consisting of O, N and S. The heteroaryl group may be attached at any heteroatom or carbon atom of the ring such that the result is a stable structure.

Examples of suitable heteroaryl groups include, but are not limited to, pyrrolyl, furyl, thienyl, oxazolyl, imidazolyl, purazolyl, isoxazolyl, isothiazolyl, triazolyl, thiadiazolyl, pyridyl, pyridazinyl, pyrimidinyl, pyrazinyl, pyranyl, furazanyl, indolizinyl, indolyl, isoindolinyl, indazolyl, benzofuryl, benzothienyl, benzimidazolyl, benzthiazolyl, purinyl, quinolizinyl, quinolinyl, isoquinolinyl, isothiazolyl, cinnolinyl, phthalazinyl, quinazolinyl, quinoxalinyl, naphthyridinyl, pteridinyl, and the like. Preferred heteroaryl groups include furanyl, thienyl, imidazolyl, pyridyl and isoquinolonyl.

As used herein, the term "heterocycloalkyl" shall denote any four to eight membered, preferably five to seven membered monocyclic, saturated or partially unsaturated ring structure containing at least one heteroatom selected from the group consisting of O, N and S, optionally containing one to three additional heteroatoms independently selected from the group consisting of O, N and S; or a nine to ten membered saturated, partially unsaturated or partially aromatic bicyclic ring system containing at least one heteroatom selected from the group consisting of O, N and S, optionally containing one to four additional heteroatoms independently selected from the group consisting of O, N and S. The heterocycloalkyl group may be attached at any heteroatom or carbon atom of the ring such that the result is a stable structure.

Examples of suitable heteroaryl groups include, but are not limited to, pyrrolinyl, pyrrolidinyl, dioxalanyl, imidazolinyl, imidazolidinyl, pyrazolinyl, pyrazolidinyl, piperidinyl, dioxanyl, morpholinyl, dithianyl, thiomorpholinyl, piperazinyl, trithianyl, indolinyl, chromenyl, 3,4-methylenedioxyphenyl, 2,3-dihydrobenzofuryl, 1,2,3,4-tetrahydroisoquinolinyl, hexamethyleneimine, and the like. Preferred heterocycloalkyl groups include pyrrolidinyl, morpholinyl, 1,2,3,4-tetrahydroisoquinolinyl, hexamethyleneimine and piperazinyl.

When a particular group is "substituted" (e.g., aryl, heterocycloalkyl, heteroaryl, and the like), that group may have one or more substituents, preferably from one to five substituents, more preferably from one to three substituents, most preferably from one to two substituents, independently selected from the list of substituents.

With reference to substituents, the term "independently" means that when more than one of such substituents is possible, such substituents may be the same or different from each other.

Under standard nomenclature used throughout this disclosure, the terminal portion of the designated side chain is described first, followed by the adjacent functionality toward the point of attachment. Thus, for example, a
"phenylC₁-C₆alkylaminocarbonylC₁-C₆alkyl" substituent refers to a group of the formula

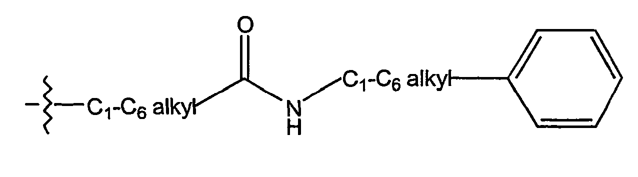

For the purposes of this invention, the pyrimidine ring system shall have the following numbering.

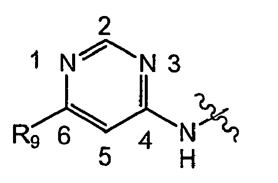

The compounds contained in the instant pharmaceutical compositions are exemplified in the Experimental Details section below. Compounds of formula (II) and (IV) are commercially available from BioFocus PCC (UK) as part of a chemical library. Alternatively, the compounds of formula (II) and (IV) exemplified below may be prepared using known methods.

As used herein, the phrase "pharmaceutically acceptable salt" means a salt of the free base which possesses the desired pharmacological activity of the free base and which is neither biologically nor otherwise undesirable. These salts may be derived from inorganic or organic acids. Examples of inorganic acids are hydrochloric acid, nitric acid, hydrobromic acid, sulfuric acid, and phosphoric acid. Examples of organic acids are acetic acid, propionic acid, glycolic acid, lactic acid, pyruvic acid, malonic acid, succinic acid, malic acid, maleic acid, fumaric acid, tartaric acid, citric acid, benzoic acid, cinnamic acid, mandelic acid, methanesulfonic acid, ethanesulfonic acid, p-toluenesulfonic acid, methyl sulfonic acid, salicyclic acid and the like.

The pharmaceutical compositions of the instant invention may be prepared according to conventional pharmaceutical techniques. The pharmaceutically acceptable carrier therein may take a wide variety of forms depending on the form of preparation desired for administration, such as systemic administration, including but not limited to intravenous, oral, nasal or parenteral. In preparing the compositions in oral dosage form, any of the usual pharmaceutical carriers may be employed, such as water, glycols, oils, alcohols, flavoring agents, preservatives, coloring agents, syrup and the like in the case of oral liquid preparations (for example, suspensions, elixirs and solutions), or carriers such as starches, sugars, diluents, granulating agents, lubricants, binders, disintegrating agents and,the like in the case of oral solid preparations (for example, powders, capsules and tablets).

Because of their ease of administration, tablets and capsules represent an advantageous oral dosage unit form, wherein solid pharmaceutical carriers are employed. If desired, tablets may be sugar-coated or enteric-coated by standard techniques. For parenterals, the carrier will usually comprise sterile water, though other ingredients for solubility or preservative purposes may also be included. Injectable suspensions may also be prepared, wherein appropriate liquid carriers, suspending agents and the like may be employed. The compounds may also be administered in the form of an aerosol.

The present invention also provides the use of a prophylactically effective amount of the compound contained in any of the instant pharmaceutical compositions of the invention for the manufacture of a medicament for reducing ischemic death in a cell population.

The present invention also provides an ex vivo a method for reducing ischemic death in a cell population comprising contacting the cell with a prophylactically effective amount of the compound contained in any of the instant pharmaceutical compositions of the invention.

As used herein, contacting a cell with an agent *"ex vivo"* includes, by way of example, contacting such agent with a cell that is part of an organized tissue or organ maintained outside the body of the subject from which it originates.

As used herein, a "prophylactically effective amount" of the instant pharmaceutical composition, or compound therein, means an amount that reduces the incidence of cell death in a population of cells. The instant pharmaceutical composition will generally contain a per dosage unit (e.g., tablet, capsule, powder, injection, teaspoonful and the like) from about 0.001 to about 100 mg/kg. In one embodiment, the instant pharmaceutical composition contains a per dosage unit of from about 0.01 to about 50 mg/kg of compound, and preferably from about 0.05 to about 20 mg/kg. Methods are known in the art for determining prophylactically effective doses for the instant pharmaceutical composition. The effective dose for administering the pharmaceutical composition to a human, for example, can be determined mathematically from the results of animal studies.

A "cell population" as used herein refers to cells in vitro such as in a culture vessel or in vivo as part of a body fluid or as an intact tissue or organ. The cell population can be homogenous (comprising of one cell type) or heterogenous (comprising a mixed cell type population). Preferred cell populations are heterogenous cell populations that comprise at least one cell type that has been identified as being protected from ischaemic death in the presence of the compounds of this invention.

In one embodiment of the instant method or use, the cells making up the cell populations are preferably mammalian cells and more preferably human cells. The cells that make up a cell population that demonstrates reduced ischemic injury in response to a traumatic invent include, but are not limited to, cell populations comprising at least one cell selected from the group consisting of a neuronal cell, a glial cell, a cardiac cell, a lymphocyte, a macrophage and a fibroblast. In the preferred embodiment, the cell is a neuronal cell.

The present invention also provides the use of a prophylactically effective amount of the compound contained in any of the instant pharmaceutical compositions of the invention for the manufacture of a medicament for reducing neuronal cell death in response to a traumatic event, wherein said medicament is adapted for administration prior to, during, or within a suitable time period following the traumatic event.

The present invention also provides an ex vivo method for reducing neuronal cell death in response to a traumatic event comprising contacting the neuronal cell with a prophylactically effective amount of the compound contained in any of the instant pharmaceutical compositions of the invention prior to, during, or within a suitable time period following the traumatic event.

The present invention further provides the use of a prophylactically effective amount of any of the instant pharmaceutical compositions of the invention for the manufacture of a medicament for reducing neuronal cell death in response to a traumatic event in a subject, wherein said medicament is adapted for administration prior to, during, or within a suitable time period following the traumatic event. The term "subject" includes, without limitation, any animal or artificially modified animal. In the preferred embodiment, the subject is a human.

The route of administering any of the instant pharmaceutical compositions to a subject is preferably systemic, including, for example, intravenous (iv), subcutaneous (sc) and oral administration. In one embodiment, the instant composition is administrated directly to the nervous system. This administration route includes, but is not limited to, the intracerebral, intraventricular, intracerebroventricular, intrathecal, intracistemal, intraspinal and/or peri-spinal routes of administration, which can employ intracranial and intravertebral needles, and catheters with or without pump devices. Infusion doses can range, for example, from about 1.0 to 1.0 x 10⁴ µg/kg/min of instant compound, over a period ranging from several minutes to several days. For topical administration, the instant compound can be mixed with a pharmaceutical carrier at a concentration of, for example, about 0.1 to about 10% of drug to vehicle.

In the instant method or use, the neuronal cell death-causing traumatic event includes, for example, a medical disorder, a physical trauma, a chemical trauma or a biological trauma.

Examples of neuronal cell death-causing medical disorders include, but are not limited to, perinatal hypoxic-ischemic injury, cardiac arrest, stroke/ischemic attack, hypoglycemia-induced neuropathy, cardiac surgery-induced cerebral ischemia, post traumatic stress disorder, stress-induced memory impairment, chronic epilepsy, multiple sclerosis, Parkinson's disease, diabetic peripheral neuropathy, neuropathic pain, Bells' palsy, sick sinus syndrome, Alzheimer's disease, Pick's disease, diffuse Lewy body disease, Cruzfeld's Jacobs and other diseases of protein aggregation, progressive supranuclear palsy (Steel-Richardson syndrome), multisystem degeneration (Shy-Drager syndrome), amyotrophic lateral sclerosis (ALS), degenerative ataxias, cortical basal degeneration, ALS-Parkinson's-Dementia complex of Guam, subacute sclerosing panencephalitis, Huntington's disease, synucleinopathies (including multiple system atrophy), primary progressive aphasia, striatonigral degeneration, Machado-Joseph disease, spinocerebellar ataxia type 3, olivopontocerebellar degenerations, Gilles De La Tourette's disease, bulbar and pseudobulbar palsy, spinal and spinobulbar muscular atrophy (Kennedy's disease), primary lateral sclerosis, familial spastic paraplegia, Werdnig-Hoffinann disease, Kugelberg-Welander disease, Tay-Sach's disease, Sandhoff disease, familial spastic disease, neuroleptic malignant syndrome, Wohlfart-Kugelberg-Welander disease, spastic paraparesis, progressive multifocal leukoencephalopathy, AIDS-related dementia, sick sinus syndrome, post herpetic neuropathy, viral meningitis, bacterial meningitis, prion diseases, and familial dysautonomia (Riley-Day syndrome).

Neuronal cell death-causing physical traumas include, for example, focal brain trauma, diffuse brain trauma, spinal cord injury, cerebral infarction, embolic occlusion, thrombotic occlusion, reperfusion, intracranial hemorrhage, whiplash, shaken infant syndrome, and radiation-induced peripheral nerve damage.

Neuronal cell death-causing chemical traumas include, for example, exposure to alcohol, chemotherapeutic agents, war gas, lead, cyanoacrylate, polyacrylamide, and toxic inhalants. Finally, neuronal cell death-causing biological traumas include, for example, exposure to HIV, herpes virus, and meningitis-causing bacteria and viruses.

In practicing the instant method or use, the pharmaceutical composition can be administered to the subject prior to, during or subsequent to the traumatic event. As used herein the term "subsequent" refers to any point in time beginning with the traumatic event and continuing until the potential of cell death resulting from the traumatic event has diminished.

Finally, the present invention provides an apparatus for administering to a subject any of the instant pharmaceutical compositions comprising a container and the pharmaceutical composition therein, wherein the container has a device for delivering to the subject a prophylactic dose of the pharmaceutical composition. In the preferred embodiment, the device for delivering the pharmaceutical composition is a syringe. Ideally, the instant apparatus is a single-use, predosed auto-injectable device containing the instant composition. Such a device would be useful, for example, in a mobile ambulatory unit or for administration to a person at risk for a neurotoxic event. Mechanical auto-injectable devices are well known in the art and are exemplified, for example, by an EpiPen® device (Meridian Medical Technologies Inc.), which is an auto-injectable device containing epinephrin for individuals subject to anaphalactic shock.

This invention will be better understood by reference to the Experimental Details that follow, but those skilled in the art will readily appreciate that these are only illustrative of the invention as described more fully in the claims which follow thereafter. Additionally, throughout this application, various publications are cited. The disclosure of these publications is hereby incorporated by reference into this application to describe more fully the state of the art to which this invention pertains.

### Experimental Details

### Example 1

### Commercially Available 4-Pyrimidinamines

| | |
|---|---|
| 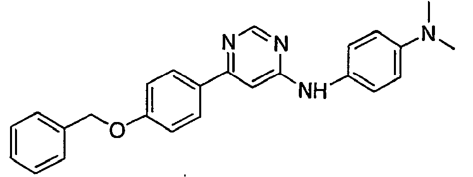 | 1,4-benzenediamine, *N*¹,*N*¹-dimethyl-*N*⁴-[6-[4-(phenylmethoxy)phenyl]-4-pyrimidinyl]- (Cmpd 42) |
| 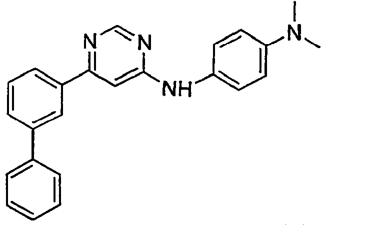 | 1,4-benzenediam.i.ne, *N*¹-(6-[1,1'-biphenyl]-3-yl-4-pyrimidinyl)-*N*⁴,*N*⁴-dimethyl- (Cmpd 43) |
| 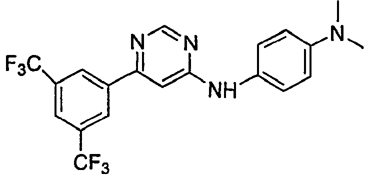 | 1,4-benzenediamine, *N¹-*[6-[3,5-bis(trifluoromethyl)phenyl]-4-pyrimidinyl]-N⁴,N⁴ -dimethyl-(Cmpd 44) |
| 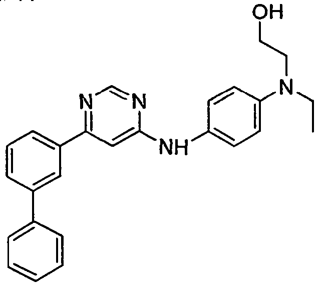 | ethanol, 2-[[4-[(6-[1,1'-biphenyl]-3-yl-4-pyrimidinyl)amino]phenyl] ethylamino]- (Cmpd 46) |
| 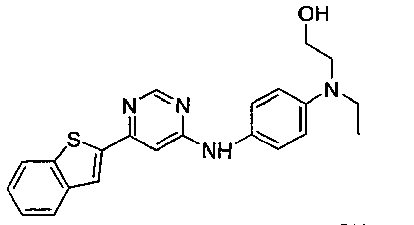 | ethanol, 2-[[4-[(6-benzo[b]thien-2-yl-4-pyrimidinyl)amino]phenyl] ethylamino]- (Cmpd 54) |
| 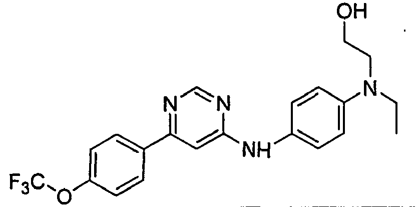 | ethanol, 2-[ethyl[4-[[6-[4-(trifluoromethoxy)phenyl]-4-pyrimidinyl] amino]phenyl] amino]-(Cmpd 51) |
| 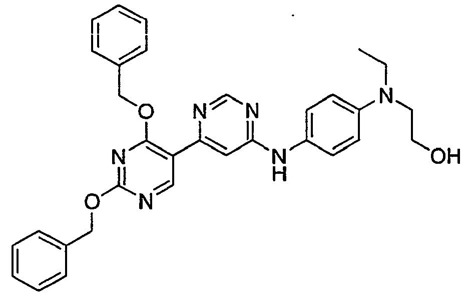 | 2- {[4-(2',4'-is-benzyloxy-[4,5 ']bipyrimidinyi-6-ylaniino)-phenyl]-ethyl-amino}-ethanol (Cmpd 58) |

### Example 2

### Characterization of Differentiated P19 Cells

### P19 Cell Differentiation

P19 cells are a pluripotent embryonal carcinoma line that can be induced to differentiate relatively rapidly into post-mitotic neurons in the presence of high dose retinoic acid (Jones-Velleneuve et al. 1982; Jones-Villeneuve et al. 1983; McBurney and Rogers 1982). They are the murine equivalent of human NT-2N neurons, which are also derived from retinoic acid differentiation of teratocarcinoma precursor cells. Differentiated NT-2N neurons, perhaps the better known of the two teratocarcinoma-derived neuronal lines, express a wide variety of neuronal markers, and undergo NMDA receptor-mediated, hypoxia-induced excitotoxic cell death (Pleasure and Lee 1993; Pleasure, Page, and Lee 1992; Rootwelt et al. 1998). Like NT-2Ns, differentiated P19 neurons also express a wide variety of neuronal markers, exhibit NMDA receptor-mediated intracellular calcium responses to agonists, and undergo excitotoxicity (Canzoniero et al. 1996; Grobin et al. 1999; Morley et al. 1995; Ray and Gottlieb 1993; Turetsky et al. 1993).

P19 cells were bought from ATCC (Manassas, VA). They were grown on 150 cm² tissue culture flasks in Dulbecco's Modified Eagle Medium (DMEM, Gibco BRL) supplemented with 10% fetal bovine serum, glutamine (2 mM), sodium pyruvate (1 mM), sodium bicarbonate (0.15% w/v), and penicillin/streptomycin (50 units/mL) in an atmosphere of 5% CO₂ at 37°C.

On day 1 of the differentiation protocol, confluent P19 cells were split to 50-70% confluency in growth medium. On day 2 of the protocol, 10 µM all-trans-retinoic acid (ATRA, Sigma) and 10 µM MK-801 were added to the growth medium. 10 µM MK-801 was included at this stage to prevent cell death in differentiating neurons that begin to express NMDA receptors. On day 4, fresh growth medium was placed on the cells, with fresh ATRA and MK-801. On day 5, cells were dissociated from the tissue culture flask by washing 4 times with calcium and magnesium-free phosphate-buffered saline, and adding 4 mL of non-enzymatic cell dissociation solution (Sigma).

Once dissociated, cells were placed in 40 mLs of differentiation medium. Differentiation medium consisted of Neurobasal medium (Gibco BRL) supplemented with 1 % N-2 supplement (Gibco BRL), 0.1 % trace elements B (Mediatech), 1 mM cadmium sulfate (Sigma), 2 mM glutamine, sodium pyruvate (1 mM), sodium bicarbonate (0.15% w/v), and 1% antibioticlantimycotic (Gibco BRL). 10 µM cytosine-D-arabinofuranoside was added to the differentiation medium to prevent growth of undifferentiated cells. No MK-801 was present from this point onward. Cells were triturated 20 times, then were split 1:4 into 96 well plates, or split 1:3 into 100 mm tissue culture dishes. Four days after replating, the cells were optimal for compound addition, and were assayed 24 hours later.

### RT-PCR

Total RNA was isolated from 100 mm tissue culture dishes of differentiated P19 neurons or undifferentiated P 19 cells using the QIAGEN RNeasy Mini kit according to manufacturer's protocols. RT-PCR amplification of murine NMDA receptor subunits was obtained from 250 ng total RNA template isolated from undifferentiated P 19 cells, cells at 4 days after retinoic acid (ATRA) induction, and cells at 9 days after ATRA induction. One-step RT-PCR reactions were set up using the LightCycler^{™}-RNA Amplification kit SYBR Green I kit (Boehringer Mannheim), according to manufacturer's protocols. Real time RT-PCR reactions were carried out in LightCycler^{™} glass capillaries using the LightCycler^{™} instrument and 250 ng template RNA (Boehringer Mannheim). The reverse transcriptase reaction was carried out for 10 min at 55°C. PCR was carried out for 30 cycles: annealing temperature was 50°C, extension temperature was 72°C, and melting temperature was 80°C. Reactions were compared to an H₂O-negative control for each primer set. 5 µL of reaction product were removed, and run on 1x TBE agarose gels. The primer sets for the various mouse NMDA receptor subunits used include zeta 1 and epsilons 1-4.

RNA samples from 4-day and 9-day post retinoic acid treatmnet, undifferentiated smaples and control sampes were separated by electrophoresis and probed with zeta1, epslon 1 and epsilon 2 internal primers

RT-PCR of NMDA receptor subunits from total RNA samples revealed that retinoic acid induction of differentiation also induces mRNA expression of zeta1, epsilon1, and epsilon2 mRNAs. Data was summarized using 3 separate experiments.

### Western Blots

Media was aspirated from cells plated onto 100 mm tissue culture dishes. Cells were harvested in RIPA lysis buffer (100mM Tris HCL pH 7.5, 150 mM NaCl, 1 mM EDTA, 1% Triton X-100,10% sodium deoxycholate, 0.1 % sodium dodecyl sulfate). Each sample was sonicated for 20 seconds, Laemmli sample buffer (BioRad) was added to a final 1x concentration, and samples were incubated for 10 minutes at 95°C. Samples to be probed with NMDA receptor antibodies (polyclonals against rat NR1, NR2A, and NR2B obtained from Chemicon) were electrophoresed on 6% tris-glycine pre-cast gels (NOVEX). Samples to be probed with p42/44 MAP kinase antibodies (New England BioLabs) were electrophoresed on 12% tris-glycine pre-cast gels (NOVEX). Electrophoresis was carried out in a NOVEX apparatus for 1.5 hours at 200 volts. Proteins were transferred to polyvinylidene difluoride membrane (PVDF, NOVEX) using a BioRad wet transfer device for 1 hour at 100 volts. Prior to transfer, PVDF membranes were dipped in 100% methanol for 1 minute, then soaked in transfer buffer for 5 minutes. After transfer, membranes were removed and were slowly shaken in blocking solution (5% milk, 0.05% tween-20 in phosphate buffered saline) at 4°C overnight. Membranes were then washed once with PBS-tween, and primary antibodies 1:1000 in PBS-tween with 5 % milk were incubated for 1 hour at room temperature. Membranes were washed 4x for 15 minutes at room temperature. Secondary antibodies coupled to horseradish peroxidase were incubated for about 45 minutes at room temperature in PBS-tween with 5 % milk. Membranes were then washed 4x for 15 minutes at room temperature. Blots were developed using ECL plus (Amersham), and exposed to film.

Western blot analysis was performed for NMDA receptor subunit protein in P19 cell lysates harvested following 4-days or 9-days of retinoic acid exposure. Appropriately sized bands (Zeta1 = ~120 kDa, Epsilon1 and Epsilon 2 = ~ 180 kDa) were detectable from terminally differentiated P19 neuron lysates, but not from undifferentiated cell lysates. The data was representative of 3 separate experiments with similar results.

Western blot analysis revealed that terminally differentiated P 19 neurons express detectable levels of zeta1, epsilon1, and epsilon 2 proteins. Epsilon3 and epsilon4 mRNAs or proteins were not detectable at any time in these cells.

These data demonstrate that such methods of differentiating P19 cells into neurons successfully result in the functional expression of NMDA receptors at the levels of mRNA, protein, MK-801 sensitive agonist-induced intracellular calcium responses, and MK-801 sensitive glutamate toxicity. These data are in very good agreement with the literature. Interestingly, however, in addition to zeta1 and epsilon2 expression, reliable expression of the epsilon1 subunit of the NMDA receptor was also achieved, which expression has not yet been achieved with other reported methods of P19 cell differentiation (Ray and Gottlieb 1993). The presence of all three subunits more closely resembles expression patterns observed in adult rodent forebrain regions, including cortex and hippocampus (Ishii et al. 1993; Laurie et al. 1995; Monyer et al. 1994; Monyer et al. 1992; Standaert et al. 1994).

### Example 3

### Differentiated P19 Excitotoxicity Assay

Cells were loaded with 5 µM Fura-2-AM (Molecular Probes) for 1 hr at 37°C. They were washed once with Hank's balanced salt solution (HBSS, Gibco BRL), and assayed in HBSS buffer. Cells were placed onto the stage of a modified ATTOFLUOR^{™} Imager (Atto Instruments, Rockville Pike, MD). High speed, dual excitation of fura-2 was carried out using a RATIOARC^{™} High-Speed Excitor (Atto Instruments). Mercury lamp light was passed through 334 nm or 380 nm bandpass filters (10 nm band width), and then passed through a 20x objective (Zeiss, Plan-Apochromat, NA=0.75) at a rate of 2.5 Hz. Emitted light was transmitted through a 400 nm dichroic mirror, and collected to an ATTOFLUOR^{™} intensified CCD camera Ratio-images were acquired, and the average intensity of the images when excited at 334nm and 380nm was analyzed using ATTOFLUOR RATIOVISION^{™} software (Atto Instruments, Rochville, MD).

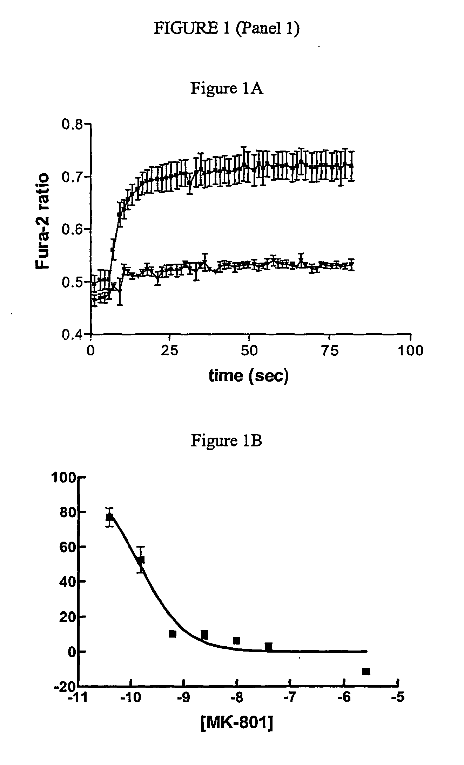

Changes of the Fura-2 330nm/380nm intensity ratio were plotted when 9 days post-ATRA P19 neurons were treated with 3 mM glutamate, 1 mM glycine in the presence or absence of 100 µM MK-801. MK-801 was administered 24 hours prior to assay. Traces are the average ± standard error from three separate experiments for each condition. Terminally differentiated P19 neurons expressed NMDA receptor subunits that form functional NMDA receptors, as illustrated by using fura-2 imaging to detect increases in intracellular calcium levels upon addition of NMDA receptor agonists (Figure 1A). The selective NMDA receptor channel blocker, MK-801, significantly inhibited this response (Figure 1A). In addition P19 neurons expressed specific binding sites for the NMDA receptor. MK-801 inhibition of [³H]-MK-801 binding was assayed in P19 membranes. MK-801 concentrations are expressed as 10^{-x} Molar. Data points represent the mean ± standard error from eight experiments. % control = ((CPM - CPM_{bottom})/(CPMₜₒₚ-CPM_{bottom}) X 100. (Figure 1B). MK-801 inhibition was concentration-dependent, and 100% inhibition was achieved.

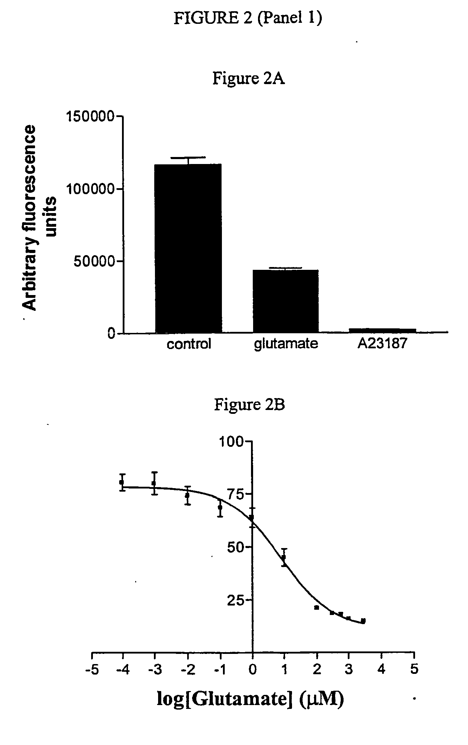

Control P19 neuron cell bodies and processes stain brightly with the live cell cytoplasmic dye carboxyfluorescein diacetate (CFDA). P 19 neurons at 9 days post ATRA induction were labeled with CFDA, and then imaged in confocal mode using an Attofluor Imager. Control cells were treated with vehicle for 24 hours, glutamate cells received 3 mM glutamate in the presence of 1 mM glycine for 24 hours, and glutamate + U0126 cells received 10 µM U0126 concurrent with 3 mM glutamate and 1 mM glycine for 24 hours. Images were taken from control alive, glutamate treated, dead and U0126-protected cells stained with fluorescein diacetate in 3 separate experiments. P19 neuron cell bodies characteristically clumped together into tight aggregates when plated onto plain tissue culture plastic. Networks of extensive processes connected clusters of neuronal cell bodies. P19 neurons treated with toxic concentrations of glutamate and glycine for 24 hours exhibited fluorescence staining in isolated cell bodies, processes were undetectable, and extensive cellular debris was evident. Relative levels of cell death were measured rapidly and quantitatively on a plate reader using the live cell fluorescent dye alamar blue (Figures 2B).

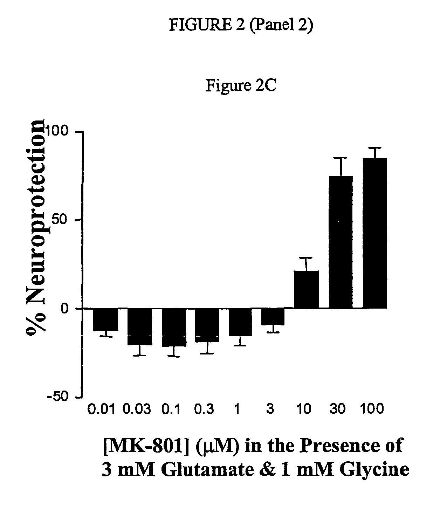

Alamar Blue fluorescence, an indicator of cell viability, was used to determine cell viability after an NMDA-induced cytotoxic insult. Counts from a single 96 well plate where 32 wells received vehicle control, 32 wells received 3 mM glutamate and 1 mM glycine for 24 hours, and 32 wells received 5 µM A23187 for 24 hours are shown in Figure 2A. Glutamate and A23187 conditions were significantly different from control as determined by one-way ANOVA with Tukey post-hoc analysis carried out using GRAPHPAD^{™} software. These data indicate a typical 60% reduction in Alamar blue fluorescence when cells were treated with glutamate and glycine. However, since raw fluorescence counts vary from experiment to experiment, Figures 2A, 2B, and 2C are expressed as a percent of control.

Glutamate toxicity dose response in the presence of a constant 1 mM glycine concentration was measured in the differentiated P19 neurons. The curve generated through the data points is the average of 6 separate dose response curves. Data points are represented as the percent of control cells ± standard error. % control = (([Glutamate]ₑₓₚ - [Glutamate]ₘₐₓ) / (Control_{vehicle} - [Glutamate]ₘₐₓ)) X 100. Glutamate toxicity EC50 was calculated to be 8.1 µM [lower 95% confidence interval = 3.5 µM; upper 95% confidence interval =19 µM]. These data demonstrate that 24 hours of glutamate treatment killed P19 neurons dose-dependently in the presence of a constant dose of glycine (Figures 2A,2B). Glycine alone did not affect P19 neuron viability.

The NMDA receptor blocker, MK-801, dose-dependently protected against glutamate toxicity in P19 neurons (Figure 2C). MK-801 dose-dependent inhibition of 3 mM glutamate and 1 mM glycine-induced P19 neuron death. % Neuroprotection = ((Inhibitor in the presence of Glutamateₘₐₓ - [Glutamate]ₘₐₓ) / (Control_{vehicle} - [Glutamate]ₘₐₓ)) X 100. Data points are the average ± standard error of three separate experiments. Maximal MK-801 protection achieved was close to control levels.

These data demonstrate that glutamate toxicity in P19 neurons requires NMDA receptor activation, since the toxicity is completely blocked in the presence of the specific NMDA receptor antagonist MK-801. However, the data do not exclude the possibility that AMPA or kainate receptors may also be activated by glutamate and contribute to the excitotoxicity. This possibility would be consistent with data from primary neuronal culture, which reports that intracellular calcium responses to glutamate agonists involve multiple components (Courtney, Lambert, and Nicholls 1990). Such components include AMPA/kainate receptor activation, membrane depolarization, voltage-gated calcium channel activation, relief of N1VIDA receptor magnesium block, and NMDA receptor activation. However, even with such a high level of complexity, in many primary neuronal models, glutamate excitotoxicity signals through the NMDA receptor and requires its activation to result in neuronal death (Tymianski et al. 1993), as is the case for P19 neuron model system.

These cells were used to measure the effects of various known kinase inhibitors (Compound Identification (ID) column in Table I) upon cytotoxicity-induced by NMDA. First, P19 cells were differentiated as described in Example 2. Then cells were exposed to 3 mM of glutamate in the presence of 1 mM glycine.

Cell viability was determined. Compounds that prevented excitotoxicity were determined by measuring the percentage of viable cells compared to differentiated P19 cells that were not presented with a toxic insult (Table I).

Cell viability was measured using two methods. The first method was a confocal, single-cell fluorescence imaging-based method using the live cell dye carboxy-fluorescein diacetate (CFDA). CFDA labels the cell bodies and processes of living cells. Therefore, live cells exhibit extensive CFDA labeling, whereas dead cells exhibit much less CFDA staining. CFDA fluorescence was measured using a modified Attofluor Imager device (Atto Instruments, Rockville Pike, MD). Cells were labeled with 1 uM CFDA for 15 min in media, then placed onto the stage of the Attofluor microscope. The dye was excited by light from a mercury lamp source passed through a 488 nm bandpass filter of 10 nm band width, passed through a CARV real-time confocal spinning disk module (Atto Instruments, Rockville, MD), and then passed through a 40x oil immersion objective (Zeiss Fluar, NA=1.3). Emitted light was transmitted through a 495 nm dichroic mirror, collected to an Attofluor intensified CCD camera, and images were visualized using Attofluor Ratio Vision software (Atto Instruments, Rockville, MD).

The second method for measuring cell viability was a plate reader method. Cells plated into black 96 well plates (Packard viewplates) were loaded with 5 % Alamar Blue dye (Biosource International). Alamar Blue is a dye that takes advantage of mitochondrial reductases to convert non-fluorescent resazurin to fluorescent resorufin (excitation 535 nm, emission 580 nm). Baseline fluorescence counts were read at room temperature in a Wallac plate reader immediately after addition of Alamar blue. Fluorescence counts of cell viability were taken the same way after 1 hour incubation at 37°C. Fluorescence was expressed as a percent of control, untreated cells after subtraction of background fluorescence. Live/dead cells were confirmed visually with a light microscope.

As used in Table I below, Compound A shall mean the compound have the formula

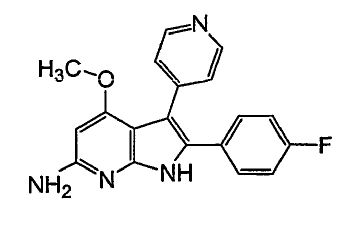

| Table I | | | | | |
|---|---|---|---|---|---|
| Comparison of Kinase Inhibitor Activity to Neuroprotective Activity | | | | | |
| Compound ID | Enzyme target | Activity | Potency | IC50 for Neuroprotection [lower-upper 95% C.I.] | Maximal Efficacy for Neuroprotection |
| Compound A | p38 kinase | Inhibitor | IC50~10 nM | 9.7 uµM [5.8-16.3] | >80% |
| U0126 | MEK1/2 | Inhibitor | IC50~0.5 uM | 1.1 µM [0.74-1.7] | >80% |
| SB202474 | p38 kinase | Inactive | Nonapplicable | >10 µM | >50% |
| SB203580 | p38 binase | Inhibitor | IC50~600 nM | Ineffective | <10% |
| Lithium | IP3 turnover | Inhibitor | Ki~0.5 µM | Ineffective | <10% |
| KN62 | CAMKII | Inhibitor | Ki~900 nM | Ineffective | <10% |
| Calphostin C | PKC | Inhibitor | IC50~50 nM | | |
| | PKA | Inhibitor | IC50>50 µM | >1 µM | ~50% |
| | PKG | Inhibitor | IC50>25 µM | | |
| | p60^{c-src} | Inhibitor | IC50>50 µM | | |

| Lavendustin A | EGF receptor p60^{c-src} | Inhibitor | IC50~11 nM IC50~500 nM | >10 µM | <25% |
|---|---|---|---|---|---|
| H-89 | PKA | Inhibitor | Ki~48nM | >10 µM | <25% |
| | CAMKIII | Inhibitor | Ki~30 µM | | |
| | Casein kinase I | Inhibitor | Ki~38.µM | | |
| | PKC | Inhibitor | Ki~31.7µM | | |

Kinase inhibition activities and potencies were derived from the literature (Chijiwa et al. 1990; Favata et al. 1998; Henry et al. 1998; Inhorn and Majerus 1987; Kobayashi et al. 1989; Lee et al. 1994; Onoda et al. 1989; Tokumitsu et al. 1990). IC50s for neuroprotection are the mean of three separate curves with upper and lower 95% confidence intervals (C.I.) shown. Curves were fit, and confidence intervals were determined using GraphPad Prism software.

### Example 4

### Evaluation of a MEK1/2 Inhibitor in the Differentiated P19 Assay

U0126 (bis[amino[(2-aminophenyl)thio]methylene] Butanedinitrile) is reported to be highly selective for MEK1/2 (Favata et al. 1998), a result that was confirmed here. The only other kinase found that U0126 inhibits is PKC-γ, but the IC50 for inhibition of this enzyme was 60-fold higher that its published IC50 against wildtype NMK1/2 (Tables II and III below).

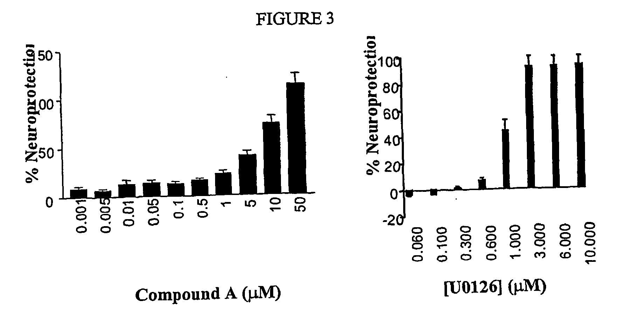

The general procedure used to assay for kinase activity was as follows: a kinase reaction mix was prepared in 50 mM Tris-HCl pH=8,10 mM MgCl₂, 0.1 mM Na₃VO₄, 1 mM DTT, 10 µM ATP, 0.25-1 µM biotinylated peptide substrate, 0.2-0.8 µCuries per well ³³P-γ-ATP [2000-3000 Ci/mmol]. Assay conditions varied slightly for each protein kinase, for example, insulin receptor kinase requires 10 mM N4mCl₂ for activity and calmodulin-dependent protein kinase requires calmodulin and 10 mM CaCl₂. Reaction mix was dispensed into the wells of a streptavidin-coated Flashplate and 1 µl drug stock in 100% DMSO was added to a 100 µL reaction volume resulting in a final concentration of 1% DMSO in the reaction. Enzyme was diluted in 50 mM Tris-HCl pH=8.0, 0.1 % BSA and added to each well. The reaction was incubated for one hour at 30°C in the presence of compound. After one hour the reaction mix was aspirated from the plate and the plate washed with PBS containing 100 mM EDTA. The plate was read on a scintillation counter to determine ³³P-γ-ATP incorporated into the immobilized peptide. Test compounds were assayed in duplicate at 8 concentrations ranging from 100 µM to 10 pM in one order of magnitude steps. A maximum and minimum signal for the assay was determined on each plate. The IC50 was calculated from the dose response curve of the percent inhibition of the maximum signal in the assay according to the formula [max signal - background/test compound signal-background (100) = % inhibition] by graphing the percent inhibition against the log concentration of test compound. Known inhibitor compounds appropriate for the kinase being assayed were also included on each plate. Results are provided in Figure 3.

### Definition and Source of Kinase Enzymes

VEGF-R (vascular endothelial growth factor receptor-2) is a fusion protein containing a polyhistidine tag at the N-terminus followed by amino acids 785-1343 of the rat VEGF-R2 kinase domain. CDK1 (cyclin dependent kinase 1) is isolated from insect cells expressing both the human CDK1 catalytic subunit and its positive regulatory subunit cyclin B. Insulin Receptor Kinase consists of residues 941-1313 of the cytoplasmic domain of the beta-subunit of the human insulin receptor. Protein Kinase A is the catalytic subunit of cAMP-dependent protein kinase-A purified from bovine heart. PKC (protein kinase-C) is the gamma or beta isoform of the human protein produced in insect cells. Casein Kinase 1 is a truncation at amino acid 318 of the C-terminal portion of the rat CK1 delta isoform produced in *E. coli.* Casein Kinase 2 includes the alpha and beta subunits of the human CK2 protein produced in *E. coli.* Calmodulin Kinase (calmodulin-dependent protein kinase 2) is a truncated version of the alpha subunit of the rat protein produced in insect cells. Glycogen Synthase Kinase-3 is the beta isoform of the rabbit enzyme produced in *E. coli.* MAP Kinase is the rat ERK-2 isoform containing a polyhistidine tag at the N-terminus produced in *E. coli* and activated by phosphorylation with MEK1 prior to purification. EGFR (epidermal growth factor receptor) is purified from human A431 cell membranes. The chart below shows selected kinases and their control inhibitors.

As used in Table III below, Compound A (Cmpd A) shall mean the compound have the formula

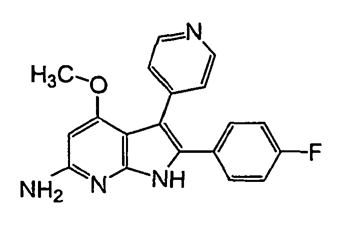

**TABLE II**

| Selected Kinases and Their Control Inhibitors | |
|---|---|
| Kinase | Control Inhibitor |
| CDK1 | Butyrolactone |
| EGFR | AG-1478 |
| Protein Kinase A | H89 |
| PKC | Staurosporine |
| Casein Kinase 1 | H89 |
| Casein Kinase 2 | |
| Calmodulin Kinase | Staurosporine |
| Insulin Kinase | Staurosporine |

| **Table III** | | | | |
|---|---|---|---|---|
| **Kinase Selectivity of Compound A and U0126** | | | | |
| **Compound ID** | **CDK1 (µM)** | **EGF-R (µM)** | **PK A (µM)** | **PKC-γ (µM)** |
| Cmpd A | >100 | >100 | >100 | 8.35 |
| U0126 | >100 | >100 | >100 | 29.5 |
| **Compound ID** | **Casein Kinase 1 (µM)** | **Casein Kinase 2 (µM)** | **Calmodulin Kinase (µM)** | **Insulin Receptor Kinase (µM)** |
| Cmpd A | 0.116 | >100 | >100 | >100 |
| U0126 | >100 | >100 | >100 | ND |

IC50 values for kinase inhibition are the mean of at least two separate curves, and were determined using GraphPad curve fitting software.

To determine whether U0126 inhibits the MEK1/2 enzymes in P19 neurons, its ability to block glutamate-induced phosphorylation of the MBK1/2 substrate p42 MAPK (ERK2) was tested. Western blot were first probed using ATRA P19 neuron lysates 9 days post treatment with an antibody specific for the phosphorylated form of p42/44 (ERK1/2) and then stripped and reprobed with antibody that recognized total p42/4.4 (ERK ½). The data demonstrate that the concentrations of glutamate and glycine that were used to induce toxicity in P19 neurons also induced a rapid, reproducible increase in p42 MAPK phosphorylation in these cells. In the presence of U0126, phospho-p42 MAPK was reduced, although no change in the amount of total p42 MAPK was evident. In another Western blot experiment, ATRA P19 neuron lysates were probed 9 days post treatment with an antibody specific for the phosphorylated form of p42/44 (ERK ½). The same blot was stripped and reprobed with an antibody that recognized total p42/44 (ERK ½). Glutamate-induced elevation of p42 MAPK phosphorylation was sustained, since it was still clearly increased at 24 h versus controls. These blots are representative of 3 separate experiments with similar results. U0126 blocked the increased levels of phosphorylation at this time point as well. No change in total p42 MAPK was evident.

To ensure that U0126 does not block the NMDA receptor directly, P19 neuron intracellular calcium responses to glutamate and glycine were tested in the presence of 10 µM U0126. Fura-2 imaging traces 9 days post ATRA P 19 neurons were treated with 3 mM glutamate, 1 mM glycine in the presence of 10 µM U0126 for 24 hours. U0126 was administered concurrently with glutamate and glycine. Traces are the average ± standard error from four separate experiments for each condition.

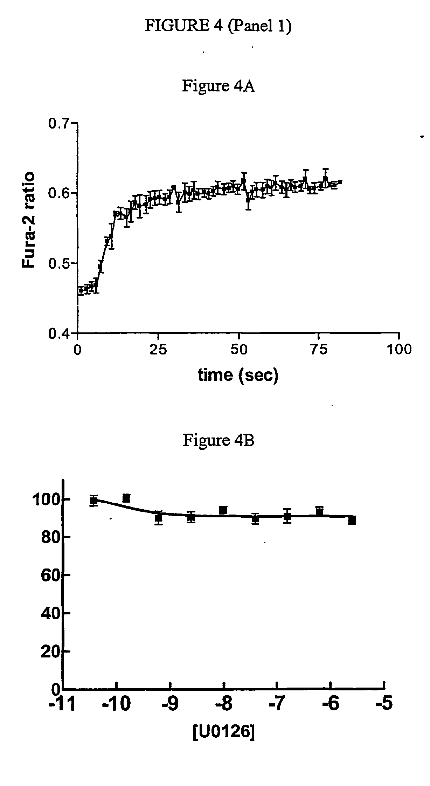

The data demonstrated that U0126 did not block this intracellular calcium response. (Figure 4A). It was also demonstrated that U0126 did not inhibit [3H]-MK-801 binding in P19 neurons. No significant inhibition was observed at any concentration of U0126. Data points represent the mean ± standard error from eight experiments. Percent control is defined as described in Figure 1B (Figure 4B). Together, these data further demonstrate that U0126 acts on a signaling pathway downstream of NMDA receptor activation.

### The p42/44 MAPK inhibitor, U0126, exhibits delayed neuroprotection

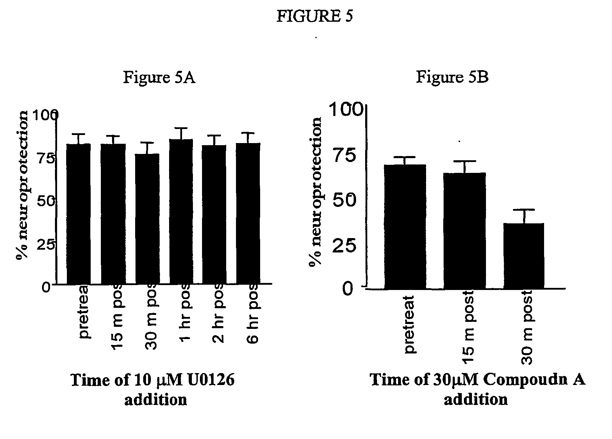

Time-course of efficacy is an extremely relevant parameter for a potential neuroprotective therapeutic agent, since the therapeutic would need to be administered hours to days after an ischemic event and still retain efficacy. The next set of experiments examined whether the MEK1/2 inhibitor, U0126, retains neuroprotective efficacy when added at various time points after glutamate challenge. Assays were performed essentially as described, although the time of administration of the U0126 was delayed relative to the initial excitotoxicity. The data demonstrate that U0126 was maximally neuroprotective up to six hours after glutamate challenge (Figure 5A). However a p38 inhibitor lost efficacy as soon as 15 minutes after glutamate challenge (Figure 5B). U0126 was maximally neuroprotective even when added several hours after the onset of glutamate challenge. This may be because glutamate mediates a sustained increase in p42 MAPK phosphorylation in P 19 neurons, detectable even at 24 hours post glutamate addition. U0126 inhibition of the upstream activating enzyme, MEK, several hours after glutamate challenge may favor phosphatase dephosphorylation of p42 MAPK, and restabilize the p42/44 MAPK signaling pathway within enough time to prevent cell death.

Thus, activation of the p42/44 MAP kinase pathway is necessary for glutamate-induced toxicity in differentiated P19 neurons. Glutamate-induced cell death occurs within 24 hours, is dose-dependent, and is NMDA receptor-mediated. Glutamate induces phosphorylation of p42 MAP kinase, which is blocked by U0126, an inhibitor of its upstream kinase, MEK. U0126 also blocks glutamate toxicity in a dose-dependent manner. It is effective when administered before, or even several hours after the onset of glutamate challenge.

A compound that is able to exert delayed neuroprotection even when added after an ischemic event is an especially sought after property of a potential stroke therapeutic. Many compounds with diverse mechanisms are reported to exhibit post-treatment delayed neuroprotection. Among these are glutamate receptor antagonists (Li et al. 1999b; Takahashi et al. 1998; Turski et al. 1998), antioxidants (Callaway et al. 1999; Pazos et al. 1999; Sakakibara et al. 2000), anticonvulsants (Schwartz-Bloom et al. 1998; Wasterlain et al. 1996; Yang et al. 1998), protease inhibitors (Cheng et al. 1998), kinase inhibitors (Tatlisumak et al. 1998), and magnesium (Heath and Vink 1999). However, this is the first demonstration in a cell culture model of NMDA receptor-mediated excitotoxicity wherein a specific inhibitor of the p42/44 MAP kinase pathway exhibits delayed neuroprotection, and wherein the time window of efficacy extended well after the onset of toxic challenge.

### U0126 Neuroprotection is Selective for Glutamate-Induced Toxicity.

To determine whether U0126 is protective against a variety of nonspecific toxic insults, or whether the efficacy of this compound is selective for glutamate-induced toxicity, its effects were tested against a variety of other inducers of P19 neuron death.

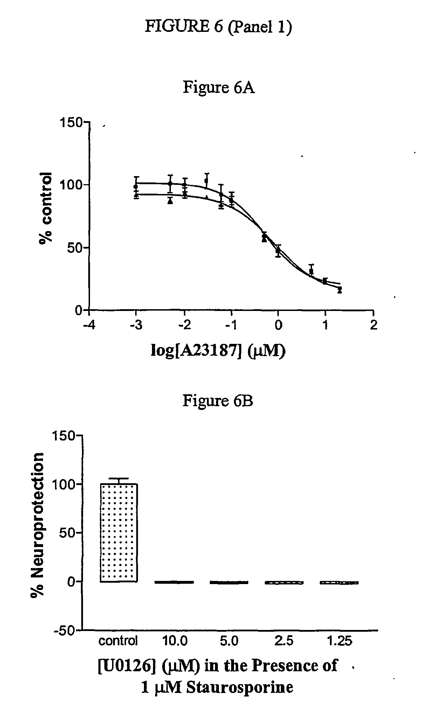

Figure 6A shows P19 neuron treatment with various concentrations of A23187 for 24 hours in the presence or absence of 10 µM U0126. Cells were then assayed for Alamar blue fluorescence. The curve generated through the data points is the average of 3 separate dose response curves. Data points are represented as percent of control cells ± standard error. The EC50 for A23187 toxicity in the absence of U0126 was calculated to be 520 nM [340 nM - 784 nM]. The EC50 for A23187 toxicity in the presence of 10 µM U0126 was calculated to be 833 nM [440 nM -1.6 µM].

Figure 6B shows that 1 µM staurosporine-induced P19 neuron toxicity could not be protected by 10 µM U0126, as measured by Alamar blue fluorescence at 24 hours after addition. Cells that received vehicle rather than staurosporine exhibited control levels of Alamar blue fluorescence. However no concentration of U0126 brought fluorescence back to control levels in staurosporine-treated cells.

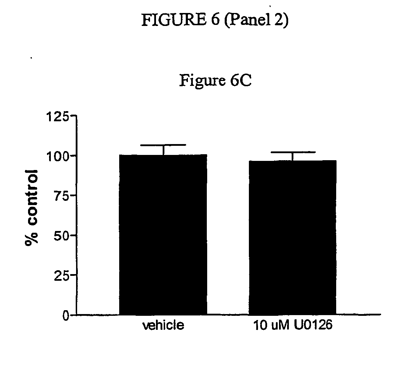

Figure 6C demonstrates Alamar blue fluorescence assayed on P19 neurons treated with vehicle or with 10 µM U0126 in the absence of any inducers of toxicity. U0126 alone did not affect these control levels of fluorescence. The data demonstrate that U0126 was not protective against staurosporine or A23187-induced death (Figures 6A,6B). Additionally, U0126 did not affect the basal viability of P19 neurons (Figure 6C).

### Example 5

### Drug Screening Using Differentiated P19 Cell Assay

Commercially available chemical libraries (BioFocus PLC, UK) were screened using the methods described herein. The stock concentration of the compounds was about 400 µM in DMSO. The concentration of compounds used in the primary screen was 1 µM. A positive compound was assigned as one that demonstrated 70% or greater neuroprotection. Dose-response testing was carried out in 96 well plates where column 1 was vehicle control, column 2 received 3 mM glutamate and 1 mM glycine, and column 3 received 3 mM glutamate, 1 mM glycine, and 10 µM U0126. Columns 4-11 received compounds at the following final concentrations (µM): 3, 1.2, 0.48, 0.192, 0.077, 0.031, 0.012 and 0.005. Each row contained a different compound for confirmation. Only rows B-G received compounds, and rows A and H remained untreated. Data are expressed as % neuroprotection = ((compound-glutamate average)/(U0126-g1utamate average) X 100).

Compounds of two genuses, 4-pyrimidinamines and 2-pyridinamines, were found to display neuroprotective properties as data here show. Only the 4-pyrimidinamines are the subject of this invention, although limited data for 2-pyridinamines are presented as well. The results of this biological testing are summarized in the following tables. "% Inh" indicates the percentage of control cells surviving after 24 hours, and represents the neuroprotective effect of the compounds screened at 1 micromolar concentration. IC50 relate to data from dose response experiments. IC50 values listed as >1 indicate no observed maximum within the highest dose tested (3 µM), yet indicate biological activity. ND refers to compounds not tested in dose-response experiments.

| | | | | | | |
|---|---|---|---|---|---|---|
| 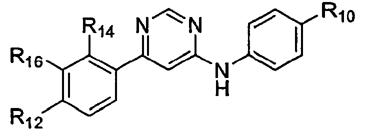 | | | | | | |

| Table IV | | | | | | |
|---|---|---|---|---|---|---|
| N,6-diphenyl-4-pyrimidinamine derivatives | | | | | | |
| Cmpd | R₁₀ | R₁₂ | R₁₆ | R₁₄ | IC₅₀ (µM | % Inh |
| 41 | (CH₃)₂N | fused to form naphthyl | | H | 0.54 | 72 |
| 42 | (CH₃)₂N | O(CH₂)Ph | H | H | 0.2 | 80 |
| 43 | (CH₃)₂N | H | Ph | H | 0.22 | 88 |
| 44 | (CH₃)₂N | H | 3,5(CF₃)₂ | H | 0.26 | 89 |
| 45 | 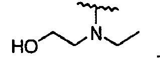 | fused to form naphthyl | | H | 0.57 | 93 |
| 46 | 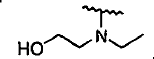 | H | Ph | H | 0.07 | 87 |
| 47 | 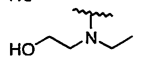 | Ph | H | H | 0.49 | 94 |
| 48 | 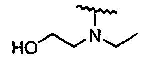 | H | 3,5(CF₃)₂ | H | 0.48 | 124 |
| 49 | 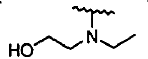 | H | NO₂ | H | 0.55 | 84 |
| 50 | 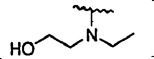 | SCH₃ | H | H | 0.41 | 132 |
| 51 | 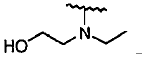 | OCF₃ | H | H | 0.23 | 144 |
| 52 | 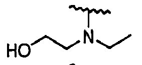 | fused to form naphthyl | | H | 0.57 | 106 |
| 56 | 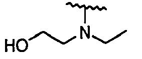 | H | H | PhO | 0.57 | 77 |
| 62 | (CH₃)₂N | CN | H | H | >1 | 175 |
| 63 | (CH₃)₂N | H | H | PhO | >1 | 115 |
| 64 | 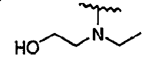 | CN | H | H | >1 | 106 |
| 65 | 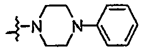 | OH | H | H | >1 | 80 |
| 66 | 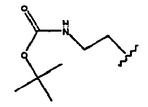 | fused to form naphthyl | | H | ND | 100 |
| 67 | (CH₃)₂N | H | H | PhCH₂ O | ND | 91 |
| 147 | 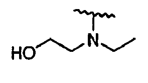 | 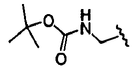 | H | H | 1.09 | 92 |
| 148 | 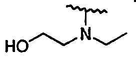 | H | (CH₃)₂N | H | 0.47 | 101 |
| 149 | (CH₃)₂N | (CH₃)₂N | H | H | 0.33 | 93 |

| | | | | |
|---|---|---|---|---|
| 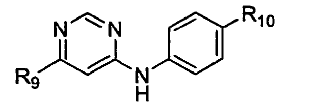 | | | | |

| Table V | | | | |
|---|---|---|---|---|
| N-phenyl-4-pyriridinamine derivatives | | | | |
| Cmpd | R₉ | R₁₀ | IC₅₀ (µM) | % Inh |
| 53 | 2,7a-dihydro-2-benzofuranyl | 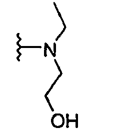 | 0.54 | 85.8 |
| 54 | 2,7a-dihydrobenzo-[b]thien-2-yl | 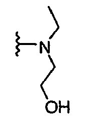 | 0.24 | 166 |
| 55 | 3-thienyl | 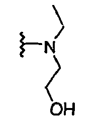 | 0.86 | 81,8 |
| 59 | 2,7a-dihydro-2-benzofuranyl | (CH₃)₂^{N} | 0.48 | 77 |
| 60 | 2,7a-dihydrobenzo-[b]thien-2-yl | (CH₃)₂^{N} | ND | 95 |
| 61 | 1-naphthyl | (CH₃)₂N | >1 | 131 |
| 150 | (3-thienyl) | CH₃CN | >1 | 95 |

| | | | |
|---|---|---|---|
| 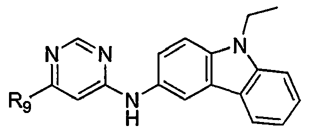 | | | |

| Table VI | | | |
|---|---|---|---|
| 9-ethyl-*N*-(4-pyrimidinyl)-9H carbazol-2-amine derivativess | | | |
| Cmpd | R₉ | IC₅₀ (µM) | % Inh |
| 151 | 3,4-(CH₃O)₂Ph | >1 | 101 |
| 152 | 3,4,5-(CH₃O)₃Ph | >1 | 80 |
| 153 | 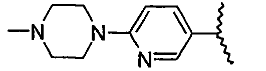 | ND | 72 |
| 154 | 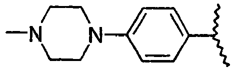 | 0.55 | 102 |

| | | | |
|---|---|---|---|
| 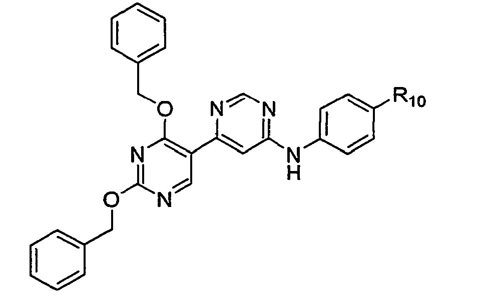 | | | |

| Table VII | | | |
|---|---|---|---|
| 6-(5-(2,4 diphenoxy)-pynmidme)N-phenyt-4-pyrimidmamme derivatives | | | |
| Cmpd | R₁₀ | IC₅₀ (µM) | % Inh |
| 57 | 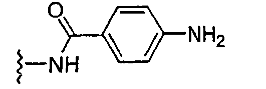 | 0.56 | 79 |
| 58 | 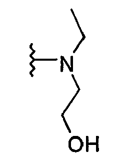 | 0.39 | 94 |

### Example 6

### 2-Pyridineamine and 4-Pyrimidineamine Derivatives Do Not Inhibit MEK Activity in P19 Neurons.

The commercially available 2-pyridinamine and 4-pyrimidinamine compounds tested here did not inhibit MEK1/2 kinase activity in P19 neurons since they did not inhibit glutamate-induced p44/42 MAP kinase phosphorylation in these cells. However U0126 did inhibit this activity as expected (Figure 8). MEK activity in P 19 neurons was induced by addition of 3 mM glutamate and 1 mM glycine, and measured by assaying for phosphorylation of the MEK substrate, p44/42 MAP kinase. Cells were treated with the compounds described herein or with U0126 in the presence or absence of glutamate/glycine, and harvested 5 minutes after treatment, for subsequent Western blot analysis. The data demonstrate that U0126 effectively inhibited phosphorylation of MEK substrate as expected, but that the 2-pyridinamine and 4-pyrimidinamine compounds did not. Therefore, the 2-pyridinamine and 4-pyrimidinamines are not MEK inhibitors.

The 2-pyridinamine and 4-pyrimidinamines were maximally neuroprotective at least two hours after the onset of excitotoxicity. Compounds were administered at a concentration of 1 µM either 15 minutes before or 2 hours after glutamate/glycine addition. U0126 was tested as a positive control. Similar to U0126, the 2-pyridinamine and 4-pyrimidinamine compounds retained maximal neuroprotective efficacy at both time points despite being active at a different target.

### Example 7

### In Vivo Model of Neurobrotection:

### Middle Cerebral Artery Occlusion Protocol

Spontaneous hypertensive (SHR) male rats, approximately 90-100 days old (~250-300g), are weighed and then anesthetized with ketamine (100mg/ml)/ xylazine (20mg/ml) cocktail (1.2ml/kg; i.p.) followed by subcutaneous administration of a long-acting antibiotic (e.g., combiotic). The level of anesthetic is assessed by corneal reflex (air puff to eye) and leg jerk in response to tail or foot pinch. Once the rat is anesthetized, it is placed on a small animal surgical board and restrained during the surgical procedure. The rat's body temperature is monitored with a rectal probe and maintained at 37°C with a homeostatic heating pad. Areas of incision are shaved and swabbed with betadine. The surgical area is aseptic. All surgical instruments are sterilized in an autoclave and/or in a glass bead dry instrument sterilizer, then rinsed with sterile saline or alcohol before use.
(1) Femoral Artery Catheter. An indwelling catheter is placed in the femoral artery for periodic blood sampling and measurement of arterial blood pressure. An incision is made over the area of the femoral artery. Tissue is blunt dissected to isolate the artery. The distal end of the artery is ligated with sterile suture and a loose ligature is placed around the proximal end for securing the catheter in place. A small incision is made in the artery for insertion of the catheter. The bevel-tipped end of PE50 tubing is inserted 5mm into the artery and then secured in place by sterile suture. The PE tubing is attached to a 1cc syringe filled with heparinized saline that is used minimally to keep the artery patent. Arterial blood is sampled three times: 10 minutes before ischemia, 2h after the onset of ischemia and 15 minutes post-reperfusion. All blood samples are taken in 100-300µl volumes to determine pH, PaO₂, PaCO₂, hematocrit and glucose. The maximum amount of blood withdrawn throughout the experiment does not exceed 1ml per animal. When blood sampling is not occurring, a blood pressure transducer is attached to the catheter to measure mean arterial pressure. At the end of the surgical procedure, the catheter is removed, the artery tied off and the incision area sutured shut.
(2) Tandem CCA-MCA Occlusion Model of Focal Cerebral Ischemia. Male spontaneously hypertensive Wistar rats (SHRs) are prepared for reversible focal cerebral ischemia by unilateral occlusion of both the common carotid artery (CCA) and the middle cerebral artery (MCA), using a modification of the technique described by Brint and co-workers (J. Cereb Blood Flow Metab 8:474-485, 1988). The left CCA is isolated through an incision in the ventral surface of the neck. For isolation of the ipsilateral MCA, a second incision is made between the lateral canthus of the left eye and the corresponding external auditory canal to bare the underlying skull. The MCA is exposed through a 5mm burrhole drilled 2-3mm rostral to the fusion of the zygomatic arch and the squamosal bone under direct visualization with a Zeiss operating microscope. The dura is opened with a sterile 26g needle and a platinum wire (0.1mm diameter) is inserted beneath the MCA just superior to the inferior cortical vein. The MCA is temporarily occluded by elevation and compression of the vessel across the platinum wire, as described by Aronowski and colleagues (Stroke, 25:2235-2240, 1994). Concurrently, the CCA is occluded with an aneurysm clip. The duration of occlusion of the CCA and the MCA is 2h. At the end of this period, the wire and the clip are carefully removed to allow reperfusion of the vessels and the incision area is sutured shut. The rat is placed in an isolation cage to recover before returning to his home cage. The rat is closely monitored for 2-4h post surgery to ensure uneventful recovery from the anesthesia and surgical procedure. Following recovery from anesthesia, the rat is housed according to the established protocol as per LAM guidelines until required for the experimental analysis.
(3) Test compounds are administered by any suitable route: intravenous, subcutaneous, or intraperitoneal. Dose and time of compound administration is based on *in vitro* assay results or literature references.
(4) Two outcome measures are used to assess compound efficacy: (a) behavioral performance on several CNS paradigms such as the Morris Water Maze, spontaneous motor activity, radial arm maze, and CNS general behavior profile; and (b) histological examination of the size of the cerebral cortical infarct. Twenty-four hours post-ischemia, the rats are tested in a behavioral paradigm and subsequently euthanized for brain histology. Rats are deeply anesthetized with an intraperitoneal injection of sodium pentobarbital (100 mg/kg, i.p). Depth of anesthesia is checked by lack of corneal reflex and tail pinch. Rats are euthanized by transcardial perfusion with approximately 50 ml heparinized physiological saline. After perfusion, the brain is removed, blocked and sectioned into 1mm slabs. Each slab is placed in TTC solution, a cell viability dye, for 15 min. The stained slabs are visualized with a Nikon SMZ-U dissecting microscope and image analysis of the affected brain areas is quantified using ImagePro 2.1 software. Infarct volume is expressed as % of contralateral hemisphere. Statistical comparisons are made across treatment groups (one-way ANOVA).

### Example 8

### In Vivo Study: Transient Model of Cerebral Ischemia

Male Wistar rats (Iffa Credo, France), weighing 250-300 g, were housed 2 per cage under a light-dark cycle of 12 hr - 12 hr (light on at 7:00 a.m., off at 7:00 p.m.) at a room temperature of21±2°C, with 50±15% humidity, for a minimum of 5 days before the experiments. During this time, the rats had free access to commercial rat chow (Trouw Nutrition France, Vigny, France, ref. 811002) and tap water.

The animals were anaesthetized with 5% halothane in air for induction, then 1-2% halothane during surgery. The rat's body temperature was maintained at 37°C with a heating pad. A 2cm incision was made at the center of the neck and the right common carotid artery (CCA), external carotid artery (ECA) and internal carotid artery (ICA) were exposed under an operating microscope. The ICA was further dissected to identify the pterygopalatine artery (PA) branch and the intracranial ICA branch. The CCA was ligated. A 3-0 silk suture was tied at the origin of the ECA and ligated. A catheter (id = 0.58 mm) was introduced into the CCA through a small puncture and advanced to the intracranial branch of the ICA. A 4-0 surgical nylon suture was introduced into the catheter. A length of approximately 19mm of nylon suture was gently advanced from the CCA into the lumen of the ICA until the suture blocked the origin of the MCA. The suture was sealed into the catheter by heat, leaving 1 cm of catheter protruding so the suture could be withdrawn to allow reperfusion. The incision was closed using skin clips. Anaesthesia was then terminated and the animals were placed under heat lamps until recovery from anaesthesia. The rats awakened 10-15 min later. After 2 hr of ischemia, reperfusion was performed by withdrawal of the suture until the tip cleared the ICA lumen. Vehicle or test compound was administered intraperitoneally (i.p.) or intravenously (i.v.) at 1hr post ischemia onset.

24 h after the onset of occlusion, the rats were killed by decapitation. The brains were immediately removed, frozen in isopentane and stored at -20°C. The brains were then cut in 20 µm thick sections in a cryocut. Every 20^{th} slice was used to measure infarct area. The sections were stained with cresyl violet and the areas of infarct in the striatum and cortex were determined by planimetry using an image analysis software (Image, NIH) after digitalization of the section images.

Following the procedure described above, MK-801, compound #46 and compound #264 (as listed in the Tables herein) were evaluated for neuroprotective effects in rats with results as listed in Table VIII. The data represent average lesion volume in mm³ for 12 measurements. Vehicle used was 15% solutol in 5% dextrose.

**Table VIII**

| **Effect of Compound #46 and Compound #264 in the transient model of cerebral ischemia in rats** | | | |
|---|---|---|---|
| **Test Compound (Admin. Amount)** | **Cortex Vol. (mm³)** | **Striatum Vol. (mm³)** | **Cortex + Striatum Vol. (mm³** |
| Vehicle | 236.3 ± 21.0 | 75.2 ± 2.5 | 311.6 ± 22.6 |
| MK-801 (3 mg/kg, i.p.) | 135.0 ± 26.1 | 70.4 ± 2.9 | 205.4 ± 28.0 |
| Cmpd 46 (0.6 mg/kg, i.v.) | 247.6 ± 24.8 | 79.1 ± 3.0 | 326.6 ± 26.0 |
| Cmpd 46 (3 mg/kg, i.v.) | 214.7 ± 26.6 | 76.1 ± 2.8 | 290.8 ± 28.1 |
| Cmpd 264 (0.3 mg/kg, i.v.) | 194.3 ± 33.9 | 72.1 ± 3.6 | 266.4 ± 35.4 |
| Cmpd 264 (1.5 mg/kg, i.v.) | 174.2 ± 30.5 | 72.4 ± 2.5 | 246.6 ± 31.0 |

### Synthesis Examples

### Example 9

### N-(6-Biphenyl-3-yl-pyrimidin-4-yl)-N'-(2-chloroethyl)-N'-ethyl-benzene-1,4-diamine

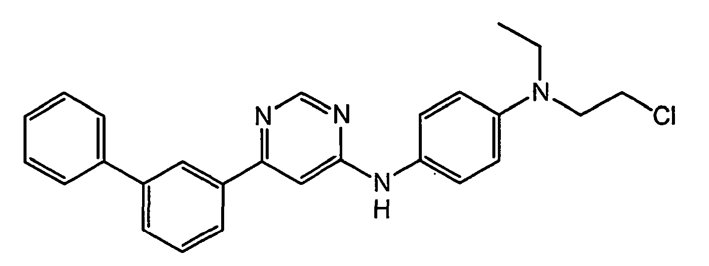

A solution of 2-{[4-(6-biphenyl-3-yl-pyrimidin-4-ylamino)-phenyl]-ethyl-amino}-ethanol (2 g, 4.8 mmol) (available from BioFocus PCC (LTK)) and dichloromethane (40 mL) was treated with thionyl chloride (1.4 ml, 19.1 mmol) and stirred overnight at room temperature. The reaction was quenched with NaHCO₃(s) and extracted with dichloromethane. The organic layer was dried with MgSO₄, filtered and evaporated to yield the title product as a crystalline solid.
Yield: (1.7g, 82%).

### Example 10

### N-(2-Aminoethyl)-N'-(6-biphenyl-3-yl-pyrimidin-4-yl)-N-ethyl-benzene-1,4,-diamine

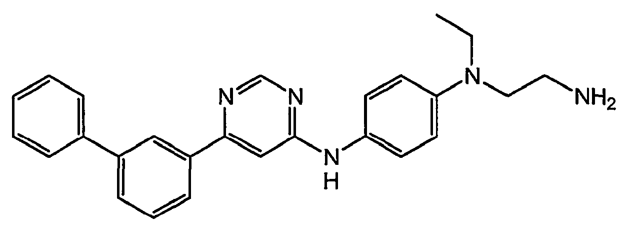

A solution of N-(6-Biphenyl-3-yl-pyrimidin-4-yl)-N'-(2-chloroethyl)-N'-ethylbenzene-1,4-diamine (0.35 g, 0.82 mmol) and N-methyl pyrrolidinone (5 mL) was treated with potassium phthalimide (0.227 g, 1.2 mmol) and the resulting mixture was stirred overnight at 100°C. The reaction was cooled, diluted with dichloromethane, and washed with water until the aqueous layer was neutral. The organic layer was dried over K₂CO₃, filtered and the solvent evaporated to yield 2-(2-{[4-(6-biphenyl-3-yl-pyrimidin-4-ylamino)-phenyl]-ethylamino}-ethyl)-isoindole-1,3-dione.

A solution of 2-(2-{[4-(6-biphenyl-3-yl-pyrimidin-4-ylamino)-phenyl]-ethylamino}-ethyl)-isoindole-1,3-dione (0.5 g, 0.82 mmol), ethanol (50 mL) and hydrazine monohydrate (3 mL) was refluxed for 3 hours, and then stirred overnight at room temperature. After cooling to 0°C, concentrated HCl/water (1:1, 20mL) was added and the reaction was refluxed for 2 hours. The ethanol was evaporated and the aqueous mixture was filtered. The filtrate was made basic with NaHCO₃(s) and was extracted with dichloromethane. The organic layer was dried with MgSO₄, filtered and solvent evaporated to yield the title product as a solid.
Yield: (0.17 g, 51 %).

### Example 11

### N-[1-((2-{[4-(6-biphenyl-3-yl-pyrimidin-4-ylamino)-phenyl]-ethyl-amino)-ethylcarbamoyl)-ethyl]-benzamide, Compound (205)

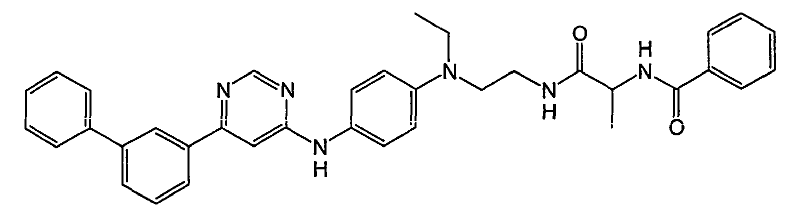

A solution of N-(2-aminoethyl)-N'-(6-biphenyl-3-yl-pyrimidin-4-yl)-N-ethylbenzene-1,4,-diamine (0.1 g, 0.185 mmol), N-benzoyl-alanine (0.032 g, 0.185 mmol), diisopropylethylamine (DIPEA) (0.066ml, 0.37 mmol), HBTU (0.07 g, 0.185 mmol), and N,N-dimethylformamide (DMF) was stirred overnight at room temperature. Ethyl acetate and NaHCO₃ were added with vigorous stirring. The organic layer was dried over Na₂SO_{4,} filtered and evaporated to a solid. This material was purified by HPLC to yield the title compounds as an orange solid.

### Example 12

### 5-(2-oxo-hexahydro-thieno[3,4-d]imidazol-6-yl)-pentanoic acid (2-{[4-(6-biphenyl-3-yl-pyrimidin-4-ylamino)-phenyl]-ethyl-amino}-ethyl)-amide, Compound (206)

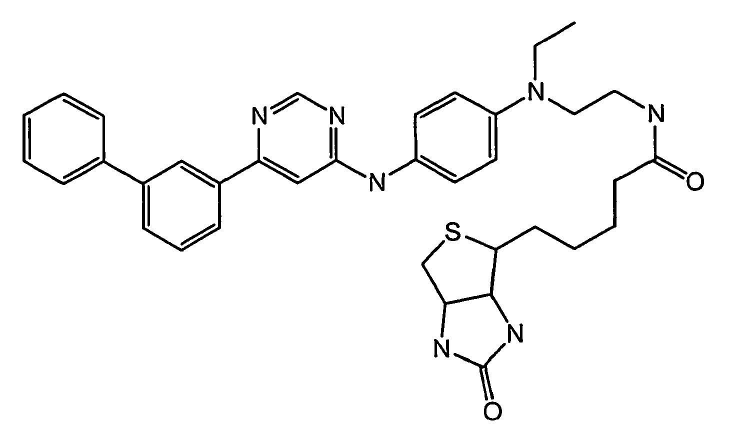

A solution of N-(2-Aminoethyl)-N'-(6-biphenyl-3-yl-pyrimidin-4-yl)-N-ethylbenzene-1,4,-diamine (0.1 g, 0.24 mmol), biotin succinate ester (0.1 g, 0.29 mmol), DIPEA (0.2 ml), and dioxane was stirred overnight at room temperature. Ethyl acetate and water were added with thorough mixing. The organic layer was separated, dried over MgSO₄, filtered and evaporated. The residue was purified by reverse phase chromatography to yield the title compounds.
Yield: (0.031 g, 41 %).

Compound 208 was similarly prepared according to the procedure outlined above with appropriate selection and substitution of reagents.

### Example 13

### Hexadecanoic acid (2-{[4-(6-biphenyl-3-yl-pyrimidin-4-yl-amino)-phenyl]-ethyl-amino}-ethyl)-amide, Compound (210)

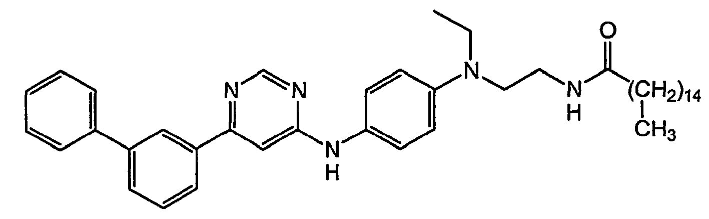

A solution of N-(2-aminoethyl)-N'-(6-biphenyl-3-yl-pyrimidin-4-yl)-N-ethylbenzene-1,4,-diamine (0.025 g, 0.06mmol), palmitoyl acid chloride (0.016 mL, 0.06 mmol), DIPEA (0.024 mL, 0.132 mmol), and dioxane (0.4 mL) was stirred at room temperature overnight. Ethyl acetate and NaHCO₃(s) were added with thorough mixing. The organic layer was separated, dried over MgSO₄, filtered and evaporated to an oil. This material was purified using reverse phase chromatography (20% CH₃CN/H₂O) to yield the title compound as an solid.
Yield: (0.011 g, 28%).

Compounds 207, 211, and 212 were similarly prepared according to the process outlined above with appropriate selection and substitution of reagents.

### Example 14

### N-(2- {[4-[(6-biphenyl-3-yl-pyrimidin-4-yl)-butyryl-amino]-phenyl]-ethyl-amino}-ethyl)-butyramide, Compound (274)

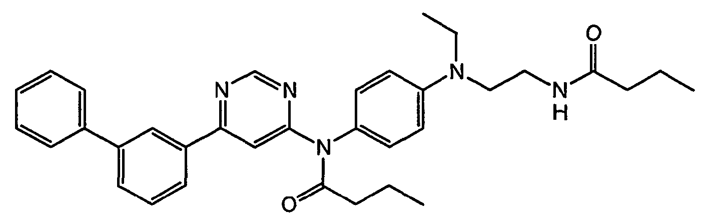

A solution of N-(2-aminoethyl)-N'-(6-biphenyl-3-yl-pyrimidin-4-yl)-N-ethylbenzene-1,4,-diamine (0.025 g, 0.06mmol), propionyl chloride (0.006 mL, 0.06 mmol, DIPEA (0.024 mL, 0.132 mmol), and dioxane (0.4 mL) was stirred at room temperature overnight. Ethyl acetate and NaHCO₃(s) were added with thorough mixing. The organic layer was separated, dried over MgSO₄, filtered and evaporated to an oil. This material was purified using reverse phase chromatography (20% CH₃CN/H₂O) to yield the title compound as an oil.
Yield: (0.012 g, 37%).

Compounds 272 and 273 were similarly prepared according to the process outlined above with appropriate selection and substitution of reagents.

### Example 15

### N-(6-biphenyl-3-yl-pyrimidin-4-yl)-N-[2-(cyclohexylmethyl-amino)ethyl]-N'-ethylbenzene-1,4-diamine, Compound (214)

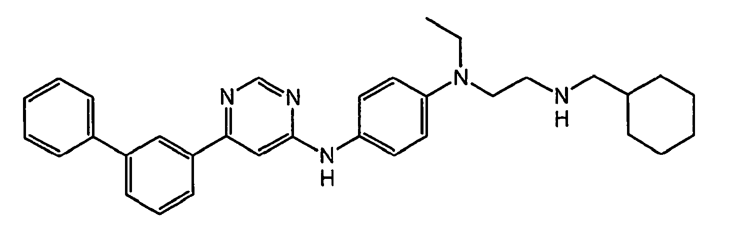

A solution of N-(6-biphenyl-3-yl-pyrimidin-4-yl)-N'-(2-chloroethyl)-N'-ethylbenzene-1,4-diamine (0.025 g, 0.058 mmol), cyclohexylamine (0.022 mL, 0.174 mmol), and 1-methyl-2- pyrrolidinone (0.6 mL) was heated at 130°C overnight in a glass microtube. The reaction mixture was filtered and the product was separated from the filtrate via reverse phase chromatography (20% CH₃CN/H₂O) to yield the title product as an oil.
Yield: (0.002g, 7%).

Compounds 209, 213, and 215-269 were similarly prepared according to the process outlined above with appropriate selection and substitution of reagents.

### Example 16

### 2-Benzoylamino-propionic acid 2-2-{[4-(6-biphenyl-3-yl-pyrimidin-4-yl-amino)-phenyl]-ethyl-amino}-ethyl ester, Compound (269)

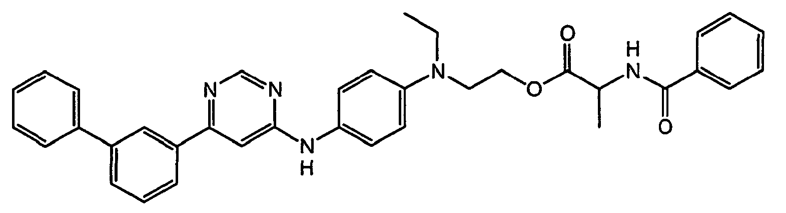

A solution of 2-1[4-(6-biphenyl-3-yl-pyrimidin-4-ylamino)-phenyl]-ethyl-amino}-ethanol (0.2 g, 0.37mmol), N-benzoylalanyl chloride (Berkowitz, et al. JOC, 60(5),1995, 1233) (0.6 mL, 1.9 mmol), and toluene (0.5 mL) was stirred at 80°C overnight. After the reaction was cooled, DMF (1mL) was added and stirring was resumed for 1 hour. Ethyl acetate and NaHCO₃(s) were added with thorough mixing. The organic layer was separated, dried over MgSO₄, filtered and evaporated to an oil. The material was purified via flash chromatography (20% methanol/CH₂Cl₂) to yield the title product as a solid.
Yield: (0.041 g, 19%).

Compounds 270 and 271 were similarly prepared according to the process outlined above with appropriate selection and substitution of reagents.

Following the procedures described above, representative compounds of formula (I) of the instant invention were prepared as listed in Tables IX, X, XI and XII.

**Table IX: Representative Compounds of Formula (I)**

| | | |
|---|---|---|
| 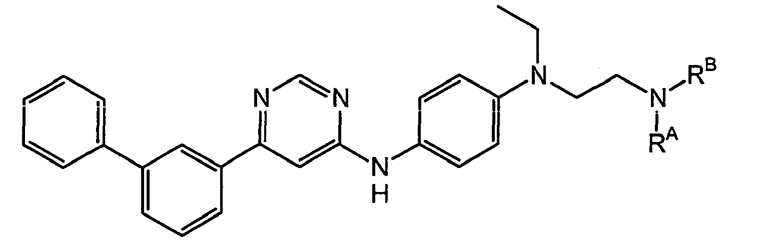 | | |

| **Cmpd No.** | **R^{A}** | **R^{B}** |
|---|---|---|
| 204 | H | H |
| 209 | Methyl | Methyl |
| 213 | H | Methoxypropyl |
| 214 | H | Cyclohexylmethyl |
| 215 | H | 3-Chlorobenzyl |
| 216 | H | 2,4-di-Methoxybenzyl |
| 217 | H | 2-ethoxybenzyl |
| 218 | H | propyl |
| 219 | H | 2,5-di-Fluorobenzyl |
| 220 | H | dehydroabietyl |
| 221 | H | Phenylethyl |
| 222 | H | 3,4-di-Methoxybenzyl |
| 223 | H | Diethylaminopropyl |
| 224 | H | 4-Bromo-2-pyridyl |
| 225 | Benzyl | Dimethylaminoethyl |
| 226 | Benzyl | n-butyl |
| 227 | H | 4-biphenyl |
| 228 | H | 2-furanylmethyl |
| 229 | H | 3-Iodobenzyl |
| 230 | H | 2,2,2-trifluoroethyl |
| 231 | H | 3,4-di-Fluorobenzyl |
| 232 | H | 2-thienylethyl |
| 233 | H | 3,5-dimethyl-2-pyridyl |
| 234 | Benzyl | Phenylethyl |
| 235 | Benzyl | 2-(ethoxy)acetyl |
| 236 | H | 2-methoxybenzyl |
| 237 | H | 4-Bromobenzyl |
| 238 | H | 3,5-di-Trifluoromethylbenzyl |
| 239 | H | 3-Methoxyphenylethyl |
| 240 | H | 3,4,5-Trimethoxybenzyl |
| 241 | H | 4-Methoxyphenylethyl |
| 242 | H | 4-imidazolylethyl |
| 243 | H | 2-Trifluoromethylbenzyl |
| 244 | H | benzyl |
| 245 | H | 3-Methoxybenzyl |
| 246 | H | 3-Methylbenzyl |
| 247 | H | 3,5-Dimethoxybenzyl |
| 248 | H | 2-Bromobenzyl |
| 249 | H | 4-Bromophenethyl |
| 250 | H | 4-Fluorobenzyl |
| 251 | H | 1-Naphthylmethyl |
| 252 | H | Phenoxyethyl |
| 253 | H | 4-Trifluoromethylbenzyl |
| 254 | H | n-butyl |
| 255 | Benzyl | benzyl |
| 256 | H | 3-pyridyl |
| 257 | H | 4-Trifluoromethylbenzyl |
| 258 | H | 2-Methylbenzyl |
| 259 | H | 3-Methylbenzyl |
| 260 | H | 2-Fluorobenzyl |
| 261 | H | 3,4-dimethoxyphenethyl |
| 262 | Ethyl | benzyl |

**Table X: Representative Compounds of Formula (I)**

| | |
|---|---|
| 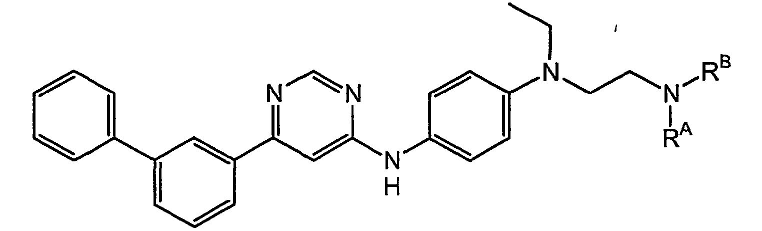 | |

| **Cmpd No.** | **R^{A} and R^{B} taken together with the N atom** |
|---|---|
| 263 | N-Pyrrolidinyl |
| 264 | N-Morpholinyl |
| 265 | N-Imidazolyl |
| 266 | N- I,2,3,4-Tetrahydroisoquinolinyl |
| 267 | N-Hexamethyleneimine |
| 268 | N-(4-Tertiarybutyloxycaxbonyl)piperazi.nyl |

**Table XI: Representative Compounds of Formula (I)**

| | |
|---|---|
| 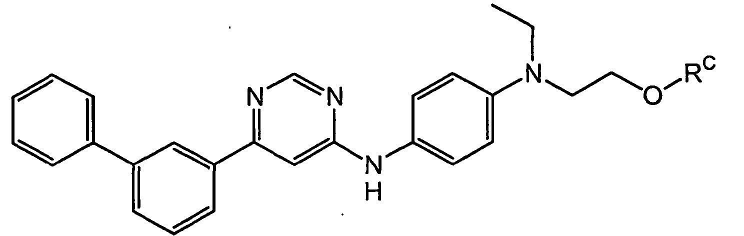 | |

| **Cmpd No.** | **R^{C}** |
|---|---|
| 269 | (±)-N-Benzoyl)-2-aminopropionoyl |
| 270 | [3aS-(3aα,4β,6aα)]- Hexahydro-2-oxo-1H-thieno[3,4-d]imidazole-4-pentanoyl |
| 271 | Benzoyl |

**Table XII: Representative Compounds of Formula (I)**

| | | |
|---|---|---|
| 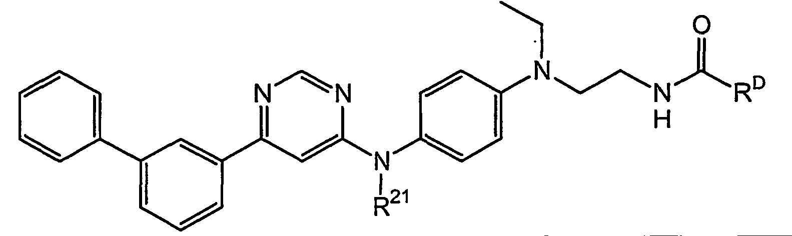 | | |

| **Cmpd No.** | **R²¹** | **R^{D}** |
|---|---|---|
| 205 | H | (±)-N-Benzoyl)-aminoeth-2-yl |
| 206 | H | [3aS-(3aα,40,6aα)]- Hexahydro-2-oxo-1H-thieno[3,4-d]imidazole-but-4-yl |
| 207 | H | 1-Chloro-1-phenyl-methyl |
| 208 | H | 3-azo-6-nitrophenyl |
| 210 | H | Pentacecanyl |
| 211 | H | 4-Biphenyl |
| 212 | H | 4-butoxyphenyl |
| 272 | 4-trifluoromethyl phenylcarbonyl | 4-trifluoromethylphenyl |
| 273 | 3-fluorophenyl carbonyl | 3-fluorophenyl |
| 274 | propylcarbonyl | propyl |

The representative compounds listed in Tables IX< X, XI and XII were tested according to the procedure described in Example 5 with results as listed in Table XIII. Unless otherwise noted, molecular weights were measure on a Hewlitt-Packard Series 1050 Chemical Ionization mass spectrometer.

**Table XIII: Measured Activity and Molecular Weights**

| **Cmpd No.** | **ED₅₀** | **MW Calc** | **MW Meas** |
|---|---|---|---|
| 204 | | 409 | 410.2 |
| 205 | | 584.7 | 585.4 |
| 206 | | 635.8 | 636.4 |
| 207 | | 562.1 | 562.2 |
| 208 | | 599.7 | 600.2 |
| 209 | 0.0023 | 437.6 | 438.1 |
| 210 | | 647.9 | 648.4 |
| 211 | | 589.7 | 590.2 |
| 212 | | 585.7 | 586.2 |
| 213 | 0.37 | 481.6 | 482.1 |
| 214 | 0.525 | 505.7 | 506.2 |
| 215 | 0.654 | 534.1 | 534.1 |
| 216 | 1.69 | 559.7 | 560.2 |
| 217 | 1 | 543.7 | 544.2 |
| 218 | 1.9 | 451.6 | 452.1 |
| 219 | 0.36 | 535.6 | 536.2 |
| 220 | 1 | 677.9 | 678.4 |
| 221 | 0.652 | 513.6 | 514.2 |
| 222 | 1.85 | 559.7 | 560.2 |
| 223 | 1.14 | 522.7 | 523.2 |
| 224 | 0.507 | 565.5 | 567.1 (M+2) |
| 225 | 0.509 | 570.7 | 571.3 |
| 226 | 0.618 | 555.8 | 556.3 |
| 227 | 3.8 | 561.7 | 562.2 |
| 228 | 0.885 | 489.6 | 490.1 |
| 229 | 1.9 | 625.6 | 626.1 |
| 230 | 1.3 | 491.6 | 492.1 |
| 231 | 0.267 | 535.6 | 536.1 |
| 232 | 0.769 | 519.7 | 520.1 |
| 233 | | 514.6 | 515.2 |
| 234 | 1 | 603.8 | 604.3 |
| 235 | 1 | 585.7 | 586.2 |
| 236 | 1.1 | 529.7 | 530.2 |
| 237 | 0.361 | 578.6 | 580.1(M+2) |
| 238 | 1 | 635.7 | 636.1 |
| 239 | 0.606 | 543.7 | 544.2 |
| 240 | 0.285 | 589.7 | 590.2 |
| 241 | 1.25 | 543.7 | 544.2 |
| 242 | 1.28 | 503.7 | 504.1 |
| 243 | 1 | 567.7 | 568.2 |
| 244 | 0.43 | 499.7 | 500.1 |
| 245 | 1.6 | 529.7 | 530.2 |
| 246 | 0.118 | 513.7 | 514.2 |
| 247 | 1 | 559.7 | 560.2 |
| 248 | 1 | 578.6 | 581.1(M+2) |
| 249 | 1 | 592.6 | 594.1(M+2) |
| 250 | 1.8 | 517.6 | 518.2 |
| 251 | 1 | 549.7 | 550.2 |
| 252 | 0.523 | 529.7 | 530.2 |
| 253 | 2 | 583.7 | 584.2 |
| 254 | 0.483 | 465.6 | 466.2 |
| 255 | 1 | 589.8 | 590.3 |
| 256 | 1.6 | 500.6 | 501.2 |
| 257 | 1 | 567.7 | 568.2 |
| 258 | 1 | 567.7 | 568.2 |
| 259 | 0.394 | 513.7 | 514.2 |
| 260 | 2.3 | 517.6 | 518.2 |
| 261 | 1.6 | 573.7 | 574.3 |
| 262 | 1 | 527.7 | 528.2 |
| 263 | | 506.7 | 507.2 |
| 264 | 0.002 | 522.7 | 523.2 |
| 265 | 1 | 460.58 | 461.1 |
| 266 | 1.2 | 525.7 | 526.2 |
| 267 | | 505.7 | 506.2 |
| 268 | 0.5 | 578.8 | 579.3 |
| 269 | | 585.7 | 586.4 |
| 270 | | 636.8 | 637.3 |
| 271 | | 514.6 | 515.1 |
| 272 | | 409 | 410.2 |
| 273 | | 584.7 | 585.4 |
| 274 | | 521.7 | 522.1 |

While the foregoing specification teaches the principles of the present invention, with examples provided for the purpose of illustration, it will be understood that the practice of the invention encompasses all of the usual variations, adaptations and/or modifications as come within the scope of the following claims.

### References

Ahn, N., et al. (1991) J. Biol Chem. 266:4220-4227.
Alessandrini, A., et al. (1999) Proc Natl Acad Sci U S A 96:12866-9.
Anderson, N., et al. (1990) Nature 343:651-653.
Bading, H., Greenberg, M. E. (1991) Science 253:912-914.
Callaway, J., et al. (1999) Stroke 12:2704-2712.
Canzoniero, L., et al. J. (1996) J. Neurosci. Res. 45:226-236.
Cheng, Y., et al. (1998) J. Clin. Invest. 101:1992-1999.
Chijiwa, T., et al. (1990) J. Biol. Chem. 265:5267-5272.
Courtney, M., et al. (1990) J Neurosci. 10:3873-3879.
Crews, C., Alessandrini, A., Erikson, R. (1992). Science 258:478-480.
Desdouits-Magnen, J., et al. (1998) J. Neurochem. 70:524-530.
Enslen, H., et al. (1996) Proc Natl Acad Sci USA 93:10803-10808.
Favata, M. F., et al., (1998) J. Biol. Chem. 273:18623-18632.
Fiore, R. S., et al. (1993) J Nezcrochem 61:1626-1633.
Grobin, A. C., (1999). Brain Research 827:1-11.
Heath, D., Vink, R. (1999). J. Neurosurg. 90:504-509.
Henry, J., et al. (1998) J. Med. Chem. 41:4196-4198.
Hu, B., Wieloch, T. (1994) J. Neurochem. 62:1357-1367.
Inhorn, R., Majerus, P. (1987) J. Biol. Chem. 262:15946-15952.
Ishii, T., et al. (1993) J. Biol. Chem. 268:2836-2843.
Jaiswal, R., et al. (1994) Mol. Cell. Biol. 14:6944-6953.
Jones-Velleneuve, et al. (1982) J. Cell Biol. 94:253-262.
Jones-Villeneuve, et al. (1983) Mol. Cell Biol. 3:2271-2279.
Jovanovic, J., et al. (1996) Proc. Natl. Acad. Sci. USA 93:3679-3683.
Kindy, M. (1993) J Cereb Blood Flow Metab 13:372-377.
Kobayashi, E., et al. (1989) Biochem. Biophys. Res. Commun. 159:548-553.
Kurino, M., et al. (1995) J Neurochem 65:1282-1289.
Lander, H., et al. (1996) J. Biol. Chem. 271:19705-19709.
Laurie, D., et al. (1995) Brain Res. Mol. Brain Res. 32:94-108.
Lee, J., et al. (1994) Nature 372:739-746.
Lev, S., et al. (1995) Nature 376:737-745.
Li, B., et al. (1999a) Eur. J. Biochem. 262:211-217.
Li, M., et al. (1999b) Brain Res. 1999:44-52.
McBurney, M. W., et al. (1982) Dev. Biol. 89:503-508.
Meldrum, B., Garthwaite, J. (1990) Trends Pharmacol Sci 11:379-387.
Monyer, H., et al. (1994) Neuron 12:529-540.
Monyer, H., et al. (1992) Science 256:1217-1221.
Moodie, S., et al. (1993) Science 260:1658-1661.
Morley, P., et al. (1995) J. Neurochem. 65:1093-1099.
Murray, B., et al. (1998) Proc. Natl. Acad. Sci. USA 95:11975-11980.
Onoda, T., et al. (1989) J. Nat. Prod. 52:1252-1257.
Papin, C., et al. (1995) Oncogene 10:1647-1651.
Park, C., et al. (1988) Ann. Neurol. 24:543-551.
Pazos, A., et al. (1999) Brain Res. 846:186-195.
Pleasure, S., Lee, V.-Y. (1993) Journal of Neurosci. Res. 35:585-602.
Pleasure, S., et al. (1992) J. Neurosci. 12:1802-1815.
Ray, W. J., Gottlieb, D. I. (1993) Biochem. Biophys. Res. Comm. 197:1475-1482.
Rootwelt, T., et al. (1998) J. Neurochem. 71:1544-1553.
Rosen, L., et al. (1994) Neuron 12:1207-1221.
Sakagami, H., Kondo, H. (1993) Brain Res Mol Brain Res 19:215-218.
Sakakibara, Y., et al. (2000) Neurosci Lett 281:111-114.
Sattler, R., et al. (1999) Science 284:1845-1848.
Schneggenburger, R., et al. (1993) Neuron 11:133-143*.*
Schwartz-Bloom, R., et al. (1998) J Cereb Blood Flow Metab 18:548-558.
Sola, C., et al. (1999) J Neurosci. Res. 57:651-652.
Stanciu, M., et al. (2000) J. Biol. Chem. 275:12200-12206.
Standaert, D., et al. (1994) J. Comp. Neurol. 343:1-16.
Takahashi, M., et al. (1998) J. Pharmacol. Exp. Ther. 287:559-566.
Tatlisumak, T., et al. (1998) Stroke 29:1952-1958.
Tokumitsu, H., et al. (1990) J. Biol. Chem. 265:4315-4320.
Turetsky, D. M., et al. (1993) J. Neurobiol. 24:1157-1169.
Turski, L., et al. (1998) Proc. Natl. Acad. Sci. USA 95:10960-10965.
Tymianski, M., et al. (1993) J. Neurosci. 13:2085-2104.
Vanhoutte, P., et al. (1999) Mol. Cell. Biol. 19:136-146.
Wasterlain, C., et al. (1996) Stroke 27:1236-1240.
Xia, Z., et al. (1995) Science 270:1326-1331.
Xia, Z., et al. (1996) J. Neurosci. 16:5425-5436.
Yang, D. D., et al. (1997) Nature 389:865-870.
Yang, Y., et al. (1998) Brain Res. 804:169-176.
Zheng, C., et al. (1993) J. Biol. Chem. 268:23933-23939.

## Claims

1. A pharmaceutical composition comprising a pharmaceutically acceptable carrier and a compound having the formula 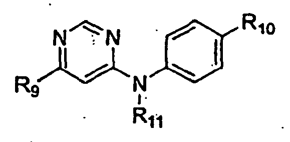 or a pharmaceutically acceptable salt thereof, wherein
(a) R₉ is selected from the group consisting ofH, thienyl, furanyl, pyrrolyl, phenyl, substituted phenyl, pyridinyl, substituted pyridiny!, naphthyl, benzo[b]thien-2-yl, 2-benzofuranyl; pyrimidine and, 2,4-(bismethoxyphenyl)-5-pyrimidinyl,
said substituted phenyl having the formula 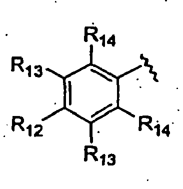 wherein (i) R₁₂ is H, OH, lower alkylthio, alkoxy, alkylamine, dialkylamine, halogen-substituted lower alkyl, halogen substituted lower alkoxy, cyano, cyanoalkyl, phenyl, phenylalkoxy or substituted piperazinyl, -N-(t-butoxy)carbamylalkyl, (ii) each R₁₃ is independently H, NO₂, alkoxy, alkylamino, dialkylamino, halogen-substituted lower alkyl, halogen-substituted lower alkoxy or phenyl, and (iii) each R₁₄ is independently H, alkoxy, phenyloxy or phenylalkoxy;
(b) R₁₀ is selected from the group consisting of cyanoalkyl, alkylamino, dialkylamino, hydroxy-substituted alkylamino and hydroxy-substituted dialkylamino; and
(c) R₁₁ is H or lower alkyl,
and wherein lower alkyl refers to an alkyl containing 1 to 4 carbon atoms, lower alkoxy refers to an O-alkyl containing 1 to 4 carbon atoms and lower alkylthio refers to an S-alkyl containing 1 to 4 carbon atoms.

2. The pharmaceutical composition of claim 1, wherein R₉ is substituted phenyl.

3. The pharmaceutical composition of claim 1, wherein R₁₁ is H and R₁₀ is dialkylamino or hydroxy-substituted dialkylamino.

4. The pharmaceutical composition of claim 1, wherein the compound is N1,N1-dimethyl-N4-[6-[4-(phenylmethoxy)phenyl]-4-pyrirnidinyl]-1,4-benzenediamine.

5. The pharmaceutical composition of claim 1, wherein the compound is N1-(6-[1,1'-biphenyl]-3-yl-4-pyrimidinyl)-N4,N4-dinlethyl-1 ,4-benzenedianine.

6. The pharmaceutical composition of claim 1, wherein the compound is N1-[6-[3,5-bis(trifluoromethyl)phenyl]-4-pyrimidinyl]-N4,N4-dimethyl-1,4-benzenediamine.

7. The pharmaceutical composition of claim 1, wherein the compound is 2-[[4-[(6-[1,1'-biphenyl]-3-yl-4-pyrimidinyl)amino]phenyl] ethylamino]-ethanol.

8. The pharmaceutical composition of claim 1, wherein the compound is 2-[[4-[(6-benzo[b]thien-2-yl-4-pyrimidinyl)amino]phenyl]ethylamino]-ethanol.

9. The pharmaceutical composition of claim 1, wherein the compound is 2-[ethyl[4-[[6-[4-(trifluoromethoxy)phenyl]-4.-pyrimidinyl]amino]phenyl] amino]-ethanol.

10. The pharmaceutical composition of claim 1, wherein the compound is of the formula

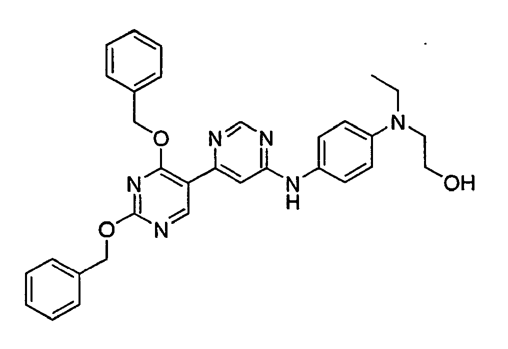

11. A pharmaceutical composition comprising a pharmaceutically acceptable carrier and a compound having the formula 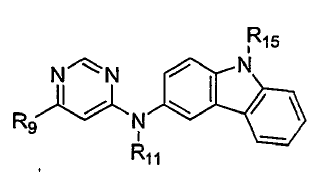 or a pharmaceutically acceptable salt thereof, wherein
(a) R₉ is selected from the group consisting of H, thienyl, furanyl, pyrrolyl, phenyl, substituted phenyl, pyridinyl, substituted pyridinyl, naphthyl, benzo[b]thien-2-yl, 2-benzofuranyl, pyrimidine and 2,4-(bismethoxyphenyl)-5-pyrimidinyl,
said substituted phenyl having the formula 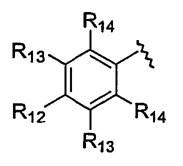 wherein (i) R₁₂ is H, OH, lower alkylthio, alkoxy, alkylamine, dialkylamine, halogen-substituted lower alkyl, halogen substituted lower alkoxy, cyano, cyanoalkyl, phenyl, phenylalkoxy or substituted piperazinyl, N-(t-butoxy)carbamylalkyl, (ii) each R₁₃ is independently H, NO₂ alkoxy, alkylamino, dialkylamino, halogen-substituted lower alkyl, halogen-substituted lower alkoxy or phenyl, and (iii) each R₁₄ is independently H, alkoxy, phenyloxy or phenylalkoxy;
(b) R₁₁ is H or lower alkyl ; and
(c) R₁₅ is H or alkyl,
and wherein lower alkyl refers to an alkyl containing 1 to 4 carbon atoms, lower alkoxy refers to an O-alkyl containing 1 to 4 carbon atoms and lower alkylthio refers to an S-alkyl containing 1 to 4 carbon atoms.

12. The use of a prophylactically effective amount of the compound recited in claim 1 or claim 11 for the manufacture of a medicament for the reduction of ischemic death in a cell population.

13. The use of claim 12, wherein the cell population comprises a cell selected from the group consisting of a neuronal cell, a glial cell, a cardiac cell, a lymphocyte, a macrophage and a fibroblast.

14. The use of a prophylactically effective amount of the compound recited in claim 1 or claim 11 for the manufacture of a medicament for the reduction of death in a cell population comprising neuronal cells in response to a traumatic event, wherein said medicament is adapted for administration prior to, during, or within a suitable time period following the traumatic event.

15. A method for reducing ischemic death in a cell population comprising contacting the cell with a prophylactically effective amount of the compound recited in claim 1 or claim 11, wherein the contacting is performed *ex vivo.*

16. A method of reducing death in a cell population comprising neuronal cells in response to a traumatic event comprising contacting the neuronal cell with a prophylactically effective amount of the compound recited in claim 1 or claim 11 prior to, during, or within a suitable time period following the traumatic event, wherein the contacting is performed *ex vivo.*

17. The pharmaceutical composition of claim 1 for use as a medicament for the reduction of neuronal cell death in response to a traumatic event in a subject, wherein the pharmaceutical composition of claim 1 is administered to the subject in a prophylactically effective amount prior to, during, or following the traumatic event.

18. The pharmaceutical composition of claim 11 for use as a medicament for the reduction of neuronal cell death in response to a traumatic event in a subject, wherein the pharmaceutical composition of claim 11 is administered to the subject in a prophylactically effective amount prior to, during or following the traumatic event.

19. The pharmaceutical composition of claim 17, wherein the subject is a human.

20. The pharmaceutical composition of claim 17, wherein the traumatic event is selected from the group consisting of a medical disorder, a physical trauma, a chemical trauma and a biological trauma.

21. The pharmaceutical composition of claim 17, wherein the pharmaceutical composition is administered prior to the traumatic event.

22. The pharmaceutical composition of claim 17, wherein the pharmaceutical composition is administered during the traumatic event.

23. The pharmaceutical composition of claim 17, wherein the pharmaceutical composition is administered subsequent to the traumatic event.

24. The pharmaceutical composition of claim 18, wherein the subject is a human.

25. The pharmaceutical composition of claim 18, wherein the traumatic event is selected from the group consisting of a medical disorder, a physical trauma, a chemical trauma and a biological trauma.

26. The pharmaceutical composition of claim 18, wherein the pharmaceutical composition is administered prior to the traumatic event.

27. The pharmaceutical composition of claim 18, wherein the pharmaceutical composition is administered during the traumatic event.

28. The pharmaceutical composition of claim 18, wherein the pharmaceutical composition is administered subsequent to the traumatic event.

29. An apparatus for administering to a subject the pharmaceutical composition of claim 1 comprising a container and the pharmaceutical composition of claim 1 therein, wherein the container has a device for delivering to the subject a prophylactic dose of the pharmaceutical composition.

30. An apparatus for administering to a subject the pharmaceutical composition of claim 11 comprising a container and the pharmaceutical composition of claim 11 therein, wherein the container has a device for delivering to the subject a prophylactic dose of the pharmaceutical composition.

31. The apparatus of claim 29, wherein the device for delivering the pharmaceutical composition is a syringe.

32. The apparatus of claim 30, wherein the device for delivering the pharmaceutical composition is a syringe.

33. A compound of the formula 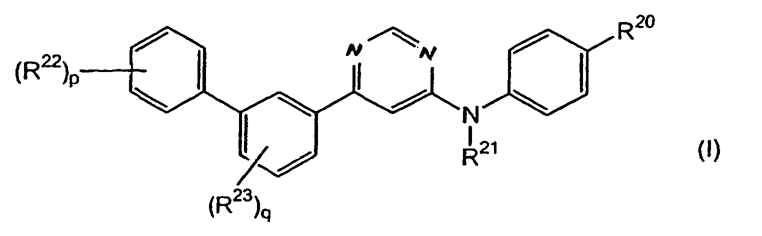 wherein
R²⁰ is selected from the group consisting of 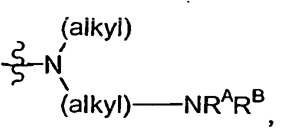 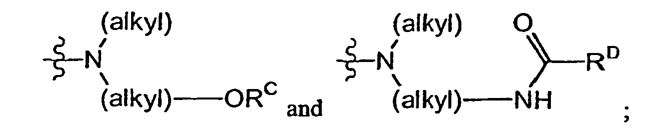
wherein R^{A} and R^{B} are independently selected from hydrogen, alkyl, halogenated alkyl, amino, alkylamino, dialkylamino, aminoalkyl, alkylaminoalkyl, dialkylaminoalkyl, aryl, aralkyl, cycloalkyl, cycloalkyl-alkyl, heteroaryl, heteroarylalkyl, alkoxyalkyl, aryloxy, aryloxyalkyl, alkoxycarbonylalkyl and dehydroabietyl; wherein the aryl cycloalkyl, heteroaryl or heterocycloalkyl portion of any of the groups is optionally substituted with one or more substituents independently selected from halogen, alkyl, alkoxy, halogenated alkyl, amino, alkylamino, dialkylamino, arylamino, aralkylamino, amido, alkylamido, dialkylamido, arylamido, aralkylamido, azo, nitro, cyano, aryl, aralkyl, aryloxy, carboxy, alkoxycarbonyl, aryloxycarbonyl, alkylthio, arylthio, alkylsulfonylN(H), or alkylsulfonylN(alkyl);
alternatively R^{A} and R^{B} are taken together with the nitrogen atom to which they are bound to form a compound selected from the group heteroaryl and heterocycloalkyl; wherein the heteroaryl or heterocycloalkyl is optionally substituted with one or more substituents independently selected from halogen, alkyl, alkoxy, alkoxycarbonyl, halogenated alkyl, alkylcarbonyl, amino, alkylamino, dialkylamino, arylamino, azo, nitro or cyano;
R^{C} is selected from the group consisting of alkyl, aralkyl, alkylcarbonyl, arylcarbonyl, aralkylcarbonyl, (±)-N-benzoyl-aminoalkylcarbonyl or [3aS-(3aα,4β,6aα)]-hexahydro-2-oxo-1 1H-thieno[3,4-d]imidazole-alkylcarbonyl;
R^{D} is selected from alkyl, aryl, aralkyl, (±)-N-benzoyl-aminoalkyl-, [3aS-(3aα,4β,6aα)]-hexahydro-2-oxo-1H-thieno(3,4-d)imidazole-alkyl or biphenyl; wherein the alkyl or aryl portion of the alkyl, aryl or aralkyl group is optionally substituted with one or more substituents independently selected from halogen, alkyl, alkoxy, amino, alkylamino, dialkylamino, azo, nitro, cyano, or trifluoromethyl);
R²¹ is selected from the group consisting of hydrogen, alkyl, aryl, aralkyl, alkylcarbonyl, arylcarbonyl and aralk-ylcarbonyl; wherein the aryl portion is optionally substituted with one or more substituents independently selected from halogen, alkyl, alkoxy, halogenated alkyl or nitro;
p is an integer selected from 0 to 3;
q is an integer selected from 0 to 3;
R²² and R²³ are each independently selected from the group consisting of halogen, alkyl, allcoxy, amino, alkylamino, dialkylamino, nitro, cyano, carboxy, alkoxycrabonyl, aryloxycarbonyl, aminocarbonyl, alkylaminocarbonyl and dialkylaminocarbonyl;
or a pharmaceutically acceptable salt thereof.

34. The compound as in Claim 33 wherein
R^{A} is selected from the group consisting of hydrogen, alkyl, aryl and aralkyl; wherein the aryl portion of any of the groups is optionaly substituted with one to three substituents independently selected from halogen, lower alkyl, lower alkoxy, halogenated lower alkyl, amino, lower alkylamino; di(lower alkyl)amino, arylamino, aralkylamino, azo, nitro, cyano, aryl, aralkyl, aryloxy, carboxy, lower alkoxycarbonyl, aryloxycarbonyl, lower alkylthio and arylthio;
R^{B} is selected from the group consisting of hydrogen, alkyl, halogenated lower alkyl, amino, lower alkylamino, di(lower alkyl)amino, amino-lower alkyl, lower alkylamino-lower alkyl, di(lower alkyl)amino-lower alkyl, aryl, aralkyl, cycloalkyl, cycloalkyl-lower alkyl, heteroaryl, heteroaryl-lower alkyl, lower alkoxy-lower alkyl, aryloxy, aryloxy-lower alkyl, lower alkoxycarbonyl-lower alkyl and dehydroabietyl; wherein the aryl, cyloalkyl, heteroaryl or heterocycloalkyl portion of any of the groups is optionally substituted with one to three substituents independently selected from halogen, lower alkyl, lower alkoxy, halogenated lower alkyl, amino, lower alkylamino, di(lower alkyl)amino, arylamino, aralkylamino, azo, nitro, cyano, aryl, aralkyl, aryloxy, carboxy, lower alkoxycarbonyl, aryloxycarbonyl, lower alkylthio and arylthio;
alternatively, R^{A} and R^{B} are taken together with the nitrogen atom to which they are bound to form a ring structure selected from the group consisting of heteroaryl and heterocycloalkyl; wherein the heteroaryl or heterocycloalkyl group is optionally substituted with one to two substituents independently selected from halogen, lower alkyl, lower alkoxy, lower alkoxycarbonyl, trifluoromethyl, lower alkylcarbonyl, amino, lower alkylamino, di(lower alkyl)amino, arylamino, azo, nitro or cyano.
R^{C} is selected from the group consisting of lower alkyl, aralkyl, lower allcylcarbonyl, arylcarbonyl, aralkylcarbonyl, (±)-N-ben2oyl-amino-lower alkylcarbonyl and [3aS-(3aa,4(3,6aa)]-hexahydro-2-oxo-1H-thieno[3,4-d]imidazole-lower alkylcarbonyl;
R^{D} is selected from the group consisting of alkyl, aryl, aralkyl, (±)-N-benzoylamino-lower alkyl, [3aS-(3aα,4β,6aα)]-hexahydro-2-oxo-1H-thieno[3,4-d]imidazole-lower alkyl and biphenyl; wherein the alkyl or aryl portion of the aryl or aralkyl group is optionally substituted with one to two substituents independently selected from halogen, lower alkyl, lower alkoxy, amino, lower alkylamino, di(lower alkyl)amino, azo, nitro, cyano or trifluoromethyl);
R²¹ is selected from the group consisting of hydrogen, lower alkyl, aryl, aralkyl, lower alkylcarbonyl, arylcarbonyl and aralkylcarbonyl (wherein the aryl portion of the aryl, aralkyl, arylcarbonyl or aralkylcarbonyl group is optionally substituted with one to two substituents independently selected from halogen, lower alkyl, lower alkoxy or trifluoromethyl);
p is an integer from 0 to 2;
q is an integer form 0 to 2;
R²² and R²³ are each independently selected from the group consisting of halogen, lower alkyl, lower alkoxy, amino, lower alkylamino, di(lower alkyl)amino, nitro, cyano, carboxy; lower alkoxycarbonyl, phenyloxycarbonyl, aminocarbonyl, lower alkylaminocarbonyl and di(lower alkyl)aminocarbonyl;
or a pharmaceutically acceptable salt thereof, and wherein lower alkyl refers to an alkyl containing 1 to 4 carbon atoms, lower alkoxy refers to an O-alkyl containing 1 to 4 carbon atoms and lower alkylthio refers to an S-alkyl containing 1 to 4 carbon atoms.

35. The compound as in Claim 34 wherein R²⁰ is 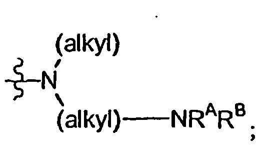 pis0 and q is 0.

36. The compound as in Claim 35 wherein
R^{A} is selected from the group consisting of hydrogen, lower alkyl and aralkyl;
R^{B} is selected from the group consisting of hydrogen, lower alkyl, halogenated lower alkyl, aralkyl, substituted aralkyl (wherein the substituents on the aralkyl are one to three independently selected from halogen, tower alkyl, lower alkoxy or trifluoromethyl), alkoxyalkyl, cycloatkyl-atkyl, dehydroabietyl, di(lower alkyl)-amino-lower alkyl, biphenyl, heteroaryl, substituted heteroaryl (wherein the substituents on the heteroaryl group are one to two independently selected from halogen or lower alkyl), heteroaryl-lower alkyl, aryloxy-lower alkyl and lower alkoxycarbonyl-lower alkyl; and
R²¹ is hydrogen;
or a pharmaceutically acceptable salt thereof, and wherein lower alkyl refers to an alkyl containing 1 to 4 carbon atoms, lower alkoxy refers to an O-alkyl containing 1 to 4 carbon atoms and lower alkylthio refers to an S-alkyl containing 1 to 4 carbon atoms.

37. The compound as in Claim 36, wherein
R^{A} is selected from the group consisting of hydrogen, methyl, ethyl and benzyl; and
R^{B} is selected from the group consisting of hydrogen, methyl, propyl, n-butyl, benzyl, phenylethyl, methoxypropyl, cyclohexylmethyl, 3-chlorobenzyl, 2,4-dimethoxybenzyl, 2-ethoxybenzyl, 2,5-difluorobenzyl, dehydroabietyl, 3,4-dimethoxybenzyl, diethylaminopropyl, 4-bromo-2-pyridyl, dimethylaminoethyl, 4-biphenyl, 2-furanylmethyl, 3-iodobenzyl, 2,2,2-trifluoroethyl, 3,4-difluorobenzyl, 2 theinylethyl, 3,5-dimethyl-2-pyridyl, 2-(ethoxy)acetyl, 2-methoxybenzyl, 4-bromobenzyl, 3,5-ditrifluoromethylbenzyl, 3-methoxyphenylethyl, 3,4,5-trimethoxybenzyl, 4-methoxyphenylethyl, 4- imidazolylethyl, 2-trifluoromethylbenzyl, 3-methoxybenzyl, 3-methylbenzyl, 3,5-dimethoxybenzyl, 2-bromobenzyl, 4-fluorobenzyl, 1-naphthylmethyl, phenoxyethyl, 4-trifluoromethylbenzyl, 3-pyridyl, 2-methylbenzyl, 2-fluorobenzyl and 3,4-dimethoxyphenyJethyl;
or a pharmaceutically acceptable salt thereof.

38. The compound as in Claim 37 wherein
R^{A} is selected from the hydrogen, methyl and benzyl; and
R^{B} is selected from the group consisting of methyl, methoxypropyl, cyclohexylmethyl, 3-chlorobenzyl, 2,5-difluorobenzyl, phenylethyl, 4-bromo-2-pyridyl, dimethylaminoethyl, n-butyl, 2-furanylmethyl, 3,4-difluorobenzyl, 2-thienylethyl, 4-bromobenzyl, 3-methoxyphenylethyl, 3,4,5-trimethoxybenzyl, benzyl, 3-methylbenzyl and phenoxyethyl;
or a pharmaceutically acceptable salt thereof.

39. The compound as in Claim 38 wherein
R^{A} is selected from the group consisting of hydrogen and methyl; and
R^{B} is selected from the group consisting of methyl, methoxypropyl, 2,5-difluorobenzyl, 3,4-difluorobenzyl, 4-bromobenzyl, 3,4,5-trimethoxybenzyl, benzyl, 3-methylbenzyl and n-butyl;
or a pharmaceutically acceptable salt thereof.

40. The compound as in Claim 39 wherein R^{A} is methyl and R^{B} is methyl; or a pharmaceutically acceptable salt thereof.

41. The compound as in Claim 35, wherein
R^{A} and R^{B} are taken together with the nitrogen atom to which they are bound to form a ring structure selected from the group consisting of heteroaryl, heterocycloalkyl and tertiarybutoxycarbonyl substituted heterocycloalkyl; and
R²¹ is hydrogen;
or a pharmaceutically acceptable salt thereof.

42. The compound as in Claim 41, wherein
R^{A}and R^{B} are taken together with the nitrogen atom to which they are bound to form a ring structure selected from the group consisting N-pyrrolidinyl, N-morpholinyl, N-imidazolyl, N-1,2,3,4-tetrahydroisoquinlinyl, N-hexamethylenenimine and N-(4-tertiarybutoxycarbonyl)piperazinyl;
or a pharmaceutically acceptable salt thereof.

43. The compound as in Claim 42 wherein
R^{A} and R^{B} are taken together with the nitrogen atom to which they are bound to form a ring structure selected from the group consisting of N-morpholinyl and N-(4-tertiarybutoxycarbonyl)piperazinyl;
or a pharmaceutically acceptable salt thereof.

44. The compound as in Claim 43 wherein
R^{A} and R^{B} are taken together with the nitrogen atom to which they are bound to form N-morpholinyl; or a pharmaceutically acceptable salt thereof.

45. The compound as in Claim 34 wherein R²⁰ is 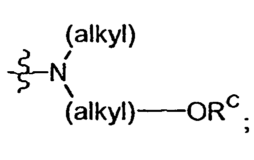 p is 0 and q is 0.

46. The compound as in Claim 45 wherein
R^{c} is selected from the group consisting of arylcarbonyl, (±)-N-benzoyl-2-aminoalkylcarbonyl and [3aS-(3aα,4(3,6aα)]-hexahydro-2-oxo-1H-thieno[3,4-d]imidazole-alkylcarbonyl; and
R²¹ is hydrogen;
or a pharmaceutically acceptable salt thereof.

47. The compound as in Claim 46, wherein
R^{c} is selected from the group consisting of benzoyl, (t)-N-benzoyl-2-aminopropionoyl and [3aS-(3aα,4β,6α)]-hexahydro-2-oxo-1H-thieno[3,4-d]imidazole-4-pentanoyl; or a pharmaceutically acceptable salt thereof.

48. The compound as in Claim 34 wherein R²⁰ is 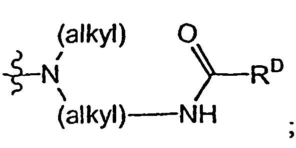 pis0 and q is 0.

49. The compound as in Claim 48 wherein
R^{D} is selected from the group consisting of (±)-N-benzoyl-2-aminoalkyl, [3aS-(3aα,4β,6aα)]-hexahydro-2-oxo-1H-thieno[3,4-d]imidazole-alkyl, alkyl, substituted aryl (wherein the substituents on the aryl are independently selected from azo, nitro, halogen, alkoxy, trifluoromethyl), biphenyl, aralkyl and substituted aralkyl (wherein the alkyl portion of the aralkyl group is substituted with a substituent selected from halogen); and
R²¹ is selected from the group consisting of hydrogen, alkylcarbonyl and substituted arylcarbonyl (wherein the substituent on the aryl portion of the arylcarbonyl group is selected from halogen or trifluoromethyl);
or a pharmaceutically acceptable salt thereof.

50. The compound as in Claim 49 wherein
R^{D} is selected from the group consisting of (±)-N-benzoyl-2-aminoeth-2-yl, [3aS-(3aα,4β,6aα)]-hexahydro-2-oxo-1H-thieno[3,4-d]imidazote-but-4-yl, 1-chloro-1-phenyl-methyl, 3-azo-6-nitrophenyl, pentacecanyl, 4-biphenyl, 4-butoxyphenyl, 4-trifluoromethylphenyl, 3-fluorophenyl and propyl; and
R²¹ is selected from the group consisting of hydrogen, propylcabronyl, 3-fluorophenylcarbonyl and 4-trifluoromethylphenylcarbonyl;
or a pharmaceutically acceptable salt thereof.

51. A pharmaceutical composition comprising a pharmaceutically acceptable carrier and a compound of claim 33.

52. A pharmaceutical composition made by mixing a compound of Claim 33 and a pharmaceutically acceptable carrier.

53. A process for making a pharmaceutical composition comprising mixing a compound of claim 33 and a pharmaceutically acceptable carrier.

54. The use of a prophylactically effective amount of the compound recited in claim 33 for the manufacture of a medicament for the reduction of ischemic death in cell population.

55. The use of claim 54, wherein at least one cell in the cell population is selected from the group consisting of a neuronal cell, a glial cell, a cardiac cell, a lymphocyte, a macrophage and a fibroblast.

56. The use of a prophylactically effective amount of the compound recited in claim 33 for the manufacture of a medicament for the reduction of death in a cell population comprising neuronal cells in response to a traumatic event, wherein said medicament is adapted for administration prior to, during, or following the traumatic event.

57. The compound as claimed in claim 33 for use as a medicament for the reduction of neuronal cell death in response to a traumatic event in a subject, wherein the compound of claim 33 is administered to the subject in a prophylactically effective amount prior to, during or following the traumatic event.

58. The compound of claim 57, wherein the traumatic event is selected from the group consisting of a medical disorder, a physical trauma, a chemical trauma and a biological trauma.

59. The use of a compound of claim 33 for the preparation of a medicament for reducing the likelihood of a cell's undergoing ischemic death, in a subject in need thereof.

## Patentansprüche

1. Pharmazeutische Zusammensetzung, umfassend einen pharmazeutisch verträglichen Trägerstoff und eine Verbindung mit der Formel 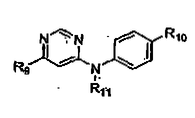 oder ein pharmazeutisch verträgliches Salz davon, worin
(a) R₉ ausgewählt ist aus der Gruppe, bestehend aus H, Thienyl, Furanyl, Pyrrolyl, Phenyl, substituiertem Phenyl, Pyridinyl, substituiertem Pyridinyl, Naphthyl, Benzo[b]thien-2-yl, 2-Benzofuranyl, Pyrimidin und 2,4-(Bismethoxyphenyl)-5-pyrimidinyl,
wobei das substituierte Phenyl die Formel besitzt 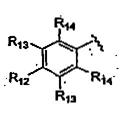 worin (i) R₁₂H, OH, Niederalkylthio, Alkoxy, Alkylamin, Dialkylamin, Halogen-substituiertes Niederalkyl, Halogen-substituiertes Niederalkoxy, Cyano, Cyanoalkyl, Phenyl, Phenylalkoxy oder substituiertes Piperazinyl, N-(t-Butoxy)carbamylalkyl ist, (ii) R₁₃ jeweils unabhängig H, NO₂ Alkoxy, Alkylamino, Dialkylamino, Halogen-substituiertes Niederalkyl, Halogen-substituiertes Niederalkoxy oder Phenyl ist und (iii) R₁₄ jeweils unabhängig H, Alkoxy, Phenyloxy oder Phenylalkoxy ist;
(b) R₁₀ ausgewählt ist aus der Gruppe, bestehend aus Cyanoalkyl, Alkylamino, Dialkylamino, Hydroxy-substituiertem Alkylamino und Hydroxy-substituiertem Dialkylamino; und
(c) R₁₁ H oder Niederalkyl ist,
und wobei Niederalkyl sich auf ein Alkyl bezieht, das 1 bis 4 Kohlenstoffatome enthält, Niederalkoxy sich auf ein O-Alkyl bezieht, das 1 bis 4 Kohlenstoffatome enthält, und Niederalkylthio sich auf ein S-Alkyl bezieht, das 1 bis 4 Kohlenstoffatome enthält.

2. Pharmazeutische Zusammensetzung nach Anspruch 1, wobei R₉ substituiertes Phenyl ist.

3. Pharmazeutische Zusammensetzung nach Anspruch 1, wobei R₁₁ H ist und R₁₀ Dialkylamino oder Hydroxy-substituiertes Dialkylamino ist.

4. Pharmazeutische Zusammensetzung nach Anspruch 1, wobei die Verbindung N1,N1-Dimethyl-N4-[6-[4-(phenylmethoxy)phenyl]-4-pyrimidinyl]-1,4-benzoldiamin ist.

5. Pharmazeutische Zusammensetzung nach Anspruch 1, wobei die Verbindung N1-(6-[1,1'-Biphenyl]-3-yl-4-pyritnidinyl)-N4,N4-dimethyl-1,4-benzoldiamin ist,

6. Pharmazeutische Zusammensetzung nach Anspruch 1, wobei die Verbindung N1-[6-[3,5-Bis(trifluormethyl)phenyl]-4-pyrimidinyl]-N4,N4-dimethyl-1,4-benzoldiamin ist.

7. Pharmazeutische Zusammensetzung nach Anspruch 1, wobei die Verbindung 2-[[4-[(6-[1,1'-Biphenyl]-3-yl-4-pyrimidinyl)amino]phenyl]ethylamino]-ethanol ist.

8. Pharmazeutische Zusammensetzung nach Anspruch 1, wobei die Verbindung 2-[[4-[(6-Benzo[b]thien-2-yl-4-pyrimidinyl)amino]phenyl]ethylamino]-ethanol ist.

9. Pharmazeutische Zusammensetzung nach Anspruch 1, wobei die Verbindung 2-[Ethyl[4-[[6-[4-(trifluorrnethoXy)phcnyl]-4.pyrimidinyl]arnino]phenyl]arnino]-ethanol ist.

10. Pharmazeutische Zusammensetzung nach Anspruch 1, wobei die Verbindung die Formel besitzt

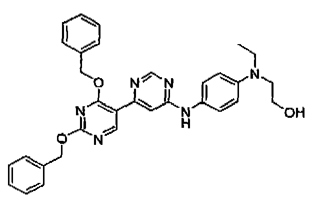

11. Pharmazeutische Zusammensetzung, umfassend einen pharmazeutisch verträglichen Trägerstoff und eine Verbindung mit der Formel 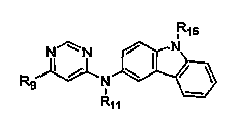 oder ein pharmazeutisch annehmbares Salz davon, worin
(a) R₉ ausgewählt ist aus der Gruppe, bestehend aus H, Thienyl, Furanyl, Pyrrolyl, Phenyl, substituiertem Phenyl, Pyridinyl, substituiertem Pyridinyl, Naphthyl, Beilzo[b]thien-2-yl, 2-Benzofuranyl, Pyrimidin und 2,4-(Bismethoxyphenyl)-5-pyrimidinyl,
wobei das substituierte Phenyl die Formel besitzt 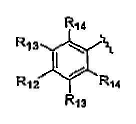 worin (i) R₁₂ H, OH, Niederalkylthio, Alkoxy, Alkylamin, Dialkylamin, Halogen-substituiertes Niederalkyl, Halogen-substituiertes Niederalkoxy, Cyano, Cyanoalkyl, Phenyl, Phenylalkoxy oder substituiertes Piperazinyl, N-(t-Butoxy)carbamylalkyl ist, (ii) R₁₃ jeweils unabhängig H, NO₂ Alkoxy, Alkylainino, Dialkylamino, flalogen-substituiertes Niederalkyl, Halogen-substituiertes Niederalkoxy oder Phenyl ist und (iii) R₁₄ jeweils unabhängig H, Alkoxy, Phenyloxy oder Phenylalkoxy ist;
(b) R₁₁ H oder Niederalkyl ist; und
(c) R₁₅ H oder Alkyl ist,
und wobei Niederalkyl sich auf ein Alkyl bezieht, das 1 bis 4 Kohlenstoffatome enthält, Niederalkoxy sich auf ein O-Alkyl bezieht, das 1 bis 4 Kohlenstoffatome enthält, und Niederalkylthio sich auf ein S-Alkyl bezieht, das 1 bis 4 Kohlenstoffatome enthält.

12. Verwendung einer prophylaktisch wirksamen Menge der in Anspruch 1 oder Anspruch 11 angegebenen Verbindung zur Herstellung eines Arzneimittels zur Verringerung von ischämischem Tod in einer Zellpopulation.

13. Verwendung nach Anspruch 12, wobei die Zellpopulation eine Zelle umfasst, die ausgewählt ist aus der Gruppe, bestehend aus einer Nervenzelle, einer Gliazelle, einer Herzzelle, einem Lymphozyten, einem Makrophagen und einem Fibroblasten.

14. Verwendung einer prophylaktisch wirksamen Menge der in Anspruch 1 oder Anspruch 11 angegebenen Verbindung zur Herstellung eines Arzneimittels zur Verringerung von Tod in einer Zellpopulation, die Nervenzellen umfasst, in Reaktion auf ein traumatisches Ereignis, wobei das Arzneimittel angepasst ist zur Verabreichung vor, während oder innerhalb eines geeigneten Zeitraums im Anschluss an das traumatische Ereignis.

15. Verfahren zur Verringerung von ischämischem Tod in einer Zellpopulation, das das Inkontaktbringen der Zelle mit einer prophylaktisch wirksamen Menge der in Anspruch 1 oder Anspruch 11 angegebenen Verbindung umfasst, wobei das Inkontaktbringen ex vivo durchgeführt wird.

16. Verfahren zur Verringerung von Tod in einer Zellpopulation, die Nervenzellen umfasst, in Reaktion auf ein traumatisches Ereignis, das das Inkontaktbringen der Nervenzelle mit einer prophylaktisch wirksamen Menge der in Anspruch 1 oder Anspruch 11 angegebenen Verbindung vor, während oder innerhalb eines geeigneten Zeitraums im Anschluss an das traumatische Ereignis umfasst, wobei das Inkontaktbringen ex vivo durchgeführt wird.

17. Pharmazeutische Zusammensetzung nach Anspruch 1 zur Verwendung als ein Arzneimittel zur Verringerung von Nervenzelltod in Reaktion auf ein traumatisches Ereignis in einem Patienten, wobei die pharmazeutische Zusammensetzung von Anspruch 1 dem Patienten in einer prophylaktisch wirksamen Menge vor, während oder im Anschluss an das traumatische Ereignis verabreicht wird.

18. Pharmazeutische Zusammensetzung nach Anspruch 11 zur Verwendung als ein Arzneimittel zur Verringerung von Nervenzelltod in Reaktion auf ein traumatisches Ereignis in einem Patienten, wobei die pharmazeutische Zusammensetzung von Anspruch 11 dem Patienten in einer prophylaktisch wirksamen Menge vor, während oder im Anschluss an das traumatische Ereignis verabreicht wird.

19. Pharmazeutische Zusammensetzung nach Anspruch 17, wobei der Patient ein Mensch ist.

20. Pharmazeutische Zusammensetzung nach Anspruch 17, wobei das traumatische Ereignis ausgewählt ist aus der Gruppe, bestehend aus einer medizinischen Störung, einem physischen Trauma, einem chemischen Trauma und einem biologischen Trauma.

21. Pharmazeutische Zusammensetzung nach Anspruch 17, wobei die pharmazeutische Zusammensetzung vor dem traumatischen Ereignis verabreicht wird.

22. Pharmazeutische Zusammensetzung nach Anspruch 17, wobei die pharmazeutische Zusammensetzung während des traumatischen Ereignisses verabreicht wird.

23. Pharmazeutische Zusammensetzung nach Anspruch 17, wobei die pharmazeutische Zusammensetzung im Anschluss an das traumatische Ereignis verabreicht wird.

24. Pharmazeutische Zusammensetzung nach Anspruch 18, wobei der Patient ein Mensch ist.

25. Pharmazeutische Zusammensetzung nach Anspruch 18, wobei das traumatische Ereignis ausgewählt ist aus der Gruppe, bestehend aus einer medizinischen Störung, einem physischen Trauma, einem chemischen Trauma und einem biologischen Trauma.

26. Pharmazeutische Zusammensetzung nach Anspruch 18, wobei die pharmazeutische Zusammensetzung vor dem traumatischen Ereignis verabreicht wird.

27. Pharmazeutische Zusammensetzung nach Anspruch 18, wobei die pharmazeutische Zusammensetzung während des traumatischen Ereignisses verabreicht wird.

28. Pharmazeutische Zusammensetzung nach Anspruch 18, wobei die pharmazeutische Zusammensetzung im Anschluss an das traumatische Ereignis verabreicht wird.

29. Vorrichtung zur Verabreichung der pharmazeutischen Zusammensetzung von Anspruch 1 an einen Patienten, die einen Behälter und die pharmazeutische Zusammensetzung von Anspruch 1 darin umfasst, wobei der Behälter eine Einrichtung zur Abgabe einer prophylaktischen Dosis der pharmazeutischen Zusammensetzung an den Patienten aufweist.

30. Vorrichtung zur Verabreichung der pharmazeutischen Zusammensetzung von Anspruch 11 an einen Patienten, die einen Behälter und die pharmazeutische Zusammensetzung von Anspruch 11 darin umfasst, wobei der Behälter eine Einrichtung zur Abgabe einer prophylaktischen Dosis der pharmazeutischen Zusammensetzung an den Patienten aufweist.

31. Vorrichtung nach Anspruch 29, wobei die Vorrichtung zur Abgabe der pharmazeutischen Zusammensetzung eine Spritze ist.

32. Vorrichtung nach Anspruch 30, wobei die Vorrichtung zur Abgabe der pharmazeutischen Zusammensetzung eine Spritze ist.

33. Verbindung mit der Formel 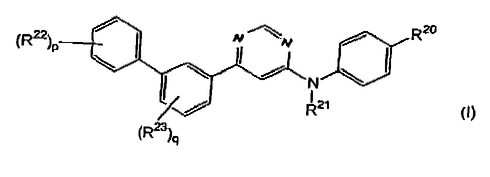 worin
R²⁰ ausgewählt ist aus der Gruppe, bestehend aus 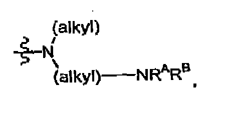 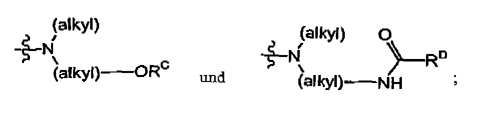
worin R^{A} und R^{B} unabhängig ausgewählt sind aus Wasserstoff, Alkyl, halogeniertem Alkyl, Amino, Alkylamino, Dialkylamino, Aminoalkyl, Alkylaminoalkyl, Dialkylaminoalkyl, Aryl, Aralkyl, Cycloalkyl, Cycloalkylalkyl, Heteroaryl, Heteroarylalkyl, Alkoxyalkyl, Aryloxy, Aryloxyalkyl, Alkoxycarbonylalkyl und Dehydroabietyl; wobei der Aryl-, Cycloalkyl-, Heteroaryl- oder FIeterocycloalkylteil jeder der Gruppen gegebenenfalls mit einem oder mehr Substituenten substituiert ist, die unabhängig ausgewählt sind aus Halogen, Alkyl, Alkoxy, halogeniertem Alkyl, Amino, Alkylamino, Dialkylamino, Arylamino, Aralkylamino, Amido, Alkylamido, Dialkylamido, Arylamido, Aralkylamido, Azo, Nitro, Cyano, Aryl, Aralkyl, Aryloxy, Carboxy, Alkoxycarbonyl, Aryloxycarbonyl, Alkylthio, Arylthio, AlkylsulfonylN(H) oder AlkylsulfonylN(alkyl);
alternativ R^{A} und R^{B} mit dem Stickstoffatom, an das sie gebunden sind, zusammengenommen sein können, um eine Verbindung zu bilden, die ausgewählt ist aus der Gruppe Heteroaryl und Heterocycloalkyl; wobei das Heteroaryl oder Heterocycloalkyl gegebenenfalls mit einem oder mehreren Substituenten substituiert ist, die unabhängig ausgewählt sind aus Halogen, Alkyl, Alkoxy, Alkoxycarbonyl, halogeniertem Alkyl, Alkylcarbonyl, Amino, Alkylamino, Dialkylamino, Arylamino, Azo, Nitro oder Cyano;
R^{c} ausgewählt ist aus der Gruppe, bestehend aus Alkyl, Aralkyl, Alkylcarbonyl, Arylcarbonyl, Aralkylearbonyl, (±)-N-Benzoylaminoalkylcarbonyl oder [3aS-(3aα,4bβ,6aα)]-Hexahydro-2-oxo-1H-thieno[3,4-d]imidazolalkylcarbonyl;
R^{D} ausgewählt ist aus Alkyl, Aryl, Aralkyl, (±)-N-Benzoylaininoalkyl, [3aS-(3aα,4b,β,6aα)]-Hexahydro-2-oxo-1H-thieno[3,4-d]imidazolalkyl oder Biphenyl; wobei der Alkyl- oder Arylteil der Alkyl-, Aryl- oder Aralkylgruppe gegebenenfalls mit einem oder mehreren Substituenten substituiert ist, die unabhängig ausgewählt sind aus Halogen, Alkyl, Alkoxy, Amino, Alkylamino, Dialkylamino, Azo, Nitro, Cyano oder Trifluormethyl;
R²¹ ausgewählt ist aus der Gruppe, bestehend Wasserstoff, Alkyl, Aryl, Aralkyl, Alkylcarbonyl, Arylcarbonyl und Aralkylearbonyl; wobei der Arylteil gegebenenfalls mit einem oder mehreren Substituenten substituiert ist, die unabhängig ausgewählt sind aus Halogen, Alkyl, Alkoxy, halogeniertem Alkyl oder Nitro;
p eine ganze Zahl ist, die ausgewählt ist von 0 bis 3;
q eine ganze Zahl ist, die ausgewählt ist von 0 bis 3;
R²² und R²³ jeweils unabhängig ausgewählt sind aus der Gruppe, bestehend aus Halogen, Alkyl, Alkoxy, Amino, Alkylamino, Dialkylamino, Nitro, Cyano, Carboxy, Alkoxycarbonyl, Aryloxycarbonyl, Aminocarbonyl, Alkylaminocarbonyl und Dialkylaminocarbonyl;
oder ein pharmazeutisch verträgliches Salz davon.

34. Verbindung nach Anspruch 33, wobei
R^{A} ausgewählt ist aus der Gruppe, bestehend aus Wasserstoff, Alkyl, Aryl und Aralkyl; wobei der Arylteil jeder der Gruppen gegebenenfalls mit einem bis drei Substituenten substituiert ist, die unabhängig ausgewählt sind aus Halogen, Niederalkyl, Niederalkoxy, halogeniertem Niederalkyl, Amino, Niederalkylamino, Di(niederalkyl)amino, Arylamino, Aralkylamino, Azo, Nitro, Cyano, Aryl, Aralkyl, Aryloxy, Carboxy, Niederalkoxycarbonyl, Aryloxycarbonyl, Niederalkylthio und Arylthio;
R^{B} ausgewählt ist aus der Gruppe, bestehend aus Wasserstoff, Alkyl, halogeniertem Niederalkyl, Amino, Niederalkylamino, Di(niederalkyl)amino, Aminoniederalkyl, Niederalkylaminoniederalkyl, Di(niederalkyl)aminoniederalkyl, Aryl, Aralkyl, Cycloalkyl, Cycloalkylniederalkyl, Heteroaryl, Heteroarylniederalkyl, Niederalkoxyniederalkyl, Aryloxy, Aryloxyniederalkyl, Niederalkoxycarbonylniederalkyl und Dehydroabietyl; wobei der Aryl-, Cycloalkyl-, Heteroaryl- oder Heterocycloalkylteil jeder der Gruppen gegebenenfalls mit einem bis drei Substituenten substituiert ist, die unabhängig ausgewählt sind aus Halogen, Niederalkyl, Niederalkoxy, halogeniertem Niederalkyl, Amino, Niederalkylamino, Di(niederalkyl)amino, Arylamino, Aralkylamino, Azo, Nitro, Cyano, Aryl, Aralkyl, Aryloxy, Carboxy, Niederalkoxycarbonyl, Aryloxycarbonyl, Niederalkylthio und Arylthio;
alternativ R^{A} und R^{B} mit dem Stickstoffatom, an das sie gebunden sind, zusammengenommen sind, um eine Ringstruktur zu bilden, die ausgewählt ist aus der Gruppe, bestehend aus Heteroaryl und Heterocycloalkyl; wobei die Heteroaryl- oder Heterocycloalkylgruppe gegebenenfalls mit einem bis zwei Substituenten substituiert ist, die ausgewählt sind aus Halogen, Niederalkyl, Niederalkoxy, Niederalkoxycarbonyl, Trifluormethyl, Niederalkylearbonyl, Amine, Niederalkylamino, Di(niederalkyl)amino, Arylamino, Azo, Nitro oder Cyano;
R^{c} ausgewählt ist aus der Gruppe, bestehend aus Niederalkyl, Aralkyl, Niederalkylcarbonyl, Arylcarbonyl, Aralkylcarbonyl, (±)-N-Benzoylaminoniederalkylcarbonyl oder [3aS-(3aα,4β,6aα)]-Hexahydro-2-oxo-1H-thieno[3,4-d]imidazolniederalkylcarbonyl;
R^{D} ausgewählt ist aus der Gruppe, bestehend aus Alkyl, Aryl, Aralkyl, (±)-N-Benzoylaminoniederalkyl, [3aS-(3aα,4β,6aα)]-Hexahydro-2-oxo-1H-thieno[3,4-d]imidazolniederalkyl und Biphenyl; wobei der Alkyl- oder Arylteil der Alkyl-, Aryl- oder Aralkylgruppe gegebenenfalls mit einem bis zwei Substituenten substituiert ist, die unabhängig ausgewählt sind aus Halogen, Niederalkyl, Niederalkoxy, Amino, Niederalkylamino, Di(niederalkyl)amino, Azo, Nitro, Cyano oder Trifluormethyl;
R²¹ ausgewählt ist aus der Gruppe, bestehend Wasserstoff, Niederalkyl, Aryl, Aralkyl, Niederalkylcarbonyl, Arylcarbonyl und Aralkylearbonyl (wobei der Arylteil der Aryl-, Aralkyl-, Arylcarbonyl- oder Aralkylcarbonylgruppe gegebenenfalls mit einem bis zwei Substituenten substituiert ist, die unabhängig ausgewählt sind aus Halogen, Niederalkyl, Niederalkoxy oder Trifluormethyl);
p eine ganze Zahl von 0 bis 2 ist;
q eine ganze Zahl von 0 bis 2 ist;
R²² und R²³ jeweils unabhängig ausgewählt sind aus der Gruppe, bestehend aus Halogen, Niederalkyl, Niederalkoxy, Amino, Niederalkylamino, Di(niederalkyl)amino, Nitro, Cyano, Carboxy, Niederalkoxycarbonyl, Phenyloxycarbonyl, Aminocarbonyl, Niederalkylaminocarbonyl und Di(niederalkyl)aminocarbonyl;
oder ein pharmazeutisch verträgliches Salz davon,
und wobei Niederalkyl sich auf ein Alkyl bezieht, das 1 bis 4 Kohlenstoffatome enthält, Niederalkoxy sich auf ein O-Alkyl bezieht, das 1 bis 4 Kohlenstoffatome enthält, und Niederalkylthio sich auf ein S-Alkyl bezieht, das 1 bis 4 Kohlenstoffatome enthält.

35. Verbindung nach Anspruch 34, wobei R²⁰ 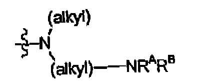 ist; p 0 ist und q 0 ist.

36. Verbindung nach Anspruch 35, wobei
R^{A} ausgewählt ist aus der Gruppe, bestehend aus Wasserstoff, Niederalkyl und Aralkyl;
R^{B} ausgewählt ist aus der Gruppe, bestehend aus Wasserstoff, Niederalkyl, halogeniertem Niederalkyl, Aralkyl, substituiertem Aralkyl (wobei die Substituenten auf dem Aralkyl ein bis drei sind, die unabhängig ausgewählt sind aus Halogen, Niederalkyl, Niederalkoxy oder Trifluormethyl), Alkoxyalkyl, Cycloalkylalkyl, Dehydroabietyl, Di(niederalkyl)aminoniederalkyl, Biphenyl, Heteroaryl, substituiertem Heteroaryl (wobei die Substituenten auf der Heteroarylgruppe ein bis zwei sind, die unabhängig ausgewählt sind aus Halogen oder Niederalkyl), Heteroarylniederalkyl, Aryloxyniederalkyl und Niederalkoxycarbonylniederalkyl; und
R²¹ Wasserstoff ist;
oder ein pharmazeutisch verträgliches Salz davon,
und wobei Niederalkyl sich auf ein Alkyl bezieht, das 1 bis 4 Kohlenstoffatome enthält, Niederalkoxy sich auf ein O-Alkyl bezieht, das 1 bis 4 Kohlenstoffatome enthält, und Niederalkylthio sich auf ein S-Alkyl bezieht, das 1 bis 4 Kohlenstoffatome enthält.

37. Verbindung nach Anspruch 36, wobei
R^{A} ausgewählt ist aus der Gruppe, bestehend aus Wasserstoff, Methyl, Ethyl und Benzyl; und
R^{B} ausgewählt ist aus der Gruppe, bestehend aus Wasserstoff, Methyl, Propyl, n-Butyl, Benzyl, Phenylethyl, Methoxypropyl, Cyclohexylmethyl, 3-Chlorbenzyl, 2,4-Dimethoxybenzyl, 2-Ethoxybenzyl, 2,5-Difluorbenzyl, Dehydroabietyl, 3,4-Dimethoxybenzyl, Diethylaminopropyl, 4-Brom-2-pyridyl, Dimethylaminoethyl, 4-Biphenyl, 2-Furanylmethyl, 3-Iodbenzyl, 2,2,2-Trifluorethyl, 3,4-Difluorbenzyl, 2-Thienylethyl, 3,5-Dimethyl-2-pyridyl, 2-(Ethoxy)acetyl, 2-Methoxybenzyl, 4-Brombenzyl, 3,5-Ditrifluormethylbenzyl, 3-Methoxyphenylethyl, 3,4,5-Trimethoxybenzyl, 4-Methoxyphenylethyl, 4-Imidazolylethyl, 2-Trifluormethylbenzyl, 3-Methoxybenzyl, 3-Methylbenzyl, 3,5-Dimethoxybenzyl, 2-Brombenzyl, 4-Fluorbenzyl, 1-Naphthylmethyl, Phenoxyethyl, 4-Trifluormethytbenzyl, 3-Pyridyl, 2-Methylbenzyl, 2-Fluorbenzyl und 3,4-Dimethoxyphenylethyl;
oder ein pharmazeutisch verträgliches Salz davon.

38. Verbindung nach Anspruch 37, wobei
R^{A} ausgewählt ist aus Wasserstoff, Methyl und Benzyl; und
R^{B} ausgewählt ist aus der Gruppe, bestehend aus Methyl, Methoxypropyl, Cyclohexylmethyl, 3-Chlorbenzyl, 2,5-Difluorbenzyl, Phenylethyl, 4-Brom-2-pyridyl, Dimethylaminoethyl, n-Butyl, 2-Furanylmethyl, 3,4-Difluorbenzyl, 2-Thienylethyl, 4-Brombenzyl, 3-Methoxyphenylethyl, 3,4,5-Trimethoxybenzyl, Benzyl, 3-Methylbenzyl und Phenoxyethyl;
oder ein pharmazeutisch verträgliches Salz davon.

39. Verbindung nach Anspruch 38, wobei
R^{A} ausgewählt ist aus der Gruppe, bestehend aus Wasserstoff und Methyl; und
R^{B} ausgewählt ist aus der Gruppe, bestehend aus Methyl, Methoxypropyl, 2,5-Difluorbenzyl, 3,4-Difluorbenzyl, 4-Brombenzyl, 3,4,5-Trimethoxybenzyl, Benzyl, 3-Methylbenzyl und n-Butyl;
oder ein pharmazeutisch verträgliches Salz davon.

40. Verbindung nach Anspruch 39, wobei R^{A} Methyl ist und R^{B} Methyl ist; oder ein pharmazeutisch verträgliches Salz davon.

41. Verbindung nach Anspruch 35, wobei
R^{A} und R^{B} mit dem Stickstoffatom, an das sie gebunden sind, zusammengenommen sind, um eine Ringstruktur zu bilden, die ausgewählt ist aus der Gruppe, bestehend aus Heteroaryl, Heterocycloalkyl und tert-Butoxycarbonyl-substituiertem Heterocycloalkyl; und
R²¹ Wasserstoff ist;
oder ein pharmazeutisch verträgliches Salz davon.

42. Verbindung nach Anspruch 41, wobei
R^{A} und R^{B} mit dem Stickstoffatom, an das sie gebunden sind, zusammengenommen sind, um eine Ringstruktur zu bilden, die ausgewählt ist aus der Gruppe, bestehend aus N-Pyrrolidinyl, N-Morpholinyl, N-Imidazolyl, N-1,2,3,4-Tetrahydroisochinolinyl, N-Hexamethylenimin und N-(4-tert-Butoxycarbonyl)piperazinyl;
oder ein pharmazeutisch verträgliches Salz davon.

43. Verbindung nach Anspruch 42, wobei
R^{A} und R^{B} mit dem Stickstoffatom, an das sie gebunden sind, zusammengenommen sind, um eine Ringstruktur zu bilden, die ausgewählt ist aus der Gruppe, bestehend aus N-Morpholinyl und N-(4-tert-Butoxycarbonyl)piperazinyl;
oder ein pharmazeutisch verträgliches Salz davon.

44. Verbindung nach Anspruch 43, wobei
R^{A} und R^{B} mit dem Stickstoffatom, an das sie gebunden sind, zusammengenommen sind, um N-Morpholinyl zu bilden; oder ein pharmazeutisch verträgliches Salz davon.

45. Verbindung nach Anspruch 34, wobei R²⁰ 0 ist und q 0 ist. 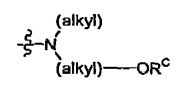 ist; P

46. Verbindung nach Anspruch 45, wobei
R^{c} ausgewählt ist aus der Gruppe, bestehend aus Arylcarbonyl, (±)-N-Benzoyl-2-aminoalkylcarbonyl und [3aS-(3aα.4β,6aα)]-Hexahydro-2-oxo-1H-thieno[3,4-d]imidazolalkylcarbonyl; und
R²¹ Wasserstoff ist;
oder ein pharmazeutisch verträgliches Salz davon

47. Verbindung nach Anspruch 46, wobei
R^{c} ausgewählt ist aus der Gruppe, bestehend aus Benzoyl, (±)-N-Benzoyl-2-aminopropionyl und [3a5-(3aα,4β,6aα)]-Hexahydro-2-oxo-1H-thieno[3,4-d]imidazol-4-pentanoyl; oder ein pharmazeutisch verträgliches Salz davon.

48. Verbindung nach Anspruch 34, wobei R²⁰ 0 ist und q 0 ist. 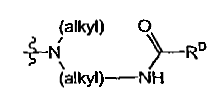 ist; p

49. Verbindung nach Anspruch 48, wobei
R^{D} ausgewählt ist aus der Gruppe, bestehend aus (±)-N-Benzoyl-2-aminoalkyl, [3aS-(3aα.4β,6aα)]-Hexahydro-2-oxo-1H-thieno[3,4-d]imidazolalkyl, Alkyl, substituiertem Aryl (wobei die Substituenten auf dem Aryl unabhängig ausgewählt sind aus Azo, Nitro, Halogen, Alkoxy, Trifluormethyl), Biphenyl, Aralkyl und substituiertem Aralkyl (wobei der Alkylteil der Aralkylgruppe mit einem Substituenten substituiert ist, der ausgewählt ist aus Halogen); und
R²¹ ausgewählt ist aus der Gruppe, bestehend aus Wasserstoff, Alkylcarbonyl und substituiertem Arylcarbonyl (wobei der Substituent auf dem Arylteil der Arylcarbonylgruppe ausgewählt ist aus Halogen oder Trifluormethyl);
oder ein pharmazeutisch verträgliches Salz davon.

50. Verbindung nach Anspruch 49, wobei
R^{D} ausgewählt ist aus der Gruppe, bestehend aus (±)-N-Benzoyl-2-aminoeth-2-yl, [3aS-(3aα,4β,6aα)]- Hexahydro-2-oxo-1H-thieno[3 ,4-d]imidazolbut-4-yl, 1-Chlor-1-phenylmethyl, 3-Azo-6-nitrophenyl, Pentadecanyl, 4-Biphenyl, 4-Butoxyphenyl, 4-Trifluormethylphenyl, 3-Fluorphenyl und Propyl; und
R²¹ ausgewählt ist aus der Gruppe, bestehend aus Wasserstoff, Propylcarbonyl, 3-Fluorphenylcarbonyl und 4-Trifluormethylphenylcarbonyl;
oder ein pharmazeutisch verträgliches Salz davon.

51. Pharmazeutische Zusammensetzung, die einen pharmazeutisch verträglichen Trägerstoff und eine Verbindung nach Anspruch 33 umfasst.

52. Pharmazeutische Zusammensetzung, hergestellt durch Vermischen einer Verbindung nach Anspruch 33 und eines pharmazeutisch verträglichen Trägerstoffes.

53. Verfahren zur Herstellung einer pharmazeutischen Zusammensetzung, das das Vermischen einer Verbindung nach Anspruch 33 und eines pharmazeutisch verträglichen Trägerstoffes umfasst.

54. Verwendung einer prophylaktisch wirksamen Menge der in Anspruch 33 angegebenen Verbindung zur Herstellung eines Arzneimittels zur Verringerung von ischämischem Tod in Zellpopulation.

55. Verwendung nach Anspruch 54, wobei wenigstens eine Zelle in der Zellpopulation ausgewählt ist aus der Gruppe, bestehend aus einer Nervenzelle, einer Gliazelle, einer Herzzelle, einem Lymphozyten, einem Makrophagen und einem Fibroblasten.

56. Verwendung einer prophylaktisch wirksamen Menge der in Anspruch 33 angegebenen Verbindung zur Herstellung eines Arzneimittels zur Verringerung von Tod in einer Zellpopulation, die Nervenzellen umfasst, in Reaktion auf ein traumatisches Ereignis, wobei das Arzneimittel angepasst ist zur Verabreichung vor, während oder im Anschluss an das traumatische Ereignis.

57. Verbindung nach Anspruch 33 zur Verwendung als ein Arzneimittel zur Verringerung von Nervenzelltod in Reaktion auf ein traumatisches Ereignis in einem Patienten, wobei die Verbindung von Anspruch 33 dem Patienten in einer prophylaktisch wirksamen Menge vor, während oder im Anschluss an das traumatische Ereignis verabreicht wird.

58. Verbindung nach Anspruch 57, wobei das traumatische Ereignis ausgewählt ist aus der Gruppe, bestehend aus einer medizinischen Störung, einem physischen Trauma, einem chemischen Trauma und einem biologischen Trauma.

59. Verwendung einer Verbindung nach Anspruch 33 zur Herstellung eines Arzneimittels zur Verringerung der Wahrscheinlichkeit, dass eine Zelle ischämischen Tod erleidet, bei einem Patienten, der derselben bedarf.

## Revendications

1. Composition pharmaceutique comprenant un véhicule pharmaceutiquement acceptable et un composé ayant la formule 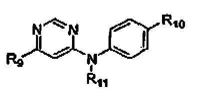 ou un sel pharmaceutiquement acceptable de celui-ci, dans laquelle
(a) R₉ est choisi dans le groupe consistant en H, un groupe thiényle, furanyle, pyrrolyle, phényle, phényle substitué, pyridinyle, pyridinyle substitué, naphtyle, benzo[b]thién-2-yle, 2-benzofuranyle, pyrimidine et 2,4-(bisméthoxyphényl)-5-pyrimidinyle,
ledit groupe phényle substitué ayant la formule 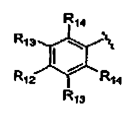 dans laquelle (i) R₁₂ est H, OH, un groupe alkylthio inférieur, alcoxy, alkylamine, dialkylamine, alkyle inférieur substitué par un atome d'halogène, alcoxy inférieur substitué par un atome d'halogène, cyano, cyanoalkyle, phényle, phénylalcoxy ou pipérazinyle substitué, N-(t-butoxy)carbamylalkyle, (ii) chaque R₁₃ est indépendamment H, NO₂, un groupe alcoxy, alkylamino, dialkylamino, alkyle inférieur substitué par un atome d'halogène, alcoxy inférieur substitué par un atome d'halogène ou phényle, et (iii) chaque R₁₄ est indépendamment H, un groupe alcoxy, phényloxy ou phénylalcoxy;
(b) R₁₀ est choisi dans le groupe consistant en un groupe cyanoalkyle, alkylamino, dialkylamino, alkylamino substitué par un groupe hydroxy et dialkylamino substitué par un groupe hydroxy; et
(c) R₁₁ est H ou un groupe alkyle inférieur,
et dans laquelle alkyle inférieur fait référence à un groupe alkyle contenant 1 à 4 atomes de carbone, alcoxy inférieur fait référence à un groupe O-alkyle contenant 1 à 4 atomes de carbone et alkylthio inférieur fait référence à un groupe S-alkyle contenant 1 à 4 atomes de carbone.

2. Composition pharmaceutique selon la revendication 1, dans laquelle R₉ est un groupe phényle substitué.

3. Composition pharmaceutique selon la revendication 1, dans laquelle R₁₁ est H et R₁₀ est un groupe dialkylamino ou dialkylamino substitué par un groupe hydroxy.

4. Composition pharmaceutique selon la revendication 1, dans laquelle le composé est la N1,N1-diméthyl-N4-[6-[4-(phénylméthoxy)phényl]-4-pyrimidinyl]-1,4-benzènediamine.

5. Composition pharmaceutique selon la revendication 1, dans laquelle le composé est la N1-(6-[1,1'-(biphényl]-3-yl-4-pyrimidinyl]-N4,N4-diméthyl-1,4-benzènediamine.

6. Composition pharmaceutique selon la revendication 1, dans laquelle le composé est la N1-[6-[3,5-bis(trifluorométhyl)phényl]-4-pyrimidinyl]-N4,N4-diméthyl-1,4-benzénediamine.

7. Composition pharmaceutique selon la revendication 1, dans laquelle le composé est le 2-[[4-[(6-[1,1'-biphényl]-3-yl-4-pyrimxdinyl)amino]phényl]éthylamino]-éthanol.

8. Composition pharmaceutique selon la revendication 1, dans laquelle le composé est le 2-[[4-[(6-benzo[b] thién-2-yl-4-pyrimidinyl)amino]phényl]éthylamino]-éthanol,

9. Composition pharmaceutique selon la revendication 1, dans laquelle le composé est le 2-[éthyl[4-[[6-[4-trifluorométhoxy)phényl]-4-pyrimidinyl]amino]phényl]amino]-éthanol.

10. Composition pharmaceutique selon la revendication 1, dans laquelle le composé est de formule

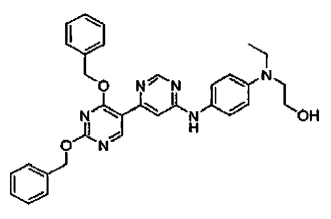

11. Composition pharmaceutique comprenant un véhicule pharmaceutiquement acceptable et un composé ayant la formule 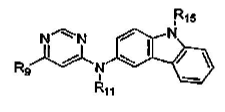 ou un sel pharmaceutiquement acceptable de celui-ci, dans laquelle
(a) R₉ est choisi dans le groupe consistant en H, un groupe thiényle, furanyle, pyrrolyle, phényle, phényle substitué, pyridinyle, pyridinyle substitué, naphtyle, benzo[b]thién-2-yle, 2-benzofuranyle, pyrimidine et 2,4-(bisméthoxyphényl)-5-pyrimidinyle,
ledit groupe phényle substitué ayant la formule 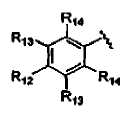 dans laquelle (i) R₁₂ est H, OH, un groupe alkylthio inférieur, alcoxy, alkylamine, dialkylamine, alkyle inférieur substitué par un atome d'halogène, alcoxy inférieur substitué par un atome d'halogène, cyano, cyanoalkyle, phényle, phénylalcoxy ou pipérazinyle substitué, N-(t-butoxy)carbamylalkyle, (ii) chaque R₁₃ est indépendamment H, NO₂ un groupe alcoxy, alkylamino, dialkylamino, alkyle inférieur substitué par un atome d'halogène, alcoxy inférieur substitué par un atome d'halogène ou phényle, et (iii) chaque R₁₄ est indépendamment H, un groupe alcoxy, phényloxy ou phénylalcoxy;
(b) R₁₁ est H ou un groupe alkyle inférieur; et
(c) R₁₅ est H ou un groupe alkyle,
et dans laquelle alkyle inférieur fait référence à un groupe alkyle contenant 1 à 4 atomes de carbone, alcoxy inférieur fait référence à un groupe O-alkyle contenant 1 à 4 atomes de carbone et alkylthio inférieur fait référence à un groupe S-alkyle contenant 1 à 4 atomes de carbone.

12. Utilisation d'une quantité prophylactiquement efficace du composé selon la revendication 1 ou la revendication 11 pour la fabrication d'un médicament destiné à réduire la mort ischémique dans une population de cellules.

13. Utilisation selon la revendication 12, dans laquelle la population de cellules comprend une cellule choisie dans le groupe consistant en une cellule neuronale, une cellule gliale, une cellule cardiaque, un lymphocyte, un macrophage et un fibroblaste.

14. Utilisation d'une quantité prophylactiquement efficace du composé selon la revendication 1 ou la revendication 11 pour la fabrication d'un médicament destiné à réduire la mort dans une population de cellules comprenant les cellules neuronales en réponse à un événement traumatique, dans laquelle ledit médicament est adapté à une administration avant, pendant ou dans une période appropriée après l'événement traumatique.

15. Procédé de réduction de la mort ischémique dans une population de cellules comprenant la mise en contact de la cellule avec une quantité prophylactiquement efficace du composé selon la revendication 1 ou la revendication 11, dans lequel la mise en contact est effectuée *ex vivo.*

16. Procédé de réduction de la mort dans une population de cellules comprenant les cellules neuronales en réponse à un événement traumatique comprenant la mise en contact de la cellule neuronale avec une quantité prophylactiquement efficace du composé selon la revendication 1 ou la revendication 11 avant, pendant
ou dans une période appropriée après l'événement traumatique, dans lequel la mise en contact est effectuée *ex vivo.*

17. Composition pharmaceutique selon la revendication 1 pour une utilisation comme médicament destiné à réduire la mort de cellules neuronales en réponse à un événement traumatique chez un sujet, dans laquelle la composition pharmaceutique selon la revendication 1 est administrée au sujet en une quantité prophylactiquement efficace avant, pendant ou après l'événement traumatique.

18. Composition pharmaceutique selon la revendication 11 pour une utilisation comme médicament destiné à réduire la mort de cellules neuronales en réponse à un événement traumatique chez un sujet, dans laquelle la composition pharmaceutique selon la revendication 11 est administrée au sujet en une quantité prophylactiquement efficace avant, pendant ou après l'événement traumatique.

19. Composition pharmaceutique selon la revendication 17, dans laquelle le sujet est un être humain.

20. Composition pharmaceutique selon la revendication 17, dans laquelle l'événement traumatique est choisi dans le groupe consistant en un trouble médical, un traumatisme physique, un traumatisme chimique et un traumatisme biologique.

21. Composition pharmaceutique selon la revendication 17, dans laquelle la composition pharmaceutique est administrée avant l'événement traumatique.

22. Composition pharmaceutique selon la revendication 17, dans laquelle la composition pharmaceutique est administrée pendant l'événement traumatique.

23. Composition pharmaceutique selon la revendication 17, dans laquelle la composition pharmaceutique est administrée à la suite de l'événement traumatique.

24. Composition pharmaceutique selon la revendication 18, dans laquelle le sujet est un être humain.

25. Composition pharmaceutique selon la revendication 18, dans laquelle l'événement traumatique est choisi dans le groupe consistant en un trouble médical, un traumatisme physique, un traumatisme chimique et un traumatisme biologique.

26. Composition pharmaceutique selon la revendication 18, dans laquelle la composition pharmaceutique est administrée avant l'événement traumatique.

27. Composition pharmaceutique selon la revendication 18, dans laquelle la composition pharmaceutique est administrée pendant l'événement traumatique.

28. Composition pharmaceutique selon la revendication 18, dans laquelle la composition pharmaceutique est administrée à la suite de l'événement traumatique.

29. Appareil d'administration à un sujet de la composition pharmaceutique selon la revendication 1 comprenant un conteneur et la composition pharmaceutique selon la revendication 1 à l'intérieur, dans lequel le conteneur comporte un dispositif pour délivrer au sujet une dose prophylactique de la composition pharmaceutique.

30. Appareil d'administration à un sujet de la composition pharmaceutique selon la revendication 11 comprenant un conteneur et la composition pharmaceutique selon la revendication 11 à l'intérieur, dans lequel le conteneur comporte un dispositif pour délivrer au sujet une dose prophylactique de la composition pharmaceutique.

31. Appareil selon la revendication 29, dans lequel le dispositif pour délivrer la composition pharmaceutique est une seringue.

32. Appareil selon la revendication 30, dans lequel le dispositif pour délivrer la composition pharmaceutique est une seringue.

33. Composé de formule 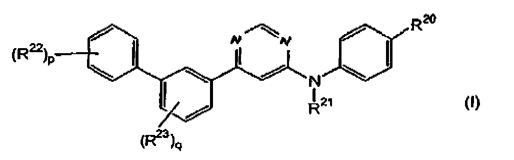 dans laquelle
R²⁰ est choisi dans le groupe consistant en 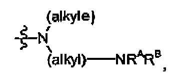 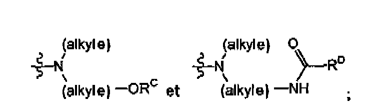 où R^{A} et R^{B} sont indépendamment choisis parmi un atome d'hydrogène, les groupe alkyle, alkyle halogène, amino, alkylamino, dialkylamino, aminvalkyle, alkylaminoalkyle, dialkylaminoalkyle, aryle, aralkyle, cycloalkyle, cycloalkyl-alkyle, hétéroaryle, hétéroaryl-alkyle, alcoxyalkyle, aryloxy, aryloxyalkyle, alcoxycarbonylalkyle et déshydroabiétyle; où la portion aryl cycloalkyle, hétéroaryle ou hëtérocycloalkyle de l'un quelconque des groupes est facultativement substituée par un ou plusieurs substituants indépendamment choisis parmi un atome d'halogène, un groupe alkyle, alcoxy, alkyle halogéné, amino, alkylamino, dialkylamino, arylamino, aralkylamino, amido, alkylamido, dialkylamido, arylamido, aralkylamido, azoïque, nitro, cyano, aryle, aralkyle, aryloxy, carboxy, alcoxycarbonyle, aryloxycarbonyle, alkylthio, arylthio, alkylsulfonylN(H) ou alkylsulfonylN(alkyle);
en variante, R^{A} et R^{B} sont pris ensemble avec l'atome d'azote auquel ils sont liés pour former un composé choisi parmi les groupes hétéroaryle et hétérocycloalkyle; où le groupe hétéroaryle ou hétérocycloalkyle est facultativement substitué par un ou plusieurs substituants indépendamment choisis parmi un atome d'halogène, un groupe alkyle, alcoxy, alcoxycarbonyle, alkyle halogéné, alkylcarbonyle, amino, alkylamino, dialkylamino, arylamino, azoïque, nitro ou cyano;
R^{c} est choisi dans le groupe consistant en un groupe alkyle, aralkyle, alkylcarbonyle, arylcarbonyle, aralkylcarbonyle, (±)-N-benzoyl-aminoelkylcarbonyle ou [3as-(3aα,4β,6aα)]-hexahydro-2-oxo-1H-thiéno[3,4-d]imidazole-alkylcarbonyle;
R^{D} est choisi parmi un groupe alkyle, aryle, aralkyle, (±)-N-benzvyl-aminoalkyle, 13as-(3aα,4β,6aα)]-hexahydro-2-oxo-1H-thiéno[3,4-d]imidazole-alkyle ou biphényle; où la portion alkyle ou aryle du groupe alkyle, aryle ou aralkyle est facultativement substituée par un ou plusieurs substituants indépendamment choisis parmi un atome d'halogène, un groupe alkyle, alcoxy, amino, alkylamino, dialkylamino, azoïque, nitro, cyano ou trifluorométhyle);
R²¹ est choisi dans le groupe consistant en un atome d'hydrogène, un groupe alkyle, aryle, aralkyle, alkylcarbonyle, arylcarbonyle et aralkylcarbonyle; où la portion aryle est facultativement substituée par un ou plusieurs substituants indépendamment choisis parmi un atome d'halogène, un groupe alkyle, alcoxy, alkyle halogéné ou nitro;
p est un entier choisi parmi 0 à 3;
q est un entier choisi parmi 0 à 3;
R²² et R²³ sont chacun indépendamment choisis dans le groupe consistant en un atome d'halogène, les groupes alkyle, alcoxy, amino, alkylamino, dialkylamino, nitro, cyano, carboxy, alcoxycarbonyle, aryloxycarbonyle, aminocarbonyle, alkylaminocarbonyle et dialkylaminocarbonyle;
ou un sel pharmaceutiquement acceptable de ceux-ci.

34. Composé selon la revendication 33, dans lequel
R^{A} est choisi dans le groupe consistant en un atome d'hydrogène, les groupes alkyle, aryle et aralkyle; où la portion aryle de l'un quelconque des groupes est facultativement substituée par un à trois substituants indépendamment choisis parmi un atome d'halogène, les groupes alkyle inférieur, alcoxy inférieur, alkyle inférieur halogéné, amino, alkylamino inférieur, di(alkyl inférieur)amino, arylamino, aralkylamino, azoïque, nitro, cyano, aryle, aralkyle, aryloxy, carboxy, alcoxycarbonyle inférieur, aryloxycarbonyle, alkylthio inférieur et arylthio;
R^{B} est choisi dans le groupe consistant en un atome d'hydrogène, les groupes alkyle, alkyle inférieur halogéné, amino, alkylamino inférieur, di(alkyl inférieur)amino, amino-alkyle inférieur, alkyl inférieur-amino-alkyle inférieur, di(alkyl inférieur)amino-alkyle inférieur, aryle, aralkyle, cycloalkyle, cycloalkyl-alkyle inférieur, hétéroaryle, hétéroaryl-alkyle inférieur, alcoxy inférieur-alkyle inférieur, aryloxy, aryloxy-alkyle inférieur, alcoxycarbonyle inférieur-alkyle inférieur et déshydroabiétyle; où la portion aryle, cycloalkyle, hétéroaryle ou hétérocycloalkyle de l'un quelconque des groupes est facultativement substituée par un à trois substituants indépendamment choisis parmi un atome d'halogène, les groupes alkyle inférieur, alcoxy inférieur, alkyle inférieur halogéné, amino, alkylamino inférieur, di(alkyl inférieur)amino, arylamino, aralkylamino, azoïque, nitro, cyano, aryle, aralkyle, aryloxy, carboxy, alcoxycarbonyle inférieur, aryloxycarbonyle, alkylthio inférieur et arylthio;
en variante, R^{A} et R^{B} sont pris ensemble avec l'atome d'azote auquel ils sont liés pour former une structure cyclique choisie dans le groupe consistant en les groupes hétéroaryle et hétérocycloalkyle; où le groupe hétéroaryle ou hétérocycloalkyle est facultativement substitué par un à deux substituants indépendamment choisis parmi un atome d'halogène, un groupe alkyle inférieur, alcoxy inférieur, alcoxycarbonyle inférieur, trifluorométhyle, alkylcarbonyle inférieur, amino, alkylamino inférieur, di(alkyl inférieur)amino, arylamino, azoïque, nitro ou cyano;
R^{c} est choisi dans le groupe consistant en les groupes alkyle inférieur, aralkyle, alkylcarbonyle inférieur, arylcarbonyle, aralkylcarbonyle, (±)-N-benzoyl-aminoalkylcarbonyle inférieur et [3aS-(3aa,4p,6aa)]-hexahydro-2-oxo-1H-thiéno[3,4-d]imidazole-alkylcarbonyle inférieur;
R^{D} est choisi dans le groupe consistant en les groupes alkyle, aryle, aralkyle, (±)-N-benzoyl-aminoalkyle inférieur, [3aS-(3aα,4β,6aα)]-hexahydro-2-oxo-1H-thiéno[3,4-d]imidazole-alkyle inférieur et biphényle; où la portion alkyle ou aryle du groupe aryle ou aralkyle est facultativement substituée par un à deux substituants indépendamment choisis parmi un atome d'halogène, un groupe alkyle inférieur, alcoxy inférieur, amino, alkylamino inférieur, di(alkyl inférieur)amino, azoïque, nitro, cyano ou trifluorométhyle);
R²¹ est choisi dans le groupe consistant en un atome d'hydrogène, un groupe alkyle inférieur, aryle, aralkyle, alkylcarbonyle inférieur, arylcarbonyle et aralkylcarbonyle (où la portion aryle du groupe aryle, aralkyle, arylcarbonyle ou aralkylcarbonyle est facultativement substituée par un à deux substituants indépendamment choisis parmi un atome d'halogène, un groupe alkyle inférieur, alcoxy inférieur ou trifluorométhyle);
p est un entier de 0 à 2;
q est un entier de 0 à 2;
R²² et R²³ sont chacun indépendamment choisis dans le groupe consistant en un atome d'halogène, les groupes alkyle inférieur, alcoxy inférieur, amino, alkylamino inférieur, di(alkyl inférieur)amino, nitro, cyano, carboxy, alcoxycarbonyle inférieur, phényloxycarbonyle, aminocarbonyle, alkylaminocarbonyle inférieur et di(alkyl inférieur)aminocarbonyle
ou un sel pharmaceutiquement acceptable de ceux-ci, et où alkyle inférieur fait référence à un groupe alkyle contenant 1 à 4 atomes de carbone, alcoxy inférieur fait référence à un groupe 0-alkyle contenant 1 à 4 atomes de carbone et alkylthio inférieur fait référence à un groupe S-alkyle contenant 1 à 4 atomes de carbone.

35. Composé selon la revendication 34, dans lequel R²⁰ est 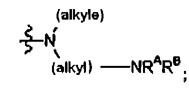 p vaut 0 et q vaut 0.

36. Composé selon la revendication 35, dans lequel
R^{A} est choisi dans le groupe consistant en un atome d'hydrogène, les groupes alkyle inférieur et aralkyle;
R^{B} est choisi dans le groupe consistant en un atome d'hydrogène, les groupes alkyle inférieur, alkyle inférieur halogéné, aralkyle, aralkyle substitué (où les substituants sur le groupe aralkyle sont un à trois substituants indépendamment choisis parmi un atome d'halogène, un groupe alkyle inférieur, alcoxy inférieur ou trifluorométhyle), alcoxyalkyle, cycloalkyl-alkyle, déshydroabiétyle, di(alkyl inférieur)-amino-alkyle inférieur, biphényle, hétéroaryle, hétéroaryle substitué (où les substituants sur le groupe hétéroaryle sont un à deux substituants indépendamment choisis parmi un atome d'halogène ou un groupe alkyle inférieur), hétéroaryl-alkyle inférieur, aryloxy-alkyle inférieur et alcoxycarbonyl inférieur-alkyle inférieur; et
R²¹ est un atome d'hydrogène;
ou un sel pharmaceutiquement acceptable de ceux-ci, et où alkyle inférieur fait référence à un groupe alkyle contenant 1 à 4 atomes de carbone, alcoxy inférieur fait référence à un groupe O-alkyle contenant 1 à 4 atomes de carbone et alkylthio inférieur fait référence à un groupe S-alkyle contenant 1 à 4 atomes de carbone.

37. Composé selon la revendication 36, dans lequel
R^{A} est choisi dans le groupe consistant en un atome d'hydrogène, les groupes méthyle, éthyle et benzyle; et
R^{B} est choisi dans le groupe consistant en un atome d'hydrogène, les groupes méthyle, propyle, n-butyle, benzyle, phényléthyle, méthoxypropyle, cyclohexylméthyle, 3-chlorvbenzyle, 2,4-diméthoxybenzyle, 2-éthoxybenzyle, 2,5-difluorobenzyle, déshydroabiétyle, 3,4-diméthoxybenzyle, diéthylaminopropyle, 4-bromo-2-pyridyle, diméthylaminoéthyle, 4-biphényle, 2-furanylméthyle, 3-iodobenzyle, 2,2,2-trifluoroéthyle, 3,4-difluorobenzyle, 2-thiényléthyle, 3,5-diméthyl-2-pyridyle, 2-(éthoxy)acétyle, 2-méthoxybenzyle, 4-bromobenzyle, 3,5-ditrifluorométhylbenzyle, 3-méthoxyphényléthyle, 3,4,5-triméthoxybenzyle, 4-méthoxyphényléthyle, 4-imidazolyléthyle, 2-trifluorométhylbenzyle, 3-méthoxybenzyle, 3-méthylbenzyle, 3,5-diméthoxybenzyle, 2-bromobenzyle, 4-fluorobenzyle, 1-naphtylméthyle, phénoxyéthyle, 4-trifluorométhylbenzyle, 3-pyridyle, 2-méthylbenzyle, 2-fluorobenzyle et 3,4-diméthoxyphényléthyle;
ou un sel pharmaceutiquement acceptable de ceux-ci.

38. Composé selon la revendication 37, dans lequel
R^{A} est choisi parmi un atome d'hydrogène, les groupes méthyle et benzyle; et
R^{B} est choisi dans le groupe consistant en les groupes méthyle, méthoxypropyle, cyclohexylméthyle, 3-chlorobenzyle, 2,5-difluorobenzyle, phényléthyle, 4-bromo-2-pyridyle, diméthylaminoéthyle, n-butyle, 2-furanylméthyle, 3,4-difluorobenzyle, 2-thiényléthyle, 4-bromobenzyle, 3-méthoxyphényléthyle, 3,4,5-triméthoxybenzyle, benzyle, 3-méthylbenzyle et phénoxyéthyle;
ou un sel pharmaceutiquement acceptable de ceux-ci.

39. Composé selon la revendication 38, dans lequel
R^{A} est choisi dans le groupe consistant en un atome d'hydrogène et un groupe méthyle; et
R^{B} est choisi dans le groupe consistant en les groupes méthyle, méthoxypropyle, 2,5-difluorobenzyle, 3,4-difluorobenzyle, 4-bromobenzyle, 3,4,5-triméthoxybenzyle, benzyle, 3-méthylbenzyle et n-butyle;
ou un sel pharmaceutiquement acceptable de ceux-ci.

40. Composé selon la revendication 39, dans lequel R^{A} est un groupe méthyle et R^{B} est un groupe méthyle; ou un sel pharmaceutiquement acceptable de ceux-ci.

41. Composé selon la revendication 35, dans lequel
R^{A} et R^{B} sont pris ensemble avec l'atome d'azote auquel ils sont liés pour former une structure cyclique choisie dans le groupe consistant en les groupes hétéroaryle, hétérocycloalkyle et hétérocycloalkyle substitué par un groupe butoxycarbonyle tertiaire; et
R²¹ est un atome d'hydrogéne;
ou un sel pharmaceutiquement acceptable de ceux-ci.

42. Composé selon la revendication 41, dans lequel
R^{A} et R^{B} sont pris ensemble avec l'atome d'azote auquel ils sont liés pour former une structure cyclique choisie dans le groupe consistant en les groupes N-pyrrolidinyle, N-morpholinyle, N-imidazolyle, N-1,2,3,4-tétrahydroisoquinoléinyle, N-hexaméthylèneimine et N-(4-butoxycarbonyl tertiaire)pipérazinyle;
ou un sel pharmaceutiquement acceptable de ceux-ci.

43. Composé selon la revendication 42, dans lequel
R^{A} et R^{B} sont pris ensemble avec l'atome d'azote auquel ils sont liés pour former une structure cyclique choisie dans le groupe consistant en les groupes N-morpholinyle et N-(4-butoxycarbonyl tertiaire)pipérazinyle;
ou un sel pharmaceutiquement acceptable de ceux-ci.

44. Composé selon la revendication 43, dans lequel
R^{A} et R^{B} sont pris ensemble avec l'atome d'azote auquel ils sont liés pour former le groupe N-morpholinyle; ou un sel pharmaceutiquement acceptable de celui-ci.

45. Composé selon la revendication 34, dans lequel R²⁰ est 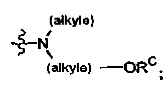 p vaut 0 et q vaut 0.

46. Composé selon la revendication 45, dans lequel
R^{c} est choisi dans le groupe consistant en les groupes arylcarbonyle, (±)-N-benzoyl-aminoalkylcarbonyle et [3aS-(3aα,4β,6aα)]-hexahydro-2-oxo-1H-thieno[3,4-d]imidazole-alkylcarbonyle; et
R²¹ est un atome d'hydrogène;
ou un sel pharmaceutiquement acceptable de ceux-ci.

47. Composé selon la revendication 46, dans lequel
R^{C} est choisi dans le groupe consistant en les groupes benzoyle, (±)-N-benzoyl-2-aininopropionoyle et [3aS-(3aα,4β,6aα)]-hexahydro-2-oxo-1H-thiéno[3,4-d]imidazole-4-pentanoyle; ou un sel pharmaceutiquement acceptable de ceux-ci.

48. composé selon la revendication 34, dans lequel R²⁰ est 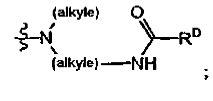 p vaut 0 et q vaut 0.

49. Composé selon la revendication 48, dans lequel
R^{D} est choisi dans le groupe consistant les groupes en (±)-N-benzoyl-2-aminoalkyle, [3aS-(3aα,4β,6aα)]-hexahydro-2-oxo-1H-thiéno[3,4-d]imidazole-alkyle, alkyle, aryle substitué (où les substituants sur le groupe aryle sont indépendamment choisis parmi les groupes azoïque, nitro, halogène, alcoxy, trifluorométhyle), biphényle, aralkyle et aralkyle substitué (où la portion alkyle du groupe aralkyle est substituée par un substituant choisi parmi un atome d'halogène); et
R²¹ est choisi dans le groupe consistant en un atome d'hydrogène, les groupes alkylcarbonyle et aralkylcarbonyle substitué (où le substituant sur la portion aryle du groupe arylcarbonyle est choisi parmi un atome d'halogène ou un groupe trifluorométhyle);
ou un sel pharmaceutiquement acceptable de ceux-ci.

50. Composé selon la revendication 49, dans lequel
R^{D} est choisi dans le groupe consistant en les groupes (±)-N-benzoyl-2-aminoéth-2-yle, [3aS-(3aa,4p,6aa)]-hexahydro-2-oxo-1H-thiéno[3,4-d]imidazole-but-4-yle, 1-chloro-1-phényl-méthyle, 3-azo-6-nitrophényle, pentadécanyle, 4-biphényle, 4-butoxyphényle, 4-trifluorométhylphényle, 3-fluorophényle et propyle; et
R²¹ est choisi dans le groupe consistant en un atome d'hydrogène, les groupes propylcarbonyle, 3-fluorophénylcarbonyle et 4-trifluorométhylphénylcarbonyle;
ou un sel pharmaceutiquement acceptable de ceux-ci.

51. Composition pharmaceutique comprenant un véhicule pharmaceutiquement acceptable et un composé selon la revendication 33.

52. Composition pharmaceutique préparée en mélangeant un composé selon la revendication 33 et un véhicule pharmaceutiquement acceptable.

53. Procédé de préparation d'une composition pharmaceutique comprenant le mélange d'un composé selon la formule 33 et d'un véhicule pharmaceutiquement acceptable.

54. Utilisation d'une quantité prophylactiquement efficace du composé selon la revendication 33 pour la fabrication d'un médicament destiné à réduire la mort ischémique dans une population de cellules.

55. Utilisation selon la revendication 54, dans laquelle au moins une cellule dans la population de cellules est choisie dans le groupe consistant en une cellule neuronale, une cellule gliale, une cellule cardiaque, un lymphocyte, un macrophage et un fibroblaste.

56. Utilisation d'une quantité prophylactiquement efficace du composé selon la revendication 33 pour la fabrication d'un médicament destiné à réduire la mort dans une population de cellules comprenant les cellules neuronales en réponse à un événement traumatique, dans laquelle ledit médicament est adapté pour une administration avant, pendant ou après l'événement traumatique.

57. Composé selon la revendication 33 par une utilisation comme médicament destiné à réduire la mort de cellules neuronales en réponse à un événement traumatique chez un sujet, dans lequel le composé selon la revendication 33 est administré au sujet en une quantité prophylactiquement efficace avant, pendant ou après l'événement traumatique.

58. Composé selon la revendication 57, dans lequel l'événement traumatique est choisi dans le groupe consistant en un trouble médical, un traumatisme physique, un traumatisme chimique et un traumatisme biologique.

59. Utilisation d'un composé selon la revendication 33 pour la préparation d'un médicament destiné à réduire la probabilité qu'une cellule subisse une mort ischémique, chez un sujet qui le nécessite.
